# EUROPEAN PATENT SPECIFICATION

(11) **EP 2 158 319 B1**
(45) Date of publication and mention of the grant of the patent: **07.12.2011**
(21) Application number: 08757088.3
(22) Date of filing: 12.05.2008
(51) Int. Cl.: C12N 15/55, A61K 38/46, A61P 19/08, C12N 15/85, C12N 5/10, C12N 9/16

(54) **BONE TARGETED ALKALINE PHOSPHATASE, KITS AND METHODS OF USE THEREOF**
AUF KNOCHEN ALS ZIEL GERICHTETE ALKALISCHE PHOSPHATASE, KITS UND VERWENDUNGSVERFAHREN DAFÜR
PHOSPHATASE ALCALINE CIBLANT LES OS, TROUSSES ET PROCÉDÉS POUR SON UTILISATION

(30) Priority: 11.05.2007 US 917589 P
(43) Date of publication of application: 03.03.2010
(60) Divisional application: 11004496.3
(73) Proprietor: Enobia Pharma Inc., Montréal, Québec H1W 4A4 (CA)
(72) Inventor: CRINE, Philippe, Outremont, Quebec H2V 2X1 (CA); BOILEAU, Guy, Brossard, Quebec J4Y 1E6 (CA); LOISEL, Thomas, P., Montreal, Quebec H8Y 1Z7 (CA); LEMIRE, Isabelle, Montreal, Quebec H1T 2P1 (CA); LEONARD, Pierre, Montreal, Quebec H4J 2C4 (CA); HEFT, Robert, Dollard-des-Ormeaux, Quebec H9A 1V5 (CA); LANDY, Hal, Cambridge, MA 02139 (US)
(74) Representative: Lahrtz, Fritz
(86) International application number: PCT/CA2008/000923
(87) International publication number: WO 2008/138131

(56) References cited:
- WO-A1-2005/103263
- WO-A2-2006/039480
- US-A1- 2007 081 984
- MILLÁN JOSÉ LUIS ET AL: "Enzyme replacement therapy for murine hypophosphatasia." JOURNAL OF BONE AND MINERAL RESEARCH : THE OFFICIAL JOURNAL OF THE AMERICAN SOCIETY FOR BONE AND MINERAL RESEARCH JUN 2008 LNKD- PUBMED:18086009, vol. 23, no. 6, 17 December 2007 (2007-12-17), pages 777-787, XP002585178 ISSN: 1523-4681
- DUMONT JENNIFER A ET AL: "MONOMERIC FC FUSIONS: IMPACT ON PHARMACOKINETIC AND BIOLOGICAL ACTIVITY OF PROTEIN THERAPEUTICS" BIODRUGS: CLINICAL IMMUNOTHERAPEUTICS, BIOPHARMACEUTICALS AND GENE THERAPY, ADIS INTERNATIONAL, FR LNKD- DOI:10.2165/00063030-200620030-00002, vol. 20, no. 3, 1 January 2006 (2006-01-01), pages 151-160, XP009082835 ISSN: 1173-8804
- NISHIOKA T. ET AL.: 'Enhancement of drug delivery to bone: Characterization of human tissue-nonspecific alkaline phosphatase tagged with an acidic oligopeptide' MOL. GENET. METAB. vol. 88, no. 3, July 2006, pages 244 - 255, XP005524861

## Description

### FIELD OF THE INVENTION

The present invention relates to bone targeted alkaline phosphatase, kits and methods of use thereof.

### BACKGROUND OF THE INVENTION

Hypophosphatasia (HPP) is a rare, heritable form of rickets or osteomalacia (Whyte 2001) with an incidence as great as 1 per 2,500 births in Canadian Mennonites (Greenberg, 1993) and of 1 per 100,000 births in the general population for the more severe form of the disease. Milder forms are more prevalent. This "inborn error of metabolism" is caused by loss-of-function mutation(s) in the gene *(ALPL)* that encodes the tissue-nonspecific isozyme of alkaline phosphatase (TNALP; a.k.a liver/bone/kidney type ALP) (Weiss et al. 1988; Henthorn et al. 1992a; Henthorn et al. 1992b; Zurutuza et al. 1999; Millán 1995). The biochemical hallmark is subnormal ALP activity in serum (hypophosphatasemia), which leads to elevated blood and/or urine levels of three phosphocompound substrates: inorganic pyrophosphate (PPᵢ) phosphoethanolamine (PEA), and pyridoxal 5'-phosphate (PLP) (Whyte 1994).

HPP features a remarkable range of severity ranging from (most severe to mildest) perinatal, infantile, childhood, adult, and odontohypophosphatasia forms, classified historically according to age at diagnosis (Whyte 2001). There may be almost complete absence of bone mineralization *in utero* with stillbirth, or spontaneous fractures and dental disease occurring first in adult life. Perinatal (lethal) Hypophosphatasia is expressed *in utero* and can cause stillbirth. Some neonates may survive several days but suffer increased respiratory compromise due to the hypoplastic and rachitic disease of the chest. In infantile HPP, diagnosed before 6 months-of-age, postnatal development seems normal until onset of poor feeding, inadequate weight gain, and appearance of rickets. Radiological features are characteristic and show impaired skeletal mineralization, sometimes with progressive skeletal demineralization leading to rib fractures and chest deformity. Childhood Hypophosphatasia has also highly variable clinical expression. Premature loss of deciduous teeth results from aplasia, hypoplasia or dysplasia of dental cementum that connects the tooth root with the periodontal ligament. Rickets causes short stature and the skeletal deformities may include bowed legs, enlargement of the wrists, knees and ankles as a result of flared metaphysis. Adult HPP usually presents during middle age, although frequently there is a history of rickets and/or early loss of teeth followed by good health during adolescence and young adult life. Recurrent metatarsal stress fractures are common and calcium pyrophosphate dihydrate deposition causes attacks of arthritis and pyrophosphate arthropathy. Odontohypophosphatasia is diagnosed when the only clinical abnormality is dental disease and radiological studies and even bone biopsies reveal no signs of rickets or osteomalacia.

The severe clinical forms of Hypophosphatasia are usually inherited as autosomal recessive traits with parents of such patients showing subnormal levels of serum AP activity (Whyte 2001). For the milder forms of hypophosphatasia, i.e., adult and odontohypophosphatasia, an autosomal dominant pattern of inheritance has also been documented (Whyte 2001).

In the healthy skeleton, TNALP is an ectoenzyme present on the surface of the plasma membrane of osteoblasts and chondrocytes, including on the membranes of their shed matrix vesicles (MVs) (Ali et al. 1970; Bernard 1978) where the enzyme is particularly enriched (Morris et al. 1992). Deposition of hydroxyapatite during bone mineralization normally initiates within the lumen of these MVs (Anderson et al. 2005a). Electron microscopy has shown that TNALP-deficient MVs from severely affected HPP patients and *Akp2*^{*-*/*-*} mice (a TNALP null mouse model, see below) contain hydroxyapatite crystals, but that extravesicular crystal propagation appears retarded (Anderson 1997; Anderson 2004). This defect is attributed to the extracellular accumulation of PPᵢ, a potent inhibitor of calcification (Meyer 1984) due to deficiency of TNALP activity (Hessle et al. 2002; Harmey et al. 2004; Harmey et al. 2006.).

When PPi is present at near physiological concentrations, in the range of 0.01-0.1 mM, PPi has the ability to stimulate mineralization in organ-cultured chick femurs (Anderson & Reynolds 1973) and also by isolated rat MVs (Anderson et al. 2005b), while at concentrations above 1 mM, PPi inhibits calcium phosphate mineral formation by coating hydroxyapatite crystals, thus preventing mineral crystal growth and proliferative self-nucleation. Thus, PPi has a dual physiological role; it can function as a promoter of mineralization at low concentrations but as an inhibitor of mineralization at higher concentrations. TNALP has been shown to hydrolyze the mineralization inhibitor PPi to facilitate mineral precipitation and growth (Rezende et al. 1998). Recent studies using the Akp2^{-/-} mice have indicated that the primary role of TNALP *in vivo* is to restrict the size of the extracellular PPi pool to allow proper skeletal mineralization (Hessle et al. 2002; Harmey et al. 2004).

The severity of Hypophosphatasia is variable and modulated by the nature of the TNALP mutation. Missense mutations in the enzyme's active site vicinity, homodimer interface, crown domain, amino-terminal arm and calcium-binding site have all been found to affect the catalytic activity of TNALP (Zurutuza et al. 1999). Additionally, other missense, nonsense, frame-shift and splice site mutations have been shown to lead to aberrant mutant proteins or intracellular trafficking defects that lead to subnormal activity on the cell surface. The multitude of mutations and the fact that compound heterozygocity is a common occurrence in Hypophosphatasia also explains the variable expressivity and incomplete penetrance often observed in this disease (Whyte 2001).

Progress on the human form of the disease benefits greatly from the existence of the TNALP null mice (Akp2^{-/-}) as an animal model. These Akp2^{-/-} mice phenocopy infantile HPP remarkably well, as they are born with a normally mineralized skeleton, but develop radiographically apparent rickets at about 6 days of age, and die between day 12-16 suffering severe skeletal hypomineralization and episodes of apnea and epileptic seizures attributable to disturbances in PLP (vitamin B₆) metabolism (Waymire et al. 1995; Narisawa et al. 1997; Fedde et al. 1999; Narisawa et al. 2001).

Some TNALP active site mutations have been shown to affect the ability of the enzyme to metabolize PPi or PLP differently (Di Mauro et al. 2002). Both PLP and PPi are confirmed natural substrates of TNALP and abnormalities in PLP metabolism explain the epileptic seizures observed in Akp2^{-/-} mice (Waymire et al. 1995; Narisawa et al. 2001), while abnormalities in PPi metabolism explain the skeletal phenotype in this mouse model of Hypophosphatasia (Hessle et al. 2002; Anderson et al. 2004; Harmey et al. 2004; Harmey et al. 2006; Anderson et al. 2005a).

There is no established medical therapy for HPP. Case reports of enzyme replacement therapy (ERT) using intravenous (i.v.) infusions of TNALP-rich plasma from Paget bone disease patients and purified placental ALP have described failure to rescue affected infants (Whyte et al. 1982; Whyte et al. 1984). In another similar study, Weninger et al. (Weninger et al. 1989) attempted ERT for a severely affected premature boy with Hypophosphatasia by infusions of purified human liver TNALP. Treatment (1.2 IU/kg/min) started at age three weeks and was repeated in weekly intervals until age 10 weeks, when the child died. Samples of TNALP were diluted with 10 ml of physiological saline and infused over 30 min via an umbilical arterial catheter. No toxic or allergic side effects were observed. Serum TNALP activity increased from 3 IU/L before treatment to a maximum level of 195 IU/L with a half-life time between 37 and 62 hours. Sequential radiographic studies however showed no improvement of bone mineralization (Weninger et al. 1989).

It seems that ALP activity must be increased not in the circulation, but in the skeleton itself. This hypothesis is supported by seemingly beneficial responses of two girls with infantile HPP following marrow cell transplantation where TNALP-containing cells were introduced throughout the skeleton (Whyte et al. 2003). Thus there seems to be a need to provide active TNALP to the skeleton of these patients. Recent reports have indicated that poly-aspartate sequences confer bone homing properties to recombinant TNALP (WO 2005/103263 to Crine et al.; Nishioka et al. 2006). WO 2005/103263 discloses therapeutics that include sALP fused to D10 and their use in enzyme replacement in HPP.

A recent report showed that the mutated form of TNALP R450C (although Nasu et al. refers to a R433C mutation, his numbering applies to the mature protein and not to one comprising the signal peptide) produces a protein having a dimeric structure joined by a disulfide bridge between the cysteine residues at position 450 of each subunit which strongly inhibited its alkaline phosphatase activity. Nasu et al. concluded that the loss of function results from the interchain disulfide bridge and is the molecular basis for the lethal hypophosphatasia associated with R450C (Nasu et al. 2006).

The present description refers to a number of documents, the content of which is herein incorporated by reference in their entirety.

### SUMMARY OF THE INVENTION

Given the current limitations in the clinical management and treatment of patients with HPP, an alternative and efficient treatment was needed. Accordingly, the present invention provides an efficient enzyme replacement therapy for the treatment of HPP.

To the Applicant's knowledge, and as opposed to previous enzyme replacement therapy efforts in either TNALP null mice or HPP infants in which TNALP or other ALP isozymes were delivered intravenously, the present invention marks the first time where near complete resolution of clinical radiographic and biochemical changes has been documented to occur with enzyme replacement alone.

### Bone targeted sALP

The bone targeted composition of the present invention comprise a fusion protein including in order from the amino side to the carboxylic side a sALP, a spacer, and a bone targeting negatively charged peptide, wherein the spacer comprises a fragment crystallizable region (Fc), and the peptide is a polyaspartate or a polyglutamate Wn, wherein n= 10-16, and wherein the sA ALP consists of amino acid residues 18-502 of Seq ID No.8.

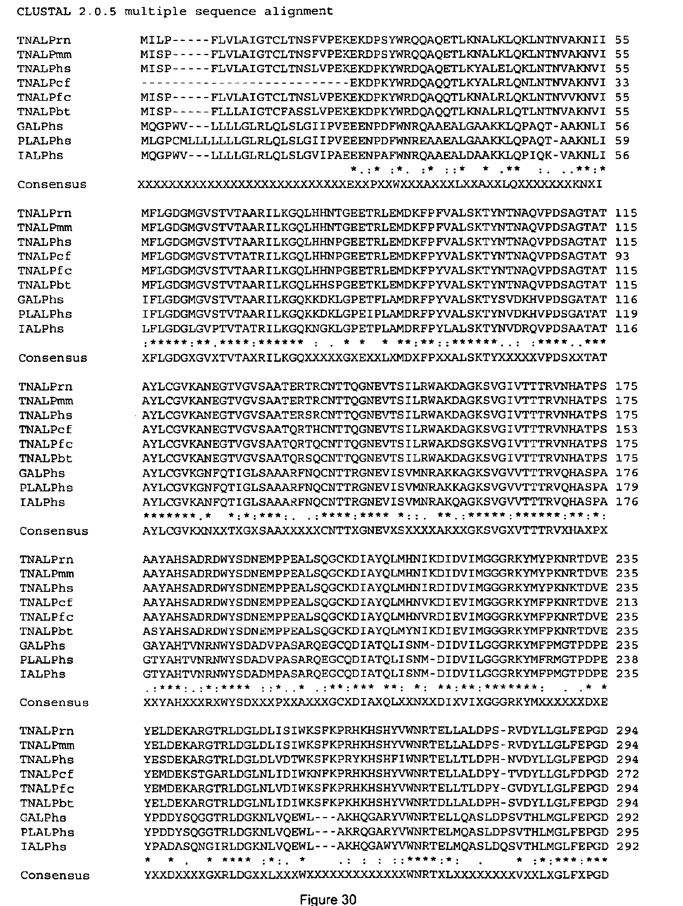

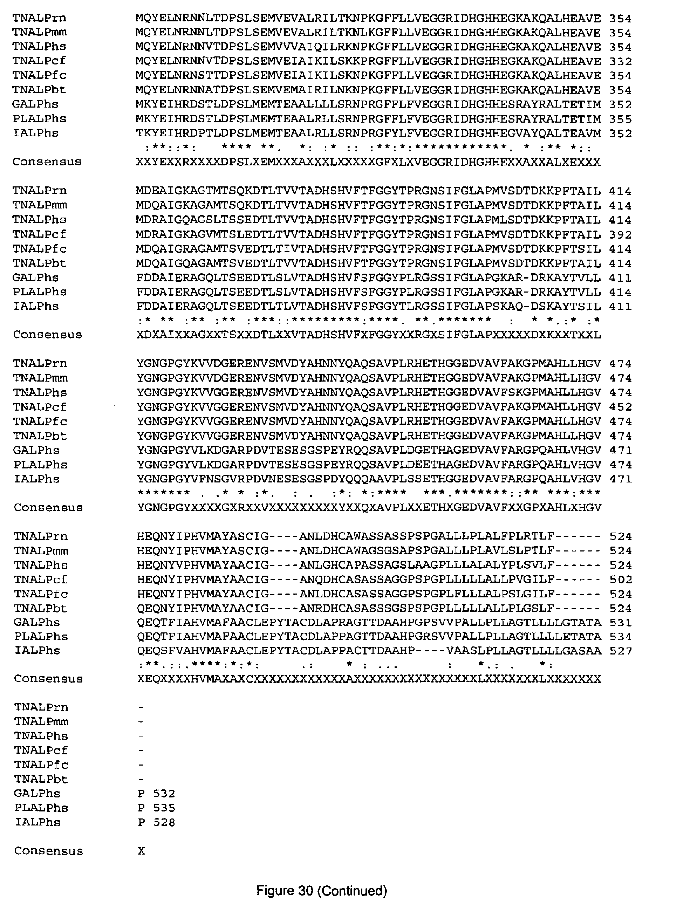

There are four known isozymes of ALP, namely tissue non specific alkaline phosphatase further described below, placental alkaline phosphatase (PALP) (e.g., [NP_112603], [NP_001623]), germ cell alkaline phosphatase (GCALP) (e.g., [P1D696]) and intestinal alkaline phosphatase (e.g., [NP_001622]). These enzymes possess very similar three dimensional structure. Each of their catalytic sites contains four metal binding domains for metal ions necessary for enzymatic activity including two Zn and one Mg. These enzymes catalyze the hydrolysis of monoesters of phosphoric acid and also catalyze a transphosphorylation reaction in the presence of high concentrations of phosphate acceptors. It has been shown in particular that PALP is physiologically active toward phosphoethanolamine (PEA), inorganic pyrophosphate (PPi) and pyridoxal 5'-phosphate (PLP), all three being known natural substrate for TNALP (Whyte, 1995). An alignment between these isozymes is presented in Figure 30.

### TNALP

As indicated above, TNALP is a membrane-bound protein anchored through a glycolipid to its C-terminal (Swiss-Prot, P05186). This glycolipid anchor (GPI) is added post translationally after removal of a hydrophobic C-terminal end which serves both as a temporary membrane anchor and as a signal for the addition of the GPI. Hence the soluble human TNALP used in all Examples below is comprised of a TNALP wherein the first amino acid of the hydrophobic C-terminal sequence, namely alanine, is replaced by a stop codon. The soluble TNALP (herein called sTNALP) so formed contains all amino acids of the native anchored form of TNALP necessary for the formation of the catalytic site but lacks the GPI membrane anchor. Known TNALP include human TNALP [NP-000469, AAI10910, AAH90861, AAH66116, AAH21289, AAI26166]; rhesus TNALP [XP-001109717];rat TNALP [NP_037191]; dog TNALP [AAF64516]; pig TNALP [AAN64273], mouse [NP_031457], bovine [NP_789828, NP_776412, AAI18209, AAC33858], and cat [NP_001036028 ].

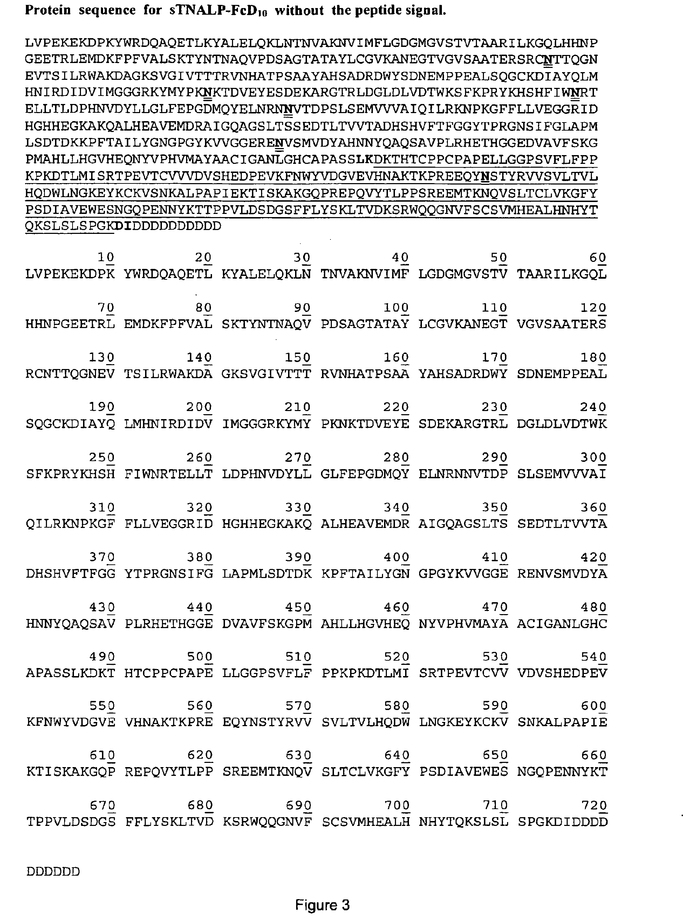

The bone targeted composition disclosed encompasses sequences satisfying a consensus sequence derived from the ALP extracellular domain of human ALP isozymes and of known functional TNALPs (human, mouse, rat, bovine, cat and dog). As used herein the terminology "extracellular domain" is meant to refer to any functional extracellular portion of the native protein (i.e. without the peptide signal). It has been shown that recombinant sTNALP retaining original amino acids 1 to 501 (18 to 501 when secreted) (see Oda et al., J. Biochem 126: 694-699, 1999), amino acids 1 to 504 (18 to 504 when secreted) (US Patent 6,905,689 to Bemd et al.) and amino acids 1 to 505 (18-505 when secreted) (US 2007/0081984 to Tomatsu et al.), are enzymatically active. Examples presented herein also show that a recombinant sTNALP retaining amino acids 1 to 502 (18 to 502 when secreted) (Figure 3) of the original TNALP is enzymatically active. This indicates that amino acid residues can be removed from the C-terminal end of the native protein without affecting its enzymatic activity. According to the present invention, the bone targeted alkaline phosphatase comprises a sALP, wherein the sALP consists of amino acid residues 18-502 of Seq ID No.8.

Table 1 below provides a list of 194 mutations known to cause HPP. It is disclosed that in the bone targeted polypeptides of the present invention, the ALP sequence does not include any of these mutations.

Hence, in sALPs dislosed, using the numbering of a consensus sequence derived from an alignment of various TNALPs and of human ALP isozymes, the amino acid at position 22 is not a phenylalanine residue; the amino acid at position 33 (position 11 in the sequence without signal peptide) is not a cysteine residue; the amino acid at position 38 (position 16 in the sequence without signal peptide) is not a valine residue; the amino acid at position 42 (position 20 in the sequence without signal peptide) is not a proline residue; the amino acid at position 45 (position 23 in the sequence without signal peptide) is not a valine residue; the amino acid residue at position 56 (position 34 in the sequence without signal peptide) is not a serine or a valine residue; the amino acid residue at position 67 (position 45 in the sequence without signal peptide) is not a leucine, an isoleucine or a valine residue; the amino acid residue at position 68 (position 46 in the sequence without signal peptide) is not a valine residue; the amino acid residue at position 73 (position 51 in the sequence without signal peptide) is not a methionine residue; the amino acid residue at position 76 (position 54 in the sequence without signal peptide) is not a cysteine, a serine, a proline or a histidine residue; the amino acid residue at position 77 (position 55 in the sequence without signal peptide) is not a threonine residue; the amino acid residue at position 80 (position 58 in the sequence without signal peptide) is not a serine residue; the amino acid residue at position 81 (position 59 in the sequence without signal peptide) is not an asparagine residue; the amino acid residue at position 105 (position 83 in the sequence without signal peptide) is not a methionine residue; the amino acid residue at position 113 (position 89 in the sequence without signal peptide) is not a leucine residue; the amino acid residue at position 116 (position 94 in the sequence without signal peptide) is not a threonine residue; the amino acid residue at position 117 (position 95 in the sequence without signal peptide) is not a serine residue; the amino acid residue at position 119 (position 97 in the sequence without signal peptide) is not a glycine residue; the amino acid residue at position 121 (position 99 in the sequence without signal peptide) is not a serine or a threonine residue; the amino acid residue at position 125 (position 103 in the sequence without signal peptide) is not an arginine residue; the amino acid residue at position 128 (position 106 in the sequence without signal peptide) is not a aspartate residue; the amino acid residue at position 133 (position 111 in the sequence without signal peptide) is not a methionine residue; the amino acid residue at position 134 (position 112 in the sequence without signal peptide) is not an arginine residue; the amino acid residue at position 137 (position 115 in the sequence without signal peptide) is not a threonine or a valine residue; the amino acid residue at position 139 (position 117 in the sequence without signal peptide) is not a histidine or an asparagine residue; the amino acid residue at position 141 (position 119 in the sequence without signal peptide) is not a histidine residue; the amino acid residue at position 153 (position 131 in the sequence without signal peptide) is not an alanine or an isoleucine residue; the amino acid residue at position 167 (position 145 in the sequence without signal peptide) is not a serine or a valine residue; the amino acid residue at position 172 (position 150 in the sequence without signal peptide) is not a methionine residue; the amino acid residue at position 175 (position 153 in the sequence without signal peptide) is not an aspartate residue; the amino acid residue at position 176 (position 154 in the sequence without signal peptide) is not a tyrosine or an arginine residue; the amino acid residue at position 181 (position 159 in the sequence without signal peptide) is not a threonine residue; the amino acid residue at position 182 (position 160 in the sequence without signal peptide) is not a threonine residue; the amino acid residue at position 184 (position 162 in the sequence without signal peptide) is not a threonine residue; the amino acid residue at position 186 (position 164 in the sequence without signal peptide) is not a leucine residue; the amino acid residue at position 189 (position 167 in the sequence without signal peptide) is not a tryptophan residue; the amino acid residue at position 194 (position 172 in the sequence without signal peptide) is not a glutamate residue; the amino acid residue at position 196 (position 174 in the sequence without signal peptide) is not a lysine or a glycine residue; the amino acid residue at position 197 (position 175 in the sequence without signal peptide) is not a threonine residue; the amino acid residue at position 198 (position 176 in the sequence without signal peptide) is not an alanine residue; the amino acid residue at position 206 (position 184 in the sequence without signal peptide) is not a tyrosine residue; the amino acid residue at position 208 (position 186 in the sequence without signal peptide) is not a glutamate residue; the amino acid residue at position 207 (position 190 in the sequence without signal peptide) is not a proline residue; the amino acid residue at position 216 (position 194 in the sequence without signal peptide) is not a aspartate residue; the amino acid residue at position 217 (position 195 in the sequence without signal peptide) is not a phenylalanine residue; the amino acid residue at position 223 (position 201 in the sequence without signal peptide) is not a threonine residue; the amino acid residue at position 225 (position 203 in the sequence without signal peptide) is not a valine or an alanine residue; the amino acid residue at position 226 (position 204 in the sequence without signal peptide) is not a valine residue; the amino acid residue at position 228 (position 206 in the sequence without signal peptide) is not a tryptophan or a glutamine residue; the amino acid residue at position 229 (position 207 in the sequence without signal peptide) is not a glutamate residue; the amino acid residue at position 231 (position 209 in the sequence without signal peptide) is not a threonine residue; the amino acid residue at position 240 (position 218 in the sequence without signal peptide) is not a glycine residue; the amino acid residue at position 251 (position 229 in the sequence without signal peptide) is not a serine residue; the amino acid residue at position 254 (position 232 in the sequence without signal peptide) is not a valine residue; the amino acid residue at position 269 (position 247 in the sequence without signal peptide) is not an arginine residue; the amino acid residue at position 277 (position 255 in the sequence without signal peptide) is not a cysteine, a leucine or a histidine residue; the amino acid residue at position 280 (position 258 in the sequence without signal peptide) is not a proline residue; the amino acid residue at position 295 (position 273 in the sequence without signal peptide) is not a phenylalanine residue; the amino acid residue at position 297 (position 275 in the sequence without signal peptide) is not a lysine residue; the amino acid residue at position 298 (position 276 in the sequence without signal peptide) is not a threonine residue; the amino acid residue at position 300 (position 278 in the sequence without signal peptide) is not a tyrosine or an alanine residue; the amino acid residue at position 301 (position 279 in the sequence without signal peptide) is not a valine, a threonine or an isoleucine residue; the amino acid residue at position 303 (position 281 in the sequence without signal peptide) is not an aspirate residue; the amino acid residue at position 304 (position 282 in the sequence without signal peptide) is not a lysine residue; the amino acid residue at position 305 (position 283 in the sequence without signal peptide) is not a proline residue; the amino acid residue at position 312 (position 290 in the sequence without signal peptide) is not a valine residue; the amino acid residue at position 313 (position 291 in the sequence without signal peptide) is not a serine or a leucine residue; the amino acid residue at position 317 (position 295 in the sequence without signal peptide) is not a lysine residue; the amino acid residue at position 332 (position 310 in the sequence without signal peptide) is not an arginine residue; the amino acid residue at position 333 (position 311 in the sequence without signal peptide) is not a cysteine, a glycine or a leucine residue; the amino acid residue at position 334 (position 312 in the sequence without signal peptide) is not a leucine residue; the amino acid residue at position 340 (position 318 in the sequence without signal peptide) is not an aspartate residue; the amino acid residue at position 345 (position 323 in the sequence without signal peptide) is not an arginine or a glutamate residue; the amino acid residue at position 354 (position 332 in the sequence without signal peptide) is not a threonine residue; the amino acid residue at position 360 (position 338 in the sequence without signal peptide) is not an aspartate residue; the amino acid residue at position 361 (position 339 in the sequence without signal peptide) is not a threonine or an isoleucine residue; the amino acid residue at position 377 (position 355 in the sequence without signal peptide) is not a leucine residue; the amino acid residue at position 380 (position 358 in the sequence without signal peptide) is not a methionine residue; the amino acid residue at position 383 (position 361 in the sequence without signal peptide) is not a valine residue; the amino acid residue at position 384 (position 362 in the sequence without signal peptide) is not a valine residue; the amino acid residue at position 387 (position 365 in the sequence without signal peptide) is not an arginine residue; the amino acid residue at position 388 (position 366 in the sequence without signal peptide) is not a leucine residue; the amino acid residue at position 395 (position 373 in the sequence without signal peptide) is not a leucine residue; the amino acid residue at position 397 (position 375 in the sequence without signal peptide) is not a cysteine or a histidine residue; the amino acid residue at position 398 (position 376 in the sequence without signal peptide) is not an alanine residue; the amino acid residue at position 401 (position 379 in the sequence without signal peptide) is not a threonine residue; the amino acid residue at position 405 (position 383 in the sequence without signal peptide) is not a serine or a valine residue; the amino acid residue at position 406 (position 384 in the sequence without signal peptide) is not a leucine residue; the amino acid residue at position 412 (position 390 in the sequence without signal peptide) is not a glycine residue; the amino acid residue at position 416 (position 394 in the sequence without signal peptide) is not a leucine residue; the amino acid residue at position 417 (position 395 in the sequence without signal peptide) is not an alanine residue; the amino acid residue at position 420 (position 398 in the sequence without signal peptide) is not a methionine residue; the amino acid residue at position 423 (position 401 in the sequence without signal peptide) is not a serine residue; the amino acid residue at position 426 (position 404 in the sequence without signal peptide) is not a serine residue; the amino acid residue at position 429 (position 407 in the sequence without signal peptide) is not an alanine residue; the amino acid residue at position 430 (position 408 in the sequence without signal peptide) is not a methionine residue; the amino acid residue at position 432 (position 410 in the sequence without signal peptide) is not a cysteine or an aspartate residue; amino acid residue at position 434 (position 412 in the sequence without signal peptide) is not a proline residue; amino acid residue at position 435 (position 413 in the sequence without signal peptide) is not a lysine residue; amino acid residue at position 442 (position 420 in the sequence without signal peptide) is not a histidine residue; amino acid residue at position 451 (position 429 in the sequence without signal peptide) is not a proline residue; amino acid residue at position 456 (position 434 in the sequence without signal peptide) is not a histidine or a cysteine residue; amino acid residue at position 458 (position 436 in the sequence without signal peptide) is not a lysine residue; amino acid residue at position 460 (position 438 in the sequence without signal peptide) is not an arginine residue; amino acid residue at position 461 (position 439 in the sequence without signal peptide) is not a serine or an aspartate residue; amino acid residue at position 462 (position 440 in the sequence without signal peptide) is not a tryptophan or an arginine residue; amino acid residue at position 465 (position 443 in the sequence without signal peptide) is not a methionine or a leucine residue; amino acid residue at position 472 (position 450 in the sequence without signal peptide) is not a leucine residue; amino acid residue at position 473 (position 451 in the sequence without signal peptide) is not a threonine residue; amino acid residue at position 474 (position 452 in the sequence without signal peptide) is not a threonine residue; amino acid residue at position 479 (position 457 in the sequence without signal peptide) is not a serine residue; amino acid residue at position 482 (position 460 in the sequence without signal peptide) is not a lysine or a glycine residue; amino acid residue at position 484 (position 462 in the sequence without signal peptide) is not a leucine residue; amino acid residue at position 495 (position 473 in the sequence without signal peptide) is not a serine residue; amino acid residue at position 496 (position 474 in the sequence without signal peptide) is not a phenylalanine residue; and amino acid residue at position 497 (position 475 in the sequence without signal peptide) is not an arginine residue.

Also it is disclosed that, when a sTNALP is used in the bone targeted sALPs of the present invention, using the numbering of the human TNALP sequence, the amino acid at position 17 is not a phenylalanine residue; the amino acid at position 28 (position 11 in the sequence without signal peptide) is not a cysteine residue; the amino acid at position 33 (position 16 in the sequence without signal peptide) is not a valine residue; the amino acid at position 37 (position 20 in the sequence without signal peptide) is not a proline residue; the amino acid at position 40 (position 23 in the sequence without signal peptide) is not a valine residue; the amino acid residue at position 51 (position 34 in the sequence without signal peptide) is not a serine or a valine residue; the amino acid residue at position 62 (position 45 in the sequence without signal peptide) is not a leucine, an isoleucine or a valine residue; the amino acid residue at position 63 (position 46 in the sequence without signal peptide) is not a valine residue; the amino acid residue at position 68 (position 51 in the sequence without signal peptide) is not a methionine residue; the amino acid residue at position 71 (position 54 in the sequence without signal peptide) is not a cysteine, a serine, a proline or a histidine residue; the amino acid residue at position 72 (position 55 in the sequence without signal peptide) is not a threonine residue; the amino acid residue at position 75 (position 58 in the sequence without signal peptide) is not a serine residue; the amino acid residue at position 76 (position 59 in the sequence without signal peptide) is not an asparagine residue; the amino acid residue at position 100 (position 83 in the sequence without signal peptide) is not a methionine residue; the amino acid residue at position 108 (position 89 in the sequence without signal peptide) is not a leucine residue; the amino acid residue at position 111 (position 94 in the sequence without signal peptide) is not a threonine residue; the amino acid residue at position 112 (position 95 in the sequence without signal peptide) is not a serine residue; the amino acid residue at position 114 (position 97 in the sequence without signal peptide) is not a glycine residue; the amino acid residue at position 116 (position 99 in the sequence without signal peptide) is not a serine or a threonine residue; the amino acid residue at position 120 (position 103 in the sequence without signal peptide) is not an arginine residue; the amino acid residue at position 123 (position 106 in the sequence without signal peptide) is not a aspartate residue; the amino acid residue at position 128 (position 111 in the sequence without signal peptide) is not a methionine residue; the amino acid residue at position 129 (position 112 in the sequence without signal peptide) is not an arginine residue; the amino acid residue at position 132 (position 115 in the sequence without signal peptide) is not a threonine or a valine residue; the amino acid residue at position 134 (position 117 in the sequence without signal peptide) is not a histidine or an asparagine residue; the amino acid residue at position 136 (position 119 in the sequence without signal peptide) is not a histidine residue; the amino acid residue at position 148 (position 131 in the sequence without signal peptide) is not an alanine or an isoleucine residue; the amino acid residue at position 162 (position 145 in the sequence without signal peptide) is not a serine or a valine residue; the amino acid residue at position 167 (position 150 in the sequence without signal peptide) is not a methionine residue; the amino acid residue at position 170 (position 153 in the sequence without signal peptide) is not an aspartate residue; the amino acid residue at position 171 (position 154 in the sequence without signal peptide) is not a tyrosine or an arginine residue; the amino acid residue at position 176 (position 159 in the sequence without signal peptide) is not a threonine residue; the amino acid residue at position 177 (position 160 in the sequence without signal peptide) is not a threonine residue; the amino acid residue at position 179 (position 162 in the sequence without signal peptide) is not a threonine residue; the amino acid residue at position 181 (position 164 in the sequence without signal peptide) is not a leucine residue; the amino acid residue at position 184 (position 167 in the sequence without signal peptide) is not a tryptophane residue; the amino acid residue at position 189 (position 172 in the sequence without signal peptide) is not a glutamate residue; the amino acid residue at position 191 (position 174 in the sequence without signal peptide) is not a lysine or a glycine residue; the amino acid residue at position 192 (position 175 in the sequence without signal peptide) is not a threonine residue; the amino acid residue at position 193 (position 176 in the sequence without signal peptide) is not an alanine residue; the amino acid residue at position 201 (position 184 in the sequence without signal peptide) is not a tyrosine residue; the amino acid residue at position 203 (position 186 in the sequence without signal peptide) is not a glutamate residue; the amino acid residue at position 207 (position 190 in the sequence without signal peptide) is not a proline residue; the amino acid residue at position 211 (position 194 in the sequence without signal peptide) is not a aspartate residue; the amino acid residue at position 212 (position 195 in the sequence without signal peptide) is not a phenylalanine residue; the amino acid residue at position 218 (position 201 in the sequence without signal peptide) is not a threonine residue; the amino acid residue at position 220 (position 203 in the sequence without signal peptide) is not a valine or an alanine residue; the amino acid residue at position 221 (position 204 in the sequence without signal peptide) is not a valine residue; the amino acid residue at position 223 (position 206 in the sequence without signal peptide) is not a tryptophane or a glutamine residue; the amino acid residue at position 224 (position 207 in the sequence without signal peptide) is not a glutamate residue; the amino acid residue at position 226 (position 209 in the sequence without signal peptide) is not a threonine residue; the amino acid residue at position 235 (position 218 in the sequence without signal peptide) is not a glycine residue; the amino acid residue at position 246 (position 229 in the sequence without signal peptide) is not a serine residue; the amino acid residue at position 249(position 232 in the sequence without signal peptide) is not a valine residue; the amino acid residue at position 264 (position 247 in the sequence without signal peptide) is not an arginine residue; the amino acid residue at position 272 (position 255 in the sequence without signal peptide) is not a cysteine, a leucine or a histidine residue; the amino acid residue at position 275 (position 258 in the sequence without signal peptide) is not a proline residue; the amino acid residue at position 289 (position 272 in the sequence without signal peptide) is not a phenylalanine residue; the amino acid residue at position 291 (position 274 in the sequence without signal peptide) is not a lysine residue; the amino acid residue at position 292 (position 275 in the sequence without signal peptide) is not a threonine residue; the amino acid residue at position 294 (position 277 in the sequence without signal peptide) is not a tyrosine or an alanine residue; the amino acid residue at position 295 (position 278 in the sequence without signal peptide) is not a valine, a threonine or an isoleucine residue; the amino acid residue at position 297 (position 280 in the sequence without signal peptide) is not an aspirate residue; the amino acid residue at position 298 (position 281 in the sequence without signal peptide) is not a lysine residue; the amino acid residue at position 299 (position 282 in the sequence without signal peptide) is not a proline residue; the amino acid residue at position 306 (position 289 in the sequence without signal peptide) is not a valine residue; the amino acid residue at position 307 (position 290 in the sequence without signal peptide) is not a serine or a leucine residue; the amino acid residue at position 311 (position 294 in the sequence without signal peptide) is not a lysine residue; the amino acid residue at position 326 (position 309 in the sequence without signal peptide) is not an arginine residue; the amino acid residue at position 327 (position 310 in the sequence without signal peptide) is not a cysteine, a glycine or a leucine residue; the amino acid residue at position 328 (position 311 in the sequence without signal peptide) is not a leucine residue; the amino acid residue at position 334 (position 317 in the sequence without signal peptide) is not an aspartate residue; the amino acid residue at position 339 (position 322 in the sequence without signal peptide) is not an arginine or a glutamate residue; the amino acid residue at position 348 (position 331 in the sequence without signal peptide) is not a threonine residue; the amino acid residue at position 354 (position 337 in the sequence without signal peptide) is not an aspartate residue; the amino acid residue at position 355 (position 338 in the sequence without signal peptide) is not a threonine or an isoleucine residue; the amino acid residue at position 371 (position 354 in the sequence without signal peptide) is not a leucine residue; the amino acid residue at position 374 (position 357 in the sequence without signal peptide) is not a methionine residue; the amino acid residue at position 377 (position 360 in the sequence without signal peptide) is not a valine residue; the amino acid residue at position 378 (position 361 in the sequence without signal peptide) is not a valine residue; the amino acid residue at position 381 (position 364 in the sequence without signal peptide) is not an arginine residue; the amino acid residue at position 382 (position 365 in the sequence without signal peptide) is not a leucine residue; the amino acid residue at position 389 (position 372 in the sequence without signal peptide) is not a leucine residue; the amino acid residue at position 391 (position 374 in the sequence without signal peptide) is not a cysteine or a histidine residue; the amino acid residue at position 392 (position 375 in the sequence without signal peptide) is not an alanine residue; the amino acid residue at position 395 (position 378 in the sequence without signal peptide) is not a threonine residue; the amino acid residue at position 399 (position 382 in the sequence without signal peptide) is not a serine or a valine residue; the amino acid residue at position 400 (position 383 in the sequence without signal peptide) is not a leucine residue; the amino acid residue at position 406 (position 389 in the sequence without signal peptide) is not a glycine residue; the amino acid residue at position 410 (position 393 in the sequence without signal peptide) is not a leucine residue; the amino acid residue at position 411 (position 394 in the sequence without signal peptide) is not an alanine residue; the amino acid residue at position 414 (position 397 in the sequence without signal peptide) is not a methionine residue; the amino acid residue at position 417 (position 400 in the sequence without signal peptide) is not a serine residue; the amino acid residue at position 420 (position 403 in the sequence without signal peptide) is not a serine residue; the amino acid residue at position 423 (position 406 in the sequence without signal peptide) is not an alanine residue; the amino acid residue at position 424 (position 407 in the sequence without signal peptide) is not a methionine residue; the amino acid residue at position 426 (position 409 in the sequence without signal peptide) is not a cysteine or an aspartate residue; amino acid residue at position 428 (position 411 in the sequence without signal peptide) is not a proline residue; amino acid residue at position 429 (position 412 in the sequence without signal peptide) is not a lysine residue; amino add residue at position 436 (position 419 in the sequence without signal peptide) is not a histidine residue; amino acid residue at position 445 (position 428 in the sequence without signal peptide) is not a proline residue; amino acid residue at position 450 (position 433 in the sequence without signal peptide) is not a histidine or a cysteine residue; amino acid residue at position 452 (position 435 in the sequence without signal peptide) is not a lysine residue; amino acid residue at position 454 (position 437 in the sequence without signal peptide) is not an arginine residue; amino acid residue at position 455 (position 438 in the sequence without signal peptide) is not a serine or an aspartate residue; amino acid residue at position 456 (position 439 in the sequence without signal peptide) is not a tryptophane or an arginine residue; amino acid residue at position 459 (position 442 in the sequence without signal peptide) is not a methionine or a leucine residue; amino acid residue at position 466 (position 449 in the sequence without signal peptide) is not a leucine residue; amino acid residue at position 467 (position 450 in the sequence without signal peptide) is not a threonine residue; amino acid residue at position 468 (position 451 in the sequence without signal peptide) is not a threonine residue; amino acid residue at position 473 (position 456 in the sequence without signal peptide) is not a serine residue; amino acid residue at position 476 (position 459 in the sequence without signal peptide) is not a lysine or a glycine residue; amino acid residue at position 478 (position 461 in the sequence without signal peptide) is not a leucine residue; amino acid residue at position 489 (position 472 in the sequence without signal peptide) is not a serine residue; amino add residue at position 490 (position 473 in the sequence without signal peptide) is not a phenylalanine residue; and amino acid residue at position 491 (position 474 in the seq ence without signal peptide) is not an arginine residue. It is also disclosed that, one or more Xs are defined as being any of the amino acids found at that position in the sequences of the alignment or a residue that constitutes a conserved or semi-conserved substitution of any of these amino acids. It is further disclosed that, Xs are defined as being any of the amino acids found at that position in the sequences of the alignment. For instance, the amino acid residue at position 51 (position 34 in the sequence without signal peptide) is an alanine or a valine residue; the amino acid residue at position 177 (position 160 in the sequence without signal peptide) is an alanine or a serine residue; the amino acid residue at position 212 (position 195 in the sequence without signal peptide) is an isoleucine or a valine residue; the amino acid residue at position 291 (position 274 in the sequence without signal peptide) is a glutamic acid or an aspartic acid residue; and the amino acid residue at position 374 (position 357 in the sequence without signal peptide) is a valine or an isoleucine residue.

It is further disclosed that, the sALP fragment in the bone targeted fusion protein consists of any one of the fragments of a consensus sequence derived from an alignment of human ALP isozymes and TNALPs from various mammalian species corresponding to amino acid residues 18-498, 18-499, 18-500, 18-501, 18-502, 18-503, 18-504, or 18 to 505 of human TNALP. These consensus fragments are amino acid residues 23 to 508, 23 to 509, 23 to 510, 23 to 511, 23 to 512, 23 to 513, 23 to 514 and 23 to 515 of SEQ ID NO: 15, respectively. In these consensus fragments, X is any amino acid except an amino acid corresponding to a pathological mutation at that position of human TNALP as reported in Table 1. It is further disclosed that, these consensus fragments are amino acid residues 23 to 508, 23 to 509, 23 to 510, 23 to 511, 23 to 512, 23 to 513, 23 to 514 and 23 to 515 of SEQ ID NO: 18, respectively. In these consensus fragments, X is any amino acid found at that position in the ALP of either one of the species and human ALP isozymes of the alignment from which the consensus is derived but is not an amino acid corresponding to a pathological mutation at that position of human TNALP as reported in Table 1 (See Figure 30).

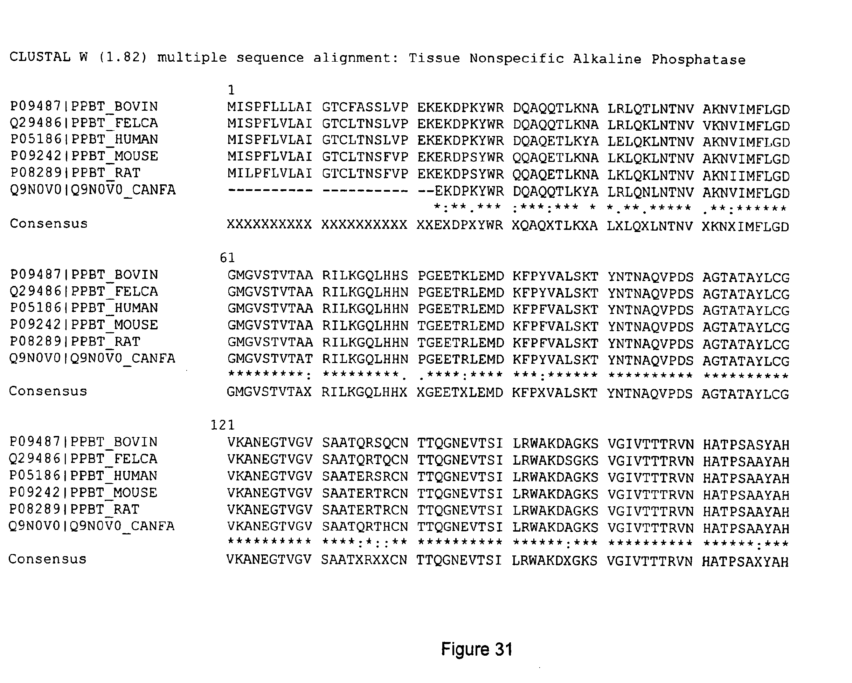

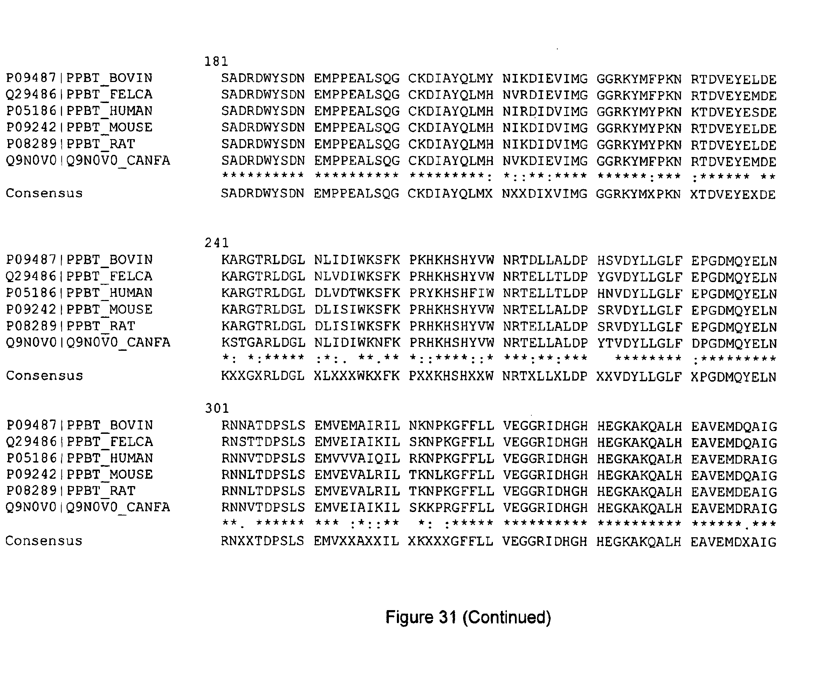

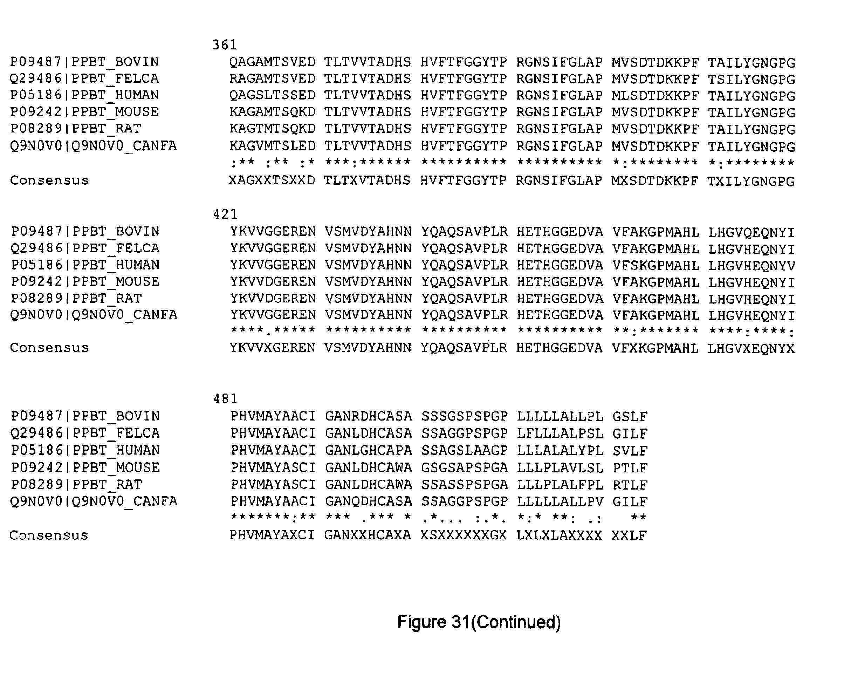

It is further disclosed that, the sALP fragment in the bone targeted fusion protein of the present invention consists of any of the fragments of a consensus sequence derived from an alignment of TNALPs from various mammalian species corresponding to amino acid residues 18-498, 18-499, 18-500, 18-501, 18-502, 18-503, 18-504, and 18 to 505 of human TNALP. These consensus fragments are amino acid residues 18-498, 18-499, 18-500, 18-501, 18-502, 18-503, 18-504, and 18 to 505 of SEQ ID NO: 16, respectively. In these consensus fragments, X is any amino acid except an amino acid corresponding to a pathological mutation at that position of human TNALP as reported in Table 1 It is further disclosed that, these consensus fragments are amino acid residues 18-498, 18-499, 18-500, 18-501, 18-502, 18-503, 18-504, and 18 to 505 of SEQ ID NO: 19, respectively. In these consensus fragments, X is any amino acid found at that position in the TNALP of either one of the species of the alignment from which the consensus is derived but is not an amino acid corresponding to a pathological mutation at that position of human TNALP as reported in Table 1 (See Figure 31).

**Table 1 : Pathological mutations in human TNALP**

| Total number of mutations 188 | | | | | | | | | | |
|---|---|---|---|---|---|---|---|---|---|---|
| Exon | Base change | Amino acid change | | Reference | Clinical form in patient | Genotype of patient | % WT | ref. | E.coli | |
| | | Non-standardized nomenclature | Standardized nomenclature | | | | | | | |
| 1 | c.-195C>T | | | Taillandier et al. 2000 | perinatal | C.-195C>T/C184 Y | | | na | Affects transcription start site |
| 2 | c.17T>A | L-12X | p.L6X | Taillandier et al. 2000 | childhood | L-12X/? | | | na | Nonsense mutation |
| 2 | c.50C> T | S-1F | p.S17F | Mornet et al. 1998 | infantile | S-1F/G58S | 19.0 | 1 | na | I |
| 3 | c.83A>G | Y11C | p.Y28C | Taillandier et al. 2001 | infantile | Y11C/R119H | 7.2 | 2 | - | |
| 3 | c.98C>T | A16V | p.A33V | Henthorn et al. 1992 | childhood | A16V/Y419H | | | - | |
| 3 | c.110T>C | L20P | p.L37P | Versailles lab oct. 2003 | perinatal | L20P/L20P | | | + | |
| 3 | c.119C>T | A23V | p.A40V | et al. 1998 | perinatal | A23V/G456S | 2.3 | 1 | + | |
| 3 | c.132C>T | Q27X | p.Q44X | Mornet E, unpublished | perinatal | Q27X/c.662in sG | | | na | Nonsense mutation |
| 3 | c.151G>T | A34S | p.A51S | Mumm et al. 2002 | infantile | A34S/T117H | | | + | |
| 3 | c.152G>T | A34V | p.A51V | Taillandier et al. 2001 | infantile | A34V/V442M | | | + | |
| 4 | c.184A>T | M45L | p.M62L ' | Taillandier et al. 1999 | infantile | M45Uc.1172d eIC | 27.4 | 1 | + | |
| 4 | c.184A>G | M45V | p.M62V | Spentchian et al. 2003 | infantile | M45V/M45V | | | + | |
| 4 | c.186G>C | M451 | p.M62II | Taillandier et al. 2005 | childhood | M45l/E174K | 0 | 16 | + | |
| 4 | c.187G>C | G46R | I p.G63R | Spentchian et al. 2003 | infantile | G46R/G46R | | | + | |
| 4 | c.188G>T | G46V | p.G63V | Lia-Baldini et al. 2001 | I infantile | G46V/N | 0.8 | 3 | + | |
| 4 | c.203C>T | T51M | p.T68M ((_{'} | Orimo et al. 2002 | I childhood | T51M/A160T | 5.2 | 4 | + | |
| 4 | c.211C>T | R54C | p.R71C | et al. 1992 | infantile | R54C/D277A | 0 | 17 | + | |
| 4 | c.211C>A | R54S | p.R71S | Orimo et al. 2002 | childhood | R54S/? | 2.9 | 4 | + | I |
| 4 | c.212G>C | R54P | p.R71P | Henthorn et al. 1992 | perinatal | R54P/Q190P | | | + | |
| 4 | c.212G>A | R54H | p.R71H | Taillandier et al. 2001 | perinatal | A23V/R54H | | | + | |
| 4 | c.219T>C | I55T | p.I72T | Versailles lab oct. | 2004 odonto | I55T/N | | | - | |
| 4 | c.223G>A | G58S | p.G75S | Mornet et al. 1998 | infantile | S-1F/G58S | 3.5 | 1 | + | |
| 4 | c.227A>G | Q59R | p.Q76R | Mornet et al. 2001 | infantile | Q59R/T117N | | | - | |
| IVS4 | c.298-2A>G | | | Taillandier et al. 2000 | perinatal | c.298-2A>G/c.997+3 A>C | | | na | This mutation affects splicing and not coding sequence |
| 5 | c.299C>T | T83M | p.T100M | Mornet et al. 2001 | infantile | 83M/E174K | | | + | |
| 5 | c.303_311del | N85_N87del | p.N102_N 104del | Versailles lab Jul 2007 | perinatal | c.303_311del/ G474R | | | na | Deletion |
| 5 | c.323C>T | P91L | p.P108L | IHerasse et al. 2003 | odonto | P91L/N | 0.4 | unp. | - | |
| 5 | c.331G>A | A94T | p.A111T | Gosekl-Sone et al. 1998 et al. | odonto | A94T/? | | | + | |
| 5 | c.334G>A | G95S | p.G112S | Witters et al. 2004 | infantile | G95S/R374C | | | - | |
| 5 | c.340G>A | A97T | p.A114T | Mumm et al. 2001 | infantile | A97T/D277A | | | + | |
| 5 | c.341C>G | A97G | p.A114G | Draquet et al. 2004 | perinatal | A97G+c.348_ 349insACCGT C /G309R | | | + | |
| 5 | c.348_349insA CCGTC | | | Draquet et al. 2004 perinatal Draquet et al. 2004 | perinatal | A97G+c.348_ 349insACCGT C /G309R | | | na | Two missense mutations and insertion |
| 5 | c.346G>T | A99S | p.A116S | Versailles lab Jul 2007 | adult | A99S/N400S | | | + | |
| 5 | c.346G>A | A99T | p.A116T | Hu et al. 2000 | adult | A99T/N | 0.8 | 3 | + | |
| 5 | c.358G>A | G103R | p.G120R | Mornet et al. 1998 | perinatal | G103R/648+1 G>A | | | + | |
| 5 | c.368C>A | A106D | p.A123D | Spentchian et al. 2006 | perinatal | A106D/S249_ H250del | | | - | |
| 5 | c.382G>A | V111M | p.V128M | Mumm et al. 2002 | perinatal | V111M/R206 W | | | - | |
| 5 | c.385G>A | G112R | p.G129R | Mornet et al. 1998 | perinatal | G112R/G474 R | | | + | |
| 5 | c.388_391delG | | | Spentchian et al 2003 Spentchian et al. 2003 TAA 91delGTAA | perinatal | E294K1388_3 E294K/388_3 | | | na | Frameshift mutation |
| 5 | c.389delT | | | Spentchian et al. 2003 Spentchian et al. 2003 | perinatal | c.389delT/c.3 c.389delT/c.3 | | | na | Frameshift mutation |
| 5 | c.392delG | | | Mumm et al. 2002 | perinat/infant | c.392delG/A3 | | | na | Frameshift mutation |
| 5 | c.394G>A | A115T | p.A132T | Versailles lab Jul 2006 | adult | A115T/E174K | | | | |
| 5 | c.395C>T | A115V | p.A132V | Watanabe et al. 2001 | adult | A115V/? | 16.9 | 14 | - | |
| 5 | c.400_401AC> | T117H | p.T134H | Mumm et al. 2002 | perinatal | T117H/F310d el | | | - | |
| 5 | c.401C>A | T117N | p.T134N | Taillandier et al. 2000 | perinatal | T117N/T117N 20.5 | | 5 | - | |
| 5 | c.406C>T | R119C | p.R136C | Versailles lab oct. 2003 | odonto | R119C/R119 H | | | - | |
| 5 | c.407G>A | R119H | p.R136H | Taillandier et al. 1999 | infantile | R119H/G145 V | 33.4 | 1 | - | |
| 5 | c.442A>G | T131A | p.T148A | Michigami et al. 2005 | perinatal | T131A/? | | | - | |
| 5 | c.443C>T | T1311 | p.T148I | Spentchian et al. 2003 | infantile | T1311I/G145S | | | - | |
| 6 | c.480delT | | | Versailles lab. Jan. 2008 | perinatal | c.480delT/R2 06W | | | na | deletion |
| 6 | c.484G>A | G145S | p.G162S | Spentchian et al. 2003 | infantile | T131l/G145S | | | + | |
| 6 | c.485G>T | G145V | p.G162V | Taillandier et al. 1999 | infantile | 119H/G145 V | 1.3 | 1 | + | |
| 6 | c.500C>T | T150M | p.T167M | Versailles lab oct. 2003 | infantile | T150M/E174K | 0 | | + | |
| 6 | c.508A>G | N153D | p.N170D | Mornet et al. 1998 | perinatal | N153D/N153 D | 0 | 13 | - | |
| 6 | c.511C>T | H154Y | p.H171Y | Taillandier et al. 1999 | infantile | H154Y/E174K | 2.1 | 1 | - | |
| 6 | c.512A>G | H154R | p.H171R | Mornet E, unpublished | adult | H154R/E174K | | | - | |
| 6 | c.526G>A | A159T | p.A176T | Taillandier et al. 2000 | childhood | A159T/R229S | 45.4 | 5 | + | |
| 6 | c.529G>A | A160T | p.A177T | Goseki-Sone et al. 1988 | adult | A160T/F310L | 83.8 | 4 | - | |
| 6 | c.535G>A | A162T | p.A179T | Weiss et al. 1988 | perinatal | A162T/A162T | 18 | 6 | + | |
| 6 | c.542C>T | S164L | p.S181L | Lia-Baldini et al. 2001 | infantile | S164L/del(ex1 | 1.3 | 3 | - | |
| 6 | c.544delG | | | Taillandier et al. 1999 | perinatal | G232V/544del | | | na | Frameshift mutation |
| 6 | c.550C>T | R167W | p.R184W | Mornet et al. 1998 | perinatal | R167W/W253 R167W/W253 | 0.6 | 3 | + | |
| 6 | c.567C>A | D172E | p.D189E | Spentchian et al. 2003 | perinatal | D172E/D172E | | | - | |
| 6 | c_{.}568_570delA | N173del | p.N190del | Michigami et al. 2005 AC 173del | perinatal | c.1559delT/N | | | **-** | Deletion of 1 a.a. |
| 6 | c.571G>A | E174K | p.E191K | Henthorn et al. 1992 | infantile | E174K/D361V | 88.0 | 1 | - | |
| 6 | c.572A>G | E174G | p.E191G 1998 | Goseki-Sone et al. | odonto | E174G/c.1559 delT | | | - | |
| 6 | c.575T>C | M175T | p.M192T | Versailles lab Jul 2007 | infantile | M175T/E294K | | | - | |
| 6 | c.577C>G | P176A | p.P193A | Mumm et al. 2002 | adult | A97T/P176A | | | + | |
| 6 | c.602G>A | C184Y | p.C201Y | Taillandier et al. 1999 | c.-perinatal | 195C>T/C184 Y | | | - | |
| 6 | c.609C>G | D186E | p.D203E | Versailles lab oct. | 2004 perinatal | D186E/D186E | | | - | |
| 6 | c.620A>C | Q190P | p.Q207P | Henthorn et al. 1992 | perinatal | R54P/Q190P | | | + | |
| 6 | c.631A>G | N194D | p.N211D | Taillandier et al. 2001 | infantile | A99T/N194D | | | + | |
| 6 | c.634A>T | I195F | p.I212F | Souka et al. 2002 | perinatal | I195F/E337D | | | - | |
| IVS6 | c.648+1G>T | | | Brun-Heath et al. 2005 | perinatal | c.648+1G>T/ D277A | | | | Affects splicing |
| IVS6 | c.648+1G>A | | | Mornet et al. 1998 | perinatal | G103R/c.648+ 1G>A | | | na | Affects splicing |
| IVS6 | c.649- | | | Versailles lab Jul 2006 1_3delinsAA | perinatal | c.649-1_3delinsAA/c 1_3delinsAA | | | | Frameshift mutation |
| 7 | c.653T>C | I201T | p.I218T | Utsch et al., 2005, contact | perinatal | I201T/R374C | 3.7 | unp. | - | |
| 7 | 659G>T | G203V | p.G220V | Taillandier et al. 2001 | odonto | E174K/G203V | | | + | |
| 7 | 659G>C | G203A | p.G220A | Spentchian et al. 2003 | perinatal | G203A/G203 | | | + | |
| 7 | 662insG | | | Mornet E, unpublished | perinatal | Q27X/662ins | | | na | Frameshift mutation |
| 7 | c.662delG | | | Spentchian et al. 2003 | perinatal | R255L/c.662d | | | na | Frameshift mutation |
| 7 | c.662G>T | G204V | p.G221V | Versailles lab oct. 2004 | perinatal | G204V/M338 G204V/M338 | | | + | |
| 7 | c.667C>T | R206W | p.R223W | Mornet et al. 1998 | perinatal | R206W/? | 2.8 | 3 | - | |
| 7 | c.668G>A | R206Q | p.R223Q | Mumm et al. 2002 | perinatal | R206Q/deletion | | | - | |
| 7 | c.670A>G | K207E | p.K224E | Mochizuki et al. 2000 | infantile | K207E/G409C | 43 | 15 | + | |
| 7 | c.677T>C | M209T | p.M226T | Baumgartner-Sigl et al. 2007 | infantile | M209T/T354I | | | - | |
| 7 | c.704A>G | E218G | p.E235G | Taillandier et al. 2001 | adult | E218G/A382S | 3.6 | 7 | + | |
| 7 | c.738G>T | R229S | p.R246S | Taillandier et al. 2000 | childhood | A159T/R229S | 4.4 | 5 | - | |
| 7 | c.746G>T | G232V | p.G249V | Fedde et al. 1996 | perinatal | G232V/N | 34.5 | 3 | + | |
| 7 | c.971A>G | K247R | p.K264R | Versailles lab Jan.2007 | perinatal | K247R/D361V | | | - | |
| 8 | c.797_802del | S249_H250del | p.S266_H | Spentchian et al. 2006 267del | perinatal | A106D/S249_ H250del | | | | Deletion of 2 a.a. |
| 8 | c.809G>A | W253X | p.W270X | Mornet et al. 1998 | perinatal | R167W/WV253 | | | na | Nonsense mutation |
| 8 | C.814C>T | R255C | p.R272C | Spentchian et al. 2006 | perinatal | R255C/T117H | | | - | |
| 8 | c.815G>T | R255L | p.R272L | Spentchian et al. 2003 | perinatal | R255L/c.662d eIG | | | - | |
| 8 | c.815G>A | R255H | p.R272H | Brun-Heath et al. 2005 | infantile | R255H/R255 H | 6.8 | 16 | - | |
| 8 | c.824T>C | L258P | p.L275P | Orimo et al. 2002 | childhood | L258P/A160T | 3.3 | 4 | - | |
| 8 | c.853_854insG | Y268X | p.Y285X | Michigami et al. 2005 | perinatal | c1559delT/Y2 68X | | | na | Nonsense mutation |
| IVS8 | c.862+5G>A | | | Taillandier et al. 1999 | infantile | c.862+5G>A/c 862+5G>A | | | na | Affects splicing |
| 9 | c.865C>T | L272F | p.L289F | Sugimoto et al. 1998 | infantile | L272F/? | 50 | 8 | - | |
| 9 | c.871G>A | E274K | p.E291K | Mornet et al. 1998 | infantile | E174K/E274K | 8.3 | 1 | - | |
| 9 | c.871G>T | E274X | p.E291X | Taillandier et al. 2000 | perinatal | A94T/E274X | | | - | Nonsense mutation |
| 9 | c.874C>A | P275T | p.P292T | Brun-Heath et al. 2005 | infantile | P275T/A16V | 4.0 | 16 | + | |
| 9 | c.876_881delA GGGGA | G276_D277del | | Spentchian et al. 2003 | perinatal | el/c.962delG | | | na | |
| 9 | c.880G>T | D277Y | p.D294Y | Taillandier et al. 2001 | infantile | A159T/D277Y | | | - | |
| 9 | c.881A>C | D277A | p.D294A | Henthorn et al. 1992 | infantile | R54C/D277A | 0 | 17 | - | |
| 9 | c.883A>G | M278V | p.M295V | Mornet et al. 2001 | childhood | E174K/M278V | | | - | |
| 9 | c.884T>C | M278T | p.M295T | Brun-Heath et al. 2005 | perinatal | M278T/R206 | 8.5 | 16 | - | |
| 9 | c.885G>A | M278I | p.M295I | Michigami et al. 2005 | perinatal | M278I/c.1559 delT | | | - | |
| 9 | c.889T>G | Y280D | p.Y297D | Brun-Heath et al. 2005 | childhood | R119H/Y280D | 1.3 | 16 | - | |
| 9 | c.892G>A | E281K | p.E298K | Orimo et al. 1994 | infantile | E281K/1559d | | | - | |
| 9 | c.896T>C | L282P | p.L299P | Versailles lab oct. | 2003 infantile | L282P/L282P | 9.7 | 15 | - | |
| 9 | c.917A>T | D289V | p.D306V | Taillandier et al. 1999 | infantile | D289V/D289V | 0 | 12 | - | |
| 9 | c.919C>T | P290S | p.P307S | Versailles lab oct. | 2004 infantile | P290S/M450T | | | + | |
| 9 | c.920C>T | P290L | p.P307L | Versailles lab Jul. | 2006 childhood | P290L/S164L | | | | |
| 9 | c.928_929delTC | | | Brun-Heath et al. 2005 | perinatal | T394A/c.928_ 929delTC | | | | Frameshift mutation |
| 9 | c.931G>A | E294K | p.E311K | Spentchian et al. 2003 | perinatal | E294K/c.388_ 391delGTAA | | | | |
| 9 | c.962delG | | | Spentchian et al. 2003 | perinatal | G276_D277d el/c.962delG | | | na | Frameshift mutation |
| 9 | c.976G>C | G309R | p.G326R | Litmanovitz et al. 2002 | perinatal | G309R/E274K | | | + | |
| 9 | c.981_983delC TT | F310del | p.F327del | Orimo et al. 1997 9delT | infantile | F310del/c.155 | -10 | 15 | + | Amino acid deletion |
| 9 | c.979T>G | F310C | p.F327C | Mornet et al. 2001 | perinatal | ((T117N/F310C | | | + | |
| 9 | c.979_980TT GG | F310G | p.F327G | Taillandier et al. 2001 | adult | E174K/F310G | | | + | |
| 9 | c.979T>C | F310L | p.F327L | Ozono et al. 1996 | infantile | F310/G439R | 72 | 9 | + | |
| 9 | c.982T>A | F311L | p.F328L | Michigami et al. 2005 | perinatal non-lethal | F311L/T83M | -10 | 15 | + | |
| IVS9 | c.997+2T>A | | | Taillandier et al. 2000 | perinatal | c.997+2T>A/C 472S | | | na | Affects splicing |
| IVS9 | c.997+2T>G | | | Brun-Heath et al. 2005 | perinatal | c.997+2T>G/c c.997+2T>G/c 997+2T>G | | | | Affects splicing |
| IVS9 | c.997+3A>C | | | Mornet et al. 1998 | perinatal | c.997+3A>C/c 997+3A>C | | | na | Affects splicing |
| IVS9 | c.998-1G>T | | | Taillandier et al. 2001 | perinatal | E174K/c.998-1G>T | | | na | Affects splicing |
| 10 | c.1001G>A | G317D | p.G334D | Greenberg et al. 1993 | perinatal | D317D/G317D | 0 | 10 | - | |
| 10 | c.1015G>A | G322R | p.G339R | Mumm et al. 2002 | perinatal | G322R/A159T | | | - | |
| 10 | c.1016G>A | G322E | p.G339E | Versailles lab oct. 2004 | infantile | G322E/V111 M | | | - | |
| 10 | c.1042G>A | A331T | p.A348T | Taillandier et al. 2000 | infantile | E174K/A331T | 33.2 | 5 | - | |
| 10 | c.1044_1055de I | L332_A335del | p.L349_A3 52del | Spentchian et al. 2006 | perinatal | L332_A335del /G474R | | | | Deletion of 4 a.a. |
| 10 | c.1062G>C | E337D | p.E354D | Souka et al. 2002 | perinatal | I195F/E337D | | | + | |
| 10 | c.1064A>C | M338T | p.M355T | Versailles lab oct. 2004 | perinatal | G204V/M338 T | | | - | |
| 10 | c.1065G>A | M338I | p.M3551 | Versailles lab. Jan. 2008 | infantile | M338I/R374C | | | - | |
| 10 | c.1101_1103de ICTC | S351del | p.S368del | Versailles lab oct. 2004 | perinatal | c.1101_1103d elCTC/T372I | | | | Deletion of 1 a.a. |
| 10 | c.1112C>T | T354I | p.T371I | gaumgartner-Sigl et al. 2007 | infantile | M209T/T354I | | | - | |
| 10 | c.1120G>A | V357M | p.V374M | Versailles lab oct. 2004 | adult | V357M/E281K | | | + | |
| 10 | c.1130C>T | A360V | p.A377V | Mornet et al. 2001 | perinatal | A360V/A360V | | | + | |
| 10 | c.1133A>T | D361V | p.D378V | Henthorn et al. 1992 | infantile | E174K/D361V | 1.2 | 3 | + | |
| 10 | c.1142A>G | H364R | p.H381 R | Taillandier et al. 2000 | infantile | A23V/H364R | | | + | |
| 10 | c.1144G>A | V365I | p.V3821 | Goseki-Sone et al. 1998 | childhood | F310L/V365I | 0 | 11 | + | |
| 10 | c.1166C>T | T3721 | p.T389I | Versailles lab oct. 2004 | perinatal | T372I/S351 del | | | - | |
| 10 | c.1171C>T | R374C | p.R391C | Zurutuza et al. 1999 | childhood | E174K/R374C | 10.3 | 1 | - | |
| 0 | c.1172G>A | R374H | p.R391H | Orimo et al. 2002 | childhood | R374H/? | 3.7 | 4 | - | |
| 10 | c.1172delC | | | Taillandier et al. 1999 | infantile | M45L/c.1172delC | | | na | Frameshift mutation |
| 10 | c.1175G>C | G375A | p.G392A | Versailles lab. Jan. 2008 | perinatal | G375A/R119 C | | | - | |
| 10 | c.1182T>C | I378T | p.I395T | Versailles lab Jul. | 2006 perinatal | I378T/E174K | | | | |
| 11 | c.1195G>T | A382S | p.A399S | Taillandier et al. 2001 | adult | E218G/A382S | | | - | |
| 11 | c.1196C>T | A382V | p.A399V | Spentchian et al. 2006 | adult | A382V/A16V | | | - | |
| | 11 c.1199C>T | P383L | p.P400L | Spentchian et al. 2006 | infantile | P383L/P383L | | | + | |
| 11 | c.1214_1215de ICA | | | Versailles lab Jul. 2006 | adult | c.1214_1215d elCA/E 174K | | | | Frameshift mutation |
| | 11 c.1216 1219de | | | Brun-Heath et al. | 2005 perinatal | c.1216 1219d | | | | |
| | IGACA | | | | | eIGACA/? | | | | |
| 11 | c.1217A>G | D389G | p.D406G | Taillandier et al. 2000 | odonto. | D389G/R433 H | 14.9 | 5 | + | |
| 11 | c.1228T>C | F393L | p.F410L | Versailles lab oct. 2004 | infantile | F393L/E174K | | | - | |
| 11 | c.1231A>G | T394A | p.T411A | Brun-Heath et al. 2005 | perinatal | T394A/c.926_ 927delTC | 0.3 | 16 | - | |
| 11 | c.1240C>A | L397M | p.L414M | Mumm et al. 2002 | perinatal | L397M/D277A | | | - | |
| 11 | c.1250A>G | N400S | p.N417S | Sergi et al. 2001 | perinatal | N400S/c.648+ 1G>A | 3 | unp. | + | |
| 11 | c.1256delC | | | Taillandier et al. 2000 | perinatal | c.1256delC/? | | | na | Frameshift mutation |
| 11 | c.1258G>A | G403S | p.G420S | Glaser et al. 2004 | perinatal | G403/G403S | 0.4 | unp | - | |
| 11 | c.1268T>C | V406A | p.V423A | Taillandier et al. 2001 | perinatal | A99T/V406A | 15.7 | 2 | - | |
| 11 | c.1270G>A | V407M | p.V424M | Versailles lab jan. 2007 | adult | V407MN407M | | | - | |
| 11 | c.1276G>T | G409C | p.G426C | Mochizuki et al. 2000 | infantile | K207A/G409C 18.5 | | 15 | - | |
| 11 | c.1277G>A | G409D | p.G426D | Mumm et al. 2002 | childhood | G409D/E174K | | | - | |
| 11 | c.1282C>T | R411X | p.R428X | Taillandier et al. 1999 | perinatal | R411X/R411X | | | na | Nonsense mutation |
| 11 | c.1283G>C | R411P | p.R428P | Spentchian et al. 2006 | perinatal | R411P/c.997+ 2T>A | | | - | |
| 11 | c.1285G>A | E412K | p.E429K | Versailles lab Jul. | 2006 odonto. | E412K/? | | | | |
| 11 | c.1306T>C | Y419H | p.Y436H | Henthorn et al. 1992 | childhood | A16V/Y419H | | | na | |
| 12 | c.1333T>C | S428P | p.S445P | Mornet et al. 1998 | infantile | S428P/? | 2.1 | 1 | - | |
| 12 | c.1349G>A | R433H | p.R450H | Taillandier et al. 2000 | odonto. | D389G/R433H | | | - | |
| 12 | c.1348C>T | R433C | p.R450C | Mornet et al. 1998 | infantile | R433C/R433C | 4.0 | 1 | - | |
| 12 | c.1354G>A | E435K | p.E452K | Spentchian et al. 2003 | perinatal | A94T/E435K | | | + | |
| 12 | c.1361A>G | H437R | p.H454R | Versailles lab oct. 2003 | childhood | E174K/H437R | | | + | |
| 12 | c.1363G>A | G438S | p. G455S | Draguet et al. 2004 | adult | G438S/G474 R | | | - | |
| 12 | c.1364G>A | G438D | p. G455D | Versailles lab jan. 2007 | perinatal | G438D/G438 D | | | - | |
| 12 | c.1366G>T | G439W | p. G456W | Versailles lab oct. 2003 | childhood | G439W/? | | | + | |
| 12 | c.1366G>A | G439R | p. G456R | Ozono et al. 1996 | infantile | G439R/? | 1.5 | unp. | + | |
| 12 | c.1375G>A | V442M | p. V459M | Taillandier et al. 2000 | infantile | A34V/V442M | | | + | |
| 12 | c.1375G>T | V442L | p. V459L | Versailles lab oct. 2004 | perinatal | V442L/E435K | | | - | |
| 12 | c.1396C>T | P449L | p. P466L | Versailles lab oct. 2003 | perinatal | P449L/? | | | + | |
| 12 | c.1400T>C | M450T | p. M467T | Versailles lab oct. 2004 | infantile | M450T/P290S | | | - | |
| 12 | c.1402G>A | A451T | p. A468T | Spentchian et al. 2003 | perinatal | A451T/A451T | | | + | |
| 12 | c.1417G>A | G456S | p. G473S | Mornet et al. 1998 | perinatal | A23V/G456S | | | + | |
| 12 | c.1426G>A | E459K | p. E476K | Taillandier et al. 1999 | perinatal | A94T/E459K | | | + | |
| 12 | c.1427A>G | E459G | p. E476G | Mornet et al. 2001 | perinatal | E459G/E459 G | | | + | |
| 12 | c.1433A>T | N461I | p. N478I | Taillandier et al. 2000 | childhood | N461I/N | 1.1 | 3 | - | |
| 12 | c.1444_1445in sC | | | Brun-Heath et al. 2005 | perinatal | c.1444_1445i nsC/G317D | | | | Frameshift mutation |
| 12 | c.1456G>C | C472S | p.C489 S | Taillandier et al. 2000 | perinatal | C472S/c.997+ 2T>A | 9.4 | 5 | - | |
| 12 | c.1468A>T | I473F | p.I490F | Lia-Baldini et al. 2001 | adult | I473F/? | 37.1 | 3 | - | |
| 12 | c.1471 G>A | G474R | p.G491 R | Mornet et al. 1998 | perinatal | G112R/G474 R | | | - | |
| 12 | c.1471delG | | | Brun-Heath et al. 2005 | odonto | c.1471delG/R | | | | Frameshift mutation |
| | | | | | | 119H | | | | |
| 12 | c.1559delT | | | Orimo et al. 1994 | infantile | E281K/c.1559 delT | 28 | 18 | na | Frameshift mutation |
| **Large deletions** | | | | | | | | | | |
| deletion of | | | | Spentchian et al. 2006 | perinatal | homozygote | | | | |
| exons 3-5 | | | | | | | | | | |
| deletion of exon 12 (3' part) | | | Spentchian et al. 2006 | infantile | compound heterozygote with S164L | | | | | |

### Spacer

Without being limited to this theory, it is believed that the Fc fragment used in the bone targeted sALP fusion protein presented in Examples below acts as a spacer which allows the protein to be more efficiently folded since expression of sTNALP-Fc-D10 was higher than that of sTNALP-D10 (see Example 2 below). One possible explanation is that the introduction of the Fc fragment alleviates the repulsive forces caused by the presence of the highly negatively charges D10 sequence added at the C-terminus of the tested sALP sequence.

Spacers for the present invention are polypeptides comprising a Fc . It is disclosed that the spacer alleviates the steric hindrance preventing two sALP domains from two sALP monomers from interacting with each other to constitute the minimal catalytically active entity.

### Fragment crystallizable region (Fc) fragments

Useful Fc fragments for the present invention include FC fragments of IgG that comprise the hinge, and the CH2 and CH3 domains. IgG-1, IgG-2, IgG-3, IgG-3 and IgG-4 for instance can be used.

### Negatively charged peptide

The negatively charged peptide according to the present invention may be a poly-aspartate or poly-glutamate selected from the group consisting of D10 to D16 or E10 to E16.

In specific embodiments, the bone targeted sALP fusion proteins of the present invention are associated so as to form dimers or tetramers.

Without being limited to this particular theory, using a polypeptide comprising a Fc as a spacer, dimers are presumably constituted of two bone targeted sALP monomers covalently linked through the two disulfide bonds located in the hinge regions of the two Fc fragments. In this dimeric configuration the steric hindrance imposed by the formation of the interchain disulfide bonds are presumably preventing the association of sALP domains to associate into the dimeric minimal catalytically active entity present in normal cells.

Without being limited to this particular theory, it is believed that in its tetrameric structure, the association of the fusion proteins would involve one sALP domain from one dimer and another one from another dimer. The steric hindrance presumably preventing two sALP domains from the same Fc-joined dimer from interacting with each other to constitute the minimal catalytically active entity could eventually be relieved by inserting a longer spacer than the Fc described in Examples presented herein between the sALP fragment and the polyaspartate or polyglutamate fragment.

The bone targeted sALP may further optionally comprise one or more additional amino acids 1) downstream from the poly-aspartate or poly-glutamate; and/or 2) between the poly-aspartate and the Fc fragment; and/or 3) between the spacer such as the Fc fragment and the sALP fragment. This is the case for instance when the cloning strategy used to produce the bone targeting conjugate introduces exogenous amino acids in these locations. However the exogenous amino acids should be selected so as not to provide an additional GPI anchoring signal. The likelihood of a designed sequence being cleaved by the transamidase of the host cell can be predicted as described by Ikezawa (Ikezawa 2002).

The present invention also encompasses the fusion protein as post-translationally modified such as by glycolisation including those expressly mentioned herein, acetylation, amidation, blockage, formylation, gamma-carboxyglutamic acid hydroxylation, methylation, phosphorylation, pyrrolidone carboxylic acid, and sulfatation.

The term "recombinant protein" is used herein to refer to a protein encoded by a genetically manipulated nucleic acid inserted into a prokaryotic or eukaryotic host cell. The nucleic acid is generally placed within a vector, such as a plasmid or virus, as appropriate for the host cell. Although Chinese Hamster Ovary (CHO) cells have been used as a host for expressing the conjugates of the present invention in the Examples presented herein, a person of ordinary skill in the art will understand that a number of other hosts may be used to produce recombinant proteins according to methods that are routine in the art. Representative methods are disclosed in Maniatis, et al. Cold Springs Harbor Laboratory (1989). "Recombinant cleavable protein" as used herein is meant to refer to a recombinant protein that may be cleaved by a host's enzyme so as to produce a secreted/soluble protein. Without being so limited HEK293 cells, PerC6, Baby hamster Kidney cells can also be used.

As used herein the terminology "conditions suitable to effect expression of the polypeptide" is meant to refer to any culture medium that will enable production of the fusion protein of the present invention. Without being so limited, it includes media prepared with a buffer, bicarbonate and/or HEPES, ions like chloride, phosphate, calcium, sodium, potassium, magnesium, iron, carbon sources like simple sugars, amino acids, potentially lipids, nucleotides, vitamins and growth factors like insulin; regular commercially available media like alpha-MEM, DMEM, Ham's-F12 and IMDM supplemented with 2-4 mM L-glutamine and 5% Fetal bovine serum; regular commercially available animal protein free media like Hyclone™ SFM4CHO, Sigma CHO DHFR-, Cambrex POWER™ CHO CD supplemented with 2-4 mM L-glutamine. These media are desirably prepared without thymidine, hypoxanthine and L-glycine to maintain selective pressure allowing stable protein-product expression.

Without being so limited, host cells useful for expressing the fusion of the present invention include L cell, C127 cells, 3T3 cells, CHO cells, BHK cells, COS-7 cells or Chinese Hamster Ovary (CHO) cell. Particular CHO cells of interest for expressing the fusion protein of the present invention include CHO-DG44 and CHO/dhfr⁻ also referred to as CHO duk⁻. This latter cell line is available through the American Type Culture Collection (ATCC number CRL-9096).

The term "bone tissue" is used herein to refer to tissue synthesized by osteoblasts composed of an organic matrix containing mostly collagen and mineralized by the deposition of hydroxyapatite crystals.

The fusion proteins comprised in the bone delivery conjugates of the present invention are useful for therapeutic treatment of bone defective conditions by providing an effective amount of the fusion protein to the bone. The fusion proteins are provided in the form of pharmaceutical compositions in any standard pharmaceutically acceptable carriers, and are administered by any standard procedure, for example by intravenous injection.

As used herein the terminology "HPP phenotype" is meant to refer to any one of rickets (defect in growth plate cartilage), osteomalacia, elevated blood and/or urine levels of inorganic pyrophosphate (PPᵢ), phosphoethanolamine (PEA), or pyridoxal 5'-phosphate (PLP), seizure, bone pains, calcium pyrophosphate dihydrate crystal deposition (CPPD) in joints leading to chondrocalcinosis and premature death. Without being so limited, a HPP phenotype can be documented by growth retardation with a decrease of long bone length (such as femur, tibia, humerus, radius, ulna), a decrease of the mean density of total bone and a decrease of bone mineralization in bones such as femur, tibia, ribs and metatarsi, and phalange, a decrease in teeth mineralization, a premature loss of deciduous teeth (e.g., aplasia, hypoplasia or dysplasia of dental cementum). Without being so limited, correction or prevention of bone mineralization defect may be observed by one or more of the following: an increase of long bone length, an increase of mineralization in bone and/or teeth, a correction of bowing of the legs, a reduction of bone pain and a reduction of CPPD crystal deposition in joints.

As used herein the terminology "correct" in the expression" correct a hypophosphatasia phenotype" is meant to refer to any partial or complete reduction of a pre-existing HPP phenotype. Similarly the terminology "prevent" in the expression "prevent a hypophosphatasia phenotype" is meant to refer to any delay or slowing in the development of a HPP phenotype or any partial or complete avoidance of the development of a HPP phenotype.

As used herein the term "subject" is meant to refer to any mammal including human, mice, rat, dog, cat, pig, cow, monkey, horse, etc. In a particular embodiment, it refers to a human.

As used herein, the term "subject in need thereof" in a method of administering a compound of the present invention is meant to refer to a subject that would benefit from receiving a compound of the present invention. In specific embodiments, it refers to a subject that already has at least one HPP phenotype or to a subject likely to develop at least one HPP phenotype or at least one more HPP phenotype. In another embodiment it further refers to a subject that has aplasia, hypoplasia or dysplasia of dental cementum or a subject likely to develop aplasia, hypoplasia or dysplasia of dental cementum.

As used herein "a subject likely to develop at least one HPP phenotype" is a subject having at least one loss-of-function mutation in the gene *(ALPL).*

As used herein "a subject likely to develop aplasia, hypoplasia or dysplasia of dental cementum" is a subject having HPP or a periodontal disease due to a bacterial infection. Periodontal disease due to a bacterial infection may induce alteration of cementum which may lead to exfoliation of teeth.

### Route of administration

Bone targeted sALPs of the present invention can be administered by routes such as orally, nasally, intravenously, intramuscularly, subcutaneously, sublingually, intrathecally, or intradermally. The route of administration can depend on a variety of factors, such as the environment and therapeutic goals. As used herein, subjects refer to animals such as humans in which prevention, or correction of bone mineralization defect characterizing HPP or other phenotypes associated with HPP or prevention or correction of defective cementum is desirable.

By way of example, pharmaceutical composition of the invention can be in the form of a liquid, solution, suspension, pill, capsule, tablet, gelcap, powder, gel, ointment, cream, nebulae, mist, atomized vapor, aerosol, or phytosome. For oral administration, tablets or capsules can be prepared by conventional means with pharmaceutically acceptable excipients such as binding agents, fillers, lubricants, disintegrants, or wetting agents. The tablets can be coated by methods known in the art. Liquid preparations for oral administration can take the form of, for example, solutions, syrups, or suspension, or they can be presented as a dry product for constitution with saline or other suitable liquid vehicle before use. Dietary supplements of the invention also can contain pharmaceutically acceptable additives such as suspending agents, emulsifying agents, non-aqueous vehicles, preservatives, buffer salts, flavouring, coloring, and sweetening agents as appropriate. Preparations for oral administration also can be suitably formulated to give controlled release of the active ingredients.

Enteric coatings can further be used on tablets of the present invention to resist prolonged contact with the strongly acidic gastric fluid, but dissolve in the mildly acidic or neutral intestinal environment. Without being so limited, cellulose acetate phthalate, Eudragit™ and hydroxypropyl methylcellulose phthalate (HPMCP) can be used in enteric coatings of pharmaceutical compositions of the present invention. Cellulose acetate phthalate concentrations generally used are 0.5-9.0% of the core weight. The addition of plasticizers improves the water resistance of this coating material, and formulations using such plasticizers are more effective than when cellulose acetate phthalate is used alone. Cellulose acetate phthalate is compatible with many plasticizers, including acetylated monoglyceride; butyl phthalybutyl glycolate; dibutyl tartrate; diethyl phthalate; dimethyl phthalate; ethyl phthalylethyl glycolate; glycerin; propylene glycol; triacetin; triacetin citrate; and tripropionin. It is also used in combination with other coating agents such as ethyl cellulose, in drug controlled-release preparations.

### Dosage

Any amount of a pharmaceutical composition can be administered to a subject. The dosages will depend on many factors including the mode of administration and the age of the subject. Typically, the amount of bone targeted ALP of the invention contained within a single dose will be an amount that effectively prevent, delay or correct bone mineralization defect in HPP without inducing significant toxicity. As used herein the term "therapeutically effective amount" is meant to refer to an amount effective to achieve the desired therapeutic effect while avoiding adverse side effects. Typically, bone targeted sALPs in accordance with the present invention can be administered to subjects in doses ranging from 0.001 to 500 mg/kg/day and, in a more specific embodiment, about 0.1 to about 100 mg/kg/day, and, in a more specific embodiment, about 0.2 to about 20 mg/kg/day. The allometric scaling method of Mahmood et al. (Mahmood et al. 2003) can be used to extrapolate the dose from mice to human. The dosage will be adapted by the clinician in accordance with conventional factors such as the extent of the disease and different parameters from the patient.

The therapeutically effective amount of the bone targeted sALP may also be measured directly. The effective amount may be given daily or weekly or fractions thereof. Typically, a pharmaceutical composition of the invention can be administered in an amount from about 0.001 mg up to about 500 mg per kg of body weight per day (e.g., 0.05, 0.01, 0.1, 0.2, 0.3, 0.5, 0.7, 0.8, 1 mg, 2 mg, 3 mg, 4mg, 5 mg, 10 mg, 15 mg, 20 mg, 30 mg, 50 mg, 100 mg, or 250 mg). Dosages may be provided in either a single or multiple dosage regimens. For example, in some embodiments the effective amount is a dose that ranges from about 0.1 to about 100 mg/kg/day, from about 0.2 mg to about 20 mg of the bone targeted sALP per day, about 1 mg to about 10 mg of the bone targeted sALP per day, from about .07 mg to about 210 mg of the bone targeted sALP per week, 1.4 mg to about 140 mg of the bone targeted sALP per week, about 0.3 mg to about 300 mg of the bone targeted sALP every three days, about 0.4 mg to about 40 mg of the bone targeted sALP every other day, and about 2 mg to about 20 mg of the bone targeted sALP every other day.

These are simply guidelines since the actual dose must be carefully selected and titrated by the attending physician based upon clinical factors unique to each patient or by a nutritionist. The optimal daily dose will be determined by methods known in the art and will be influenced by factors such as the age of the patient as indicated above and other clinically relevant factors. In addition, patients may be taking medications for other diseases or conditions. The other medications may be continued during the time that a bone targeted sALP is given to the patient, but it is particularly advisable in such cases to begin with low doses to determine if adverse side effects are experienced.

### Carriers/vehicles

Preparations containing a bone targeted sALP may be provided to patients in combination with pharmaceutically acceptable sterile aqueous or non-aqueous solvents, suspensions or emulsions. Examples of non-aqueous solvents are propylene glycol, polyethylene glycol, vegetable oil, fish oil, and injectable organic esters. Aqueous carriers include water, water-alcohol solutions, emulsions or suspensions, including saline and buffered medical parenteral vehicles including sodium chloride solution, Ringer's dextrose solution, dextrose plus sodium chloride solution, Ringer's solution containing lactose, or fixed oils. Intravenous vehicles may include fluid and nutrient replenishers, electrolyte replenishers, such as those based upon Ringer's dextrose, and the like.

In yet another embodiment, the pharmaceutical compositions of the present invention can be delivered in a controlled release system. In one embodiment polymeric materials including polylactic acid, polyorthoesters, crosslinked amphipathic block copolymers and hydrogels, polyhydroxy butyric acid and polydihydropyrans can be used (see also Smolen and Ball, Controlled Drug Bioavailability, Drug product design and performance, 1984, John Wiley & Sons; Ranade and Hollinger, Drug Delivery Systems, pharmacology and toxicology series, 2003, 2nd edition, CRRC Press), in another embodiment, a pump may be used (Saudek et al., 1989, N. Engl. J. Med. 321: 574).

The fusion proteins of the present invention could be in the form of a lyophilized powder using appropriate excipient solutions (e.g., sucrose) as diluents.

Further, the nucleotide segments or proteins according to the present invention can be introduced into individuals in a number of ways. For example, osteoblasts can be isolated from the afflicted individual, transformed with a nucleotide construct according to the invention and reintroduced to the afflicted individual in a number of ways, including intravenous injection. Alternatively, the nucleotide construct can be administered directly to the afflicted individual, for example, by injection. The nucleotide construct can also be delivered through a vehicle such as a liposome, which can be designed to be targeted to a specific cell type, and engineered to be administered through different routes.

The fusion proteins of the present invention could also be advantageously delivered through gene therapy. Useful gene therapy methods include those described in WO6060641A2, US7179903 and WO0136620A2 to Genzyme using for instance an adenovirus vector for the therapeutic protein and targeting hepatocytes as protein producing cells.

A "gene delivery vehicle" is defined as any molecule that can carry inserted polynucleotides into a host cell. Examples of gene delivery vehicles are liposomes, biocompatible polymers, including natural polymers and synthetic polymers; lipoproteins; polypeptides; polysaccharides; lipopolysaccharides; artificial viral envelopes; metal particles; and bacteria, or viruses, such as baculovirus, adenovirus and retrovirus, bacteriophage, cosmid, plasmid, fungal vectors and other recombination vehicles typically used in the art which have been described for expression in a variety of eukaryotic and prokaryotic hosts, and may be used for gene therapy as well as for simple protein expression. "Gene delivery," "gene transfer," and the like as used herein, are terms referring to the introduction of an exogenous polynucleotide (sometimes referred to as a "transgene") into a host cell, irrespective of the method used for the introduction. Such methods include a variety of well-known techniques such as vector-mediated gene transfer (e.g., viral infection/transfection, or various other protein-based or lipid-based gene delivery complexes) as well as techniques facilitating the delivery of "naked" polynucleotides (such as electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome. A number of vectors are known to be capable of meditating transfer of genes to mammalian cells, as is known in the art and described herein.

A "viral vector" is defined as a recombinantly produced virus or viral; particle that comprises a polynucleotide to be delivered into a host cell, either in viva, ex viva or in vitro. Examples of viral vectors include retroviral vectors, adenovirus vectors, adeno-associated virus vectors such as those described in WO06002203A2, alphavirus vectors and the like. Alphavirus vectors, such as Semliki Forest virus-based vectors and Sindbis virus-based vectors, have also been developed for use in gene therapy and immunotherapy.

In aspects where gene transfer is mediated by a DNA viral vector, such as an adenovirus (Ad) or adeno-associated virus (MVs), a vector construct refers to the polynucleotide comprising the viral genome or part thereof, and a transgene. Adenoviruses (Ads) are a relatively well characterized, homogenous group of viruses, including over 50 serotypes. See, e.g., International PCT Application No. WO 95/27071. Ads are easy to grow and do not require integration into the host cell genome. Recombinant Ad derived vectors, particularly those that reduce the potential for recombination and generation of wild-type virus, have also been constructed. See, International PCT Application Nos. WO 95/00655 and WO 95/11984. Vectors that contain both a promoter and a cloning site into which a polynucleotide can be operatively linked are well known in the art. Such vectors are capable of transcribing RNA in vitro or in vivo, and are commercially available from sources such as Stratagene (La Jolla, CA) and Promega Biotech (Madison, WI). In order to optimize expression and/or in vitro transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation.

The bone targeted sALP of the present invention may also be used in combination with at least one other active ingredient to correct a bone mineralization defect or another detrimental symptom of HPP. It may also be used in combination with at least one with at least one other active ingredient to correct cementum defect.

The term "high stringency conditions" are meant to refer to conditions enabling sequences with a high homology to bind. Without being so limited, examples of such conditions are listed In the handbook "Molecular cloning, a laboratory manual, second edition of 1989 from Sambrook *et al*.: 6XSSC or 6XSSPE, Denhardt's reagent or not, 0.5% SDS and the temperature used for obtaining high stringency conditions is most often in around 68°C (see pages 9.47 to 9.55 of Sambrook) for nucleic acid of 300 to 1500 nucleotides. Although the optimal temperature to be used for a specific nucleic acid probe may be empirically calculated, and although there is room for alternatives in the buffer conditions selected, within these very well known condition ranges, the nucleic acid captured will not vary significantly. Indeed, Sambrook clearly indicates that the "choice depends to a large extent on personal preference" (see page 9.47). Sambrook specifies that the formula to calculate the optimal temperature which varies according to the fraction of guanine and cytosine in the nucleic acid probe and the length of the probe (10 to 20°C lower than Tₘ wherein Tₘ = 81.5°C + 16.6(log₁₀[Na⁺]) + 0.41 (fraction G+C)-0.63 (% formamide -(600/l)) (see pages 9.50 and 9.51 of Sambrook).

### Kits

The present invention also relates to a kit for correcting or preventing an HPP phenotype or a cementum defect comprising a nucleic acid, a protein or a ligand in accordance with the present invention. For instance it may comprise a bone targeted composition of the present invention or a vector encoding same, and instructions to administer said composition or vector to a subject to correct or prevent a HPP phenotype. Such kits may further comprise at least one other active agent able to prevent or correct a HPP phenotype. When the kit is used to prevent or correct a HPP phenotype in a HPP subject, the kit may also further comprise at least one other active agent capable of preventing or correcting any other detrimental symptoms of HPP. In addition, a compartmentalized kit in accordance with the present invention includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another.

More specifically, in accordance with a first aspect of the present invention, there is provided a bone targeted alkaline phosphatase comprising a polypeptide having the structure : Z-sALP-Y-spacer-X-Wn-V, wherein sALP consists of amino acid residues 18-502 of Seq ID No. 8; wherein V is absent or is an amino acid sequence of at least one amino acid; X is absent or is an amino acid sequence of at least one amino acid; Y is absent or is an amino acid sequence of at least one amino acid; Z is absent or is an amino acid sequence of at least one amino acid; and Wn is a polyaspartate or a polyglutamate wherein n = 10 to 16, and the spacer comprises a fragment crystallizable region (Fc).

It is disclosed that the sALP comprises amino acid residues 23-508 of SEQ ID NO: 15. It is also disclosed that the sALP consists of amino acid residues 23-512 of SEQ ID NO: 15. It is also disclosed that, the sALP comprises amino acid residues 23-508 of SEQ ID NO: 18. It is also disclosed that the sALP consists of amino acid residues 23-512 of SEQ ID NO: 18. It is also disclosed that the sALP comprises amino acid residues 18-498 of SEQ ID NO: 16. It is also disclosed that the sALP consists of amino acid residues 18-502 of SEQ ID NO: 16. It is also disclosed that, the sALP comprises amino acid residues 18-498 of SEQ ID NO: 19. It is also disclosed that the sALP consists of amino acid residues 18-502 of SEQ ID NO: 19. It is also disclosed that the sALP comprises amino acid residues 18-498 of SEQ ID NO: 19. It is also disclosed that the sALP consists of amino acid residues 18-502 of SEQ ID NO: 19. It is also disclosed that, the sALP comprises amino acid residues 18-498 of SEQ ID NO: 8. According to the present invention, the sALP consists of amino acid residues 18-502 of SEQ ID NO: 8.

According to the present invention, the spacer comprises a fragment crystallizable region (Fc). In a specific embodiment, the Fc comprises a CH2 domain, a CH3 domain and a hinge region. In another specific embodiment, the Fc is a constant domain of an immunoglobulin selected from the group consisting of IgG-1, IgG-2, IgG-3, IgG-3 and IgG-4. In another specific embodiment, the Fc is a constant domain of an immunoglobulin IgG-1. In another specific embodiment, the Fc is as set forth in SEQ ID NO: 3. In another specific embodiment, Wn is a polyaspartate. In another specific embodiment, n=10. In another specific embodiment, Z is absent. In another specific embodiment, Y is two amino acid residues. In another specific embodiment, Y is leucine-lysine. In another specific embodiment, X is 2 amino acid residues. In another specific embodiment, X is aspartate-isoleucine. In another specific embodiment, V is absent. In another specific embodiment, the polypeptide is as set forth in SEQ ID NO: 4.

In another specific embodiment, the bone targeted alkaline phosphatase comprises the polypeptide in a form comprising a dimer. In another specific embodiment, the bone targeted alkaline phosphatase comprises the polypeptide in a form of a tetramer.

In another specific embodiment, the bone targeted alkaline phosphatase is in a pharmaceutically acceptable carrier. In another specific embodiment, the pharmaceutically acceptable carrier is a saline. In another specific embodiment, the bone targeted alkaline phosphatase is in a lyophilized form. In another specific embodiment, the bone targeted alkaline phosphatase is in a daily dosage of about 0.2 to about 20 mg/kg. In another specific embodiment, the bone targeted alkaline phosphatase is in a dosage of about 0.6 to about 60 mg/kg for administration every three days. In another specific embodiment, the bone targeted alkaline phosphatase is in a weekly dosage of about 1.4 to about 140 mg/kg. In another specific embodiment, the bone targeted alkaline phosphatase is in a weekly dosage of about 0.5 mg/kg.

More specifically, in accordance with another aspect of the present invention, there is provided an isolated nucleic acid comprising a sequence that encodes the polypeptide of the present invention.

In accordance with another aspect of the present invention, there is provided an isolated nucleic acid consisting of a sequence that encodes the polypeptide of the present invention. More specifically, in accordance with another aspect of the present invention, there is provided an isolated nucleic acid comprising a sequence as set forth in SEQ ID NO: 17.

In accordance with another aspect of the present invention, there is provided a recombinant expression vector comprising the nucleic acid of the present invention. More specifically, in accordance with another aspect of the present invention, there is provided a recombinant adeno-associated virus vector comprising the nucleic acid of the present invention. More specifically, in accordance with another aspect of the present invention, there is provided an isolated recombinant host cell transformed or transfected with the vector of the present invention.

In accordance with another aspect of the present invention, there is provided a method of producing the bone targeted alkaline phosphatase of the present invention, comprising culturing the host cell of the present invention, under conditions suitable to effect expression of the bone targeted alkaline phosphatase and recovering the bone targeted alkaline phosphatase from the culture medium.

In a specific embodiment, the host cell is a L cell, C127 cell, 3T3 cell, CHO cell, BHK cell, COS-7 cell or a Chinese Hamster Ovary (CHO) cell. In another specific embodiment, the host cell is a Chinese Hamster Ovary (CHO) cell. In a specific embodiment, the host cell is a CHO-DG44 cell.

In accordance with another aspect of the present invention, there is provided a kit comprising the bone targeted alkaline phosphatase of the present invention, and instructions to administer the polypeptide to a subject to correct or prevent a hypophosphatasia (HPP) phenotype.

In accordance with another aspect of the present invention, there is provided a kit comprising the bone targeted alkaline phosphatase of the present invention, and instructions to administer the polypeptide to a subject to correct or prevent aplasia, hypoplasia or dysplasia of dental cementum.

In accordance with another aspect of the present invention, there is provided a method of using the bone targeted alkaline phosphatase of the present invention, for correcting or preventing at least one hypophosphatasia (HPP) phenotype, comprising administering a therapeutically effective amount of the bone targeted alkaline phosphatase to a subject in need thereof, whereby the at least one HPP phenotype is corrected or prevented in the subject.

In a specific embodiment, the subject has at least one HPP phenotype. In another specific embodiment, the subject is likely to develop at least one HPP phenotype. In another specific embodiment, the at least one HPP phenotype comprises HPP-related seizure. In another specific embodiment, the at least one HPP phenotype comprises premature loss of deciduous teeth. In another specific embodiment, the at least one HPP phenotype comprises incomplete bone mineralization. In another specific embodiment, incomplete bone mineralization is incomplete femoral bone mineralization. In another specific embodiment, incomplete bone mineralization is incomplete tibial bone mineralization. In another specific embodiment, incomplete bone mineralization is incomplete metatarsal bone mineralization. In another specific embodiment, incomplete bone mineralization is incomplete ribs bone mineralization. In another specific embodiment, the at least one HPP phenotype comprises elevated blood and/or urine levels of inorganic pyrophosphate (PPi). In another specific embodiment, the at least one HPP phenotype comprises elevated blood and/or urine levels of phosphoethanolamine (PEA). In another specific embodiment, the at least one HPP phenotype comprises elevated blood and/or urine levels of pyridoxal 5'-phosphate (PLP). In another specific embodiment, the at least one HPP phenotype comprises inadequate weight gain. In another specific embodiment, the at least one HPP phenotype comprises rickets. In another specific embodiment, the at least one HPP phenotype comprises bone pain. In another specific embodiment, the at least one HPP phenotype comprises calcium pyrophosphate dihydrate crystal deposition. In another specific embodiment, the at least one HPP phenotype comprises aplasia, hypoplasia or dysplasia of dental cementum. In another specific embodiment, the subject in need thereof has infantile HPP. In another specific embodiment, the subject in need thereof has childhood HPP. In another specific embodiment, the subject in need thereof has perinatal HPP. In another specific embodiment, the subject in need thereof has adult HPP. In another specific embodiment, the subject in need thereof has odontohypophosphatasia HPP.

A method of using the bone targeted alkaline phosphatase of the present invention, for correcting or preventing aplasia, hypoplasia or dysplasia of dental cementum, comprising administering a therapeutically effective amount of the bone targeted alkaline phosphatase to a subject in need thereof, whereby aplasia, hypoplasia or dysplasia of dental cementum is corrected or prevented in the subject is disclosed.

In a specific embodiment, the administering comprises transfecting a cell in the subject with a nucleic acid encoding the alkaline phosphatase. In another specific embodiment, the transfecting the cell is performed *in vitro* such that the bone targeted alkaline phosphatase is expressed and secreted in an active form and administered to the subject with said cell. In another specific embodiment, the administering comprises subcutaneous administration of the bone targeted alkaline phosphatase to the subject. In another specific embodiment, the administering comprises intravenous administration of the bone targeted alkaline phosphatase to the subject.

In accordance with another aspect of the present invention, there is provided the bone targeted alkaline phosphatase of the present invention, for use in correcting or preventing at least one HPP phenotype.

In accordance with another aspect of the present invention, there is provided the bone targeted alkaline phosphatase of the present invention, for use in correcting or preventing aplasia, hypoplasia or dysplasia of dental cementum.

In accordance with another aspect of the present invention, there is provided a use of the bone targeted alkaline phosphatase of the present invention, in the making of a medicament.

In accordance with another aspect of the present invention, there is provided a use of the bone targeted alkaline phosphatase of the present invention, for correcting or preventing at least one HPP phenotype.

In accordance with another aspect of the present invention, there is provided a use of the bone targeted alkaline phosphatase of the present invention, for correcting or preventing aplasia, hypoplasia or dysplasia of dental cementum.

### BRIEF DESCRIPTION OF THE DRAWINGS

In the appended drawings:

Figure 1 presents the design and schematic structure of the bone targeted ALP of the present invention exemplified by hsTNALP-FcD10. Panel A presents a schematic representation of the complete primary translation product of the human tissue non-specific alkaline phosphatase gene (TNALP) including the N-terminal signal peptide and the transient membrane-anchored signal for GPI-addition. Panel B presents the primary translation product of the fusion protein. Panel C presents the primary translation product lacking the cleavable TNALP signal peptide;

Figure 2 presents the protein sequence for hTNALP-FcD10 ((SEQ ID NO: 1), including the N-terminal peptide signal-17 first aa), wherein the hTNALP portion (SEQ ID NO: 2) is italicized including the peptide signal portion shown italicized and underlined, and the Fc fragment is underlined (SEQ ID NO: 3);

Figure 3 presents the protein sequence for the hsTNALP-FcD10 used in Examples presented herein (SEQ ID NO: 4) (without the N-terminal peptide signal) wherein the hsTNALP portion (SEQ ID NO: 5) is italicized, and the Fc fragment is underlined (SEQ ID NO: 3). Double underlined asparagine (N) residues correspond to putative N-glycosylation sites and bold amino acid residues (LK & DI) correspond to linkers between hsTNALP and Fc, and Fc and D10 domains respectively. These linkers are derived from endonuclease restriction sites introduced during cDNA engineering;

Figure 4 graphically presents the comparative expression of sTNALP-D10 and sTNALP-FcD10 in CHO-DG44 cells;

Figure 5 presents sTNALP-FcD10 purification on protein-A Sepharose molecular sieve chromatography on Sephacryl™ 3-300 as well as SDS-PAGE analysis of purified sTNALP-FcD10 under reducing (DTT +) and non reducing (DTT-) conditions. It also presents a schematized version of sTNALP-FcD10.The protein purified by Protein A-Sepharose™ affinity chromatography was analyzed by SDS-PAGE and bands stained with Sypro™ Ruby. Main species of sTNALP-FcD10 migrated with an apparent molecular mass of 90,000 Da under reducing conditions and 200,000 Da under non reducing conditions;

Figure 6 presents the position of the papain cleavage site in sTNALP-FcD10;

Figure 7 presents a non denaturing SEC-HPLC analysis of sTNALP-FcD10 on TSK-Gel G3000WXL column. Plain curve: papain digested sample. -X- curve: identical sample incubated in the same conditions without papain (control);

Figure 8 presents a SDS-PAGE analysis of sTNALP-FcD10 incubated with or without papain showing which fragment is responsible for which band on the gel. Analysis was performed under reducing (+ DTT) or non reducing (- DTT) conditions;

Figure 9 presents an *in vitro* binding assay. sTNALP-FcD10 and bovine kidney tissue non specific alkaline phosphatase were compared in the reconstituted mineral binding assay as described in Example 2. Total activity is the sum of the enzymatic activity recovered in the free and bound fractions. Total activity was found to be 84% and 96% of initial amount of enzymatic activity introduced in each set of assays for the bovine and sTNALP-FcD10 forms of enzyme, respectively. Results are the average of two bindings;

Figure 10 presents pharmacokinetic and distribution profiles of sTNALP-FcD10 in serum, tibia and muscle of adult WT mice. Concentrations of sTNALP-FcD10 in serum, tibia and muscle, is expressed in µg/g tissue (wet weight) after a single bolus intravenous injection of 5 mg/kg in adult WT mice;

Figure 11 presents pharmacokinetic profile of sTNALP-FcD10 serum concentration in newborn WT mice. Serum concentrations of sTNALP-FcD10 as a function of time after a single i.p. (panel A) or s.c. (panel B) injection of 3.7 mg/kg in (1 day old) newborn WT mice;

Figure 12 presents the predicted pharmacokinetic profile of sTNALP-FcD10 in serum. Predicted maximal (Cmax) and minimal (Cmin) circulating steady-state levels of sTNALP-FcD10 after repeated (every 24 hrs) subcutaneous injections of 10 mg/Kg in newborn mice;

Figure 13 presents the experimentally tested pharmacokinetic profile of sTNALP-FcD10 in the serum of newborn mice. Measured minimal (Cmin) circulating steady-state levels of sTNALP-FcD10 24 h after the last subcutaneous injections of 10 mg/Kg in newborn mice. Homo: homozygous, hetero: heterozygous;

Figure 14 presents short-term (15 days), low dose (1 mg/Kg), efficacy results in terms of sTNALP-FcD10 serum concentrations in treated Akp2^{-/-} mice. sTNALP-FcD10 serum concentrations at day 16 of mice treated for 15 days with daily s.c. injections of 1 mg/kg sTNALP-FcD10;

Figure 15 presents short-term (15 days), low dose (1 mg/Kg), efficacy results in terms of serum PPi concentrations in treated Akp2^{-/-} mice. Measurement of serum PPi concentrations. A low dose of 1 mg/kg was sufficient to normalize PPi levels in ERT-treated mice;

Figure 16 presents short-term (15 days), low dose (1 mg/Kg), efficacy results in terms of physeal morphology in treated Akp2^{-/-} mice. Goldner's trichrome staining of the growth plates of WT, untreated and treated Akp2^{-/-} mice. The proximal tibial growth plates (physes) showed excessive widening of the hypertrophic zone in both sTNALP-FcD10 and vehicle injected in Akp2^{-/-} mice, consistent with early rickets. However, physeal morphology seemed less disturbed in the animals treated with sTNALP-FcD10;

Figure 17 presents short-term (15 days), low dose (1 mg/Kg), efficacy results in terms of physeal hypertrophic area size of treated Akp2^{-/-} mice. Size of the hypertrophic area of the growth plate is expressed as a percentage of the total growth plate area. Note the normalization of the hypertrophic area in the treated mice;

Figure 18 presents short-term (15 days), high dose (8.2 mg/Kg), efficacy results in terms of body weight in treated Akp2^{-/-} mice. Effect of sTNALP-FcD10 on body weight;

Figure 19 presents short-term (15 days), high dose (8.2 mg/Kg), efficacy results in terms of long bone length in treated Akp2^{-/-} mice. Effect of sTNALP-FcD10 on femur and tibial length (measurements done at day 16);

Figure 20 presents short-term (15 days), high dose (8.2 mg/Kg), efficacy results in terms of sTNALP-FcD10 serum concentration in treated Akp2^{-/-} mice. sTNALP-FcD10 serum concentrations at day 16 of mice treated for 15 days with daily s.c. injections of 8.2 mg/kg sTNALP-FcD10;

Figure 21 presents short-term (15 days), high dose (8.2 mg/Kg), efficacy results in terms of mineralization of bones in treated Akp2^{-/-} mice. X-ray analysis of feet, rib cages and hind limbs of Akp2^{-/-} mice (16 days) and a Faxitron™ image distribution table. Feet and rib cages were classified as severe, moderate or healthy to take into account the extent of the bone mineralization defects. Legs were simply classified as abnormal (at least one defect) or healthy (no visible defect);

Figure 22 presents short-term (15 days), high dose (8.2 mg/Kg), efficacy results in terms of defects in teeth in treated Akp2^{-/-} mice. Histological analysis of teeth of Akp2^{-/-} mice injected vehicle or sTNALP-FcD10 and wild-type mice.. Thin sections were prepared and stained as described in Millan et al. PDL=Peridontal ligament;

Figure 23 presents long-term (52 days), high dose (8.2 mg/Kg), efficacy results in terms of survival in treated Akp2^{-/-} mice. Long-term survival of Akp2^{-/-} mice treated with sTNALP-FcD10 compared to the early demise of Akp2^{-/-} treated only with control vehicle;

Figure 24 presents long-term (52 days), high dose (8.2 mg/Kg), efficacy results in terms of size, mobility and appearance in treated Akp2^{-/-} mice. mice. Treatment normalizes size, mobility and appearance of treated Akp2^{-/-} mice. Untreated mouse from the same litter is shown for comparison;

Figure 25 presents long-term (52 days), high dose (8.2 mg/Kg), efficacy results in terms of mineralization and length of bones in treated Akp2^{-/-} mice. X-ray images of the metatarsal bones of 46 and 53-days old treated Akp2^{-/-} mice in comparison with WT mice;

Figure 26 presents long-term (52 days), high dose (8.2 mg/Kg), efficacy results in terms of sTNALP-FcD10 serum concentration in treated Akp2^{-/-} mice. sTNALP-FcD10 serum concentrations at day 53 of mice treated for 52 days with daily s.c. injections of 8.2 mg/kg sTNALP-FcD10;

Figure 27 presents A) survival curves of Akp2^{-/-} mice receiving sTNALP-FcD10 at doses of either 4.3 mg/kg daily (Tx-1) or 15.2 mg/kg every 3 days (Tx-3) or 15.2 mg/kg every week (Tx-7) and B) median survival for each of these regimen . Survival of the treated mice was compared to the survival of mice injected vehicle;

Figure 28 presents A) survival curves of Akp2^{-/-} mice receiving sTNALP-FcD10 at doses of 8.2 mg/kg daily (RTx) starting at day 15 after birth and B) median survival for treated and vehicle injected mice. Survival of the treated mice is compared to the survival of mice injected vehicle (RVehide);

Figure 29 presents the effects on body weight of daily 8.2 mg/kg doses of sTNALP-FcD10 injected to Akp2^{-/-} mice (RTx) starting at day 15 after birth. Daily body weights are compared to that of vehicle-injected Akp2^{-/-} mice (RVehicle) or wild-type littermates (WT);

Figure 30 presents an alignment of various ALPs established by CLUSTAL™ W (1.82) multiple sequence alignment, namely a bovine TNALP sequence (SEQ ID NO: 6); a cat TNALP sequence (SEQ ID NO: 7), a human TNALP sequence (SEQ ID NO: 8), a mouse TNALP sequence (SEQ ID NO: 9), a rat TNALP sequence (SEQ ID NO: 10) and a partial dog TNALP sequence (SEQ ID NO: 11) wherein the nature of the first 22 amino acid residues are unknown; a human IALP (SEQ ID NO: 12) (Accession no: NP_001622), a human GCALP (SEQ ID NO: 13) (Accession no: P10696), and a human PLALP (SEQ ID NO: 14) (Accession no: NP_112603)."*" denotes that the residues in that column are identical in all sequences of the alignment,":" denotes that conserved substitutions have been observed, and "." denotes that semi-conserved substitutions have been observed. A consensus sequence derived from this alignment (SEQ ID NO: 15) is also presented wherein x is any amino acid;

Figure 31 presents an alignment of TNALPs from various species established by CLUSTAL™ W (1.82) multiple sequence alignment, namely the bovine sequence (SEQ ID NO: 6); the cat sequence (SEQ ID NO: 7), the human sequence (SEQ ID NO: 8), the mouse sequence (SEQ ID NO: 9), the rat sequence (SEQ ID NO: 10) and a partial dog sequence (SEQ ID NO: 11) wherein the nature of the first 22 amino acid residues are unknown. "*" denotes that the residues in that column are identical in all sequences of the alignment,":" denotes that conserved substitutions have been observed, and "." denotes that semi-conserved substitutions have been observed. A consensus sequence derived from this alignment (SEQ ID NO: 16) is also presented wherein x is any amino acid; and

Figure 32 presents the nucleic acid sequence (SEQ ID NO:17) encoding the polypeptide sequence described in Figure 1.

### DESCRIPTION OF ILLUSTRATIVE EMBODIMENTS

Examples provided below present the first successful treatment of TNALP knockout (Akp2-/-) mice using subcutaneous injections of a recombinant form of ALP. Akp2^{-/-} mice recapitulate the severe, often lethal, infantile form of Hypophosphatasia.

The well-described TNSALP-homozygous null murine model which mirrors many of the skeletal and biochemical abnormalities associated with infantile HPP was used. Mice were treated with a novel soluble recombinant form of human TNSALP engineered at its carboxy-terminus to contain both a spacer in the form of the crystalline fragment (Fc) region of human IgG-1 fused to a bone targeting sequence composed of ten sequential aspartic acid (D10) residues. It was shown that relative to native TNSALP purified from kidney, the modified recombinant form of the enzyme, binds hydroxyapatite much more avidly, while retaining its enzymatic activity. Treatment with the recombinant TNSALP of the present invention surprisingly normalized plasma PPi levels, and improved mineralization of the feet thoraces, hind limbs and dentition of homozygous null mice when compared to mice who received the vehicle alone. The treatment was also shown to prolong survival, with near radiographic normalization of the skeletal phenotype.

In addition to its beneficial *in vivo* therapeutic effect, it was surprisingly discovered that the recombinant active form of the modified enzyme which contains a spacer is expressed at higher levels than its recombinant counterpart lacking such spacer. In addition, it was demonstrated that the enzyme functions as a tetramer.

The present invention is illustrated in further details by the following non-limiting examples.

### EXAMPLE 1

### Expression and purification of recombinant sTNALP-FcD10

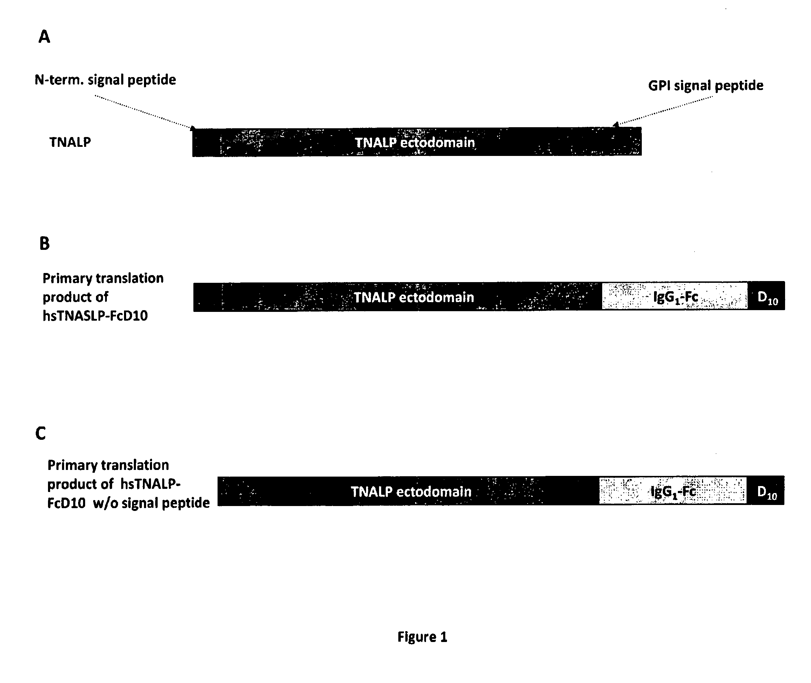

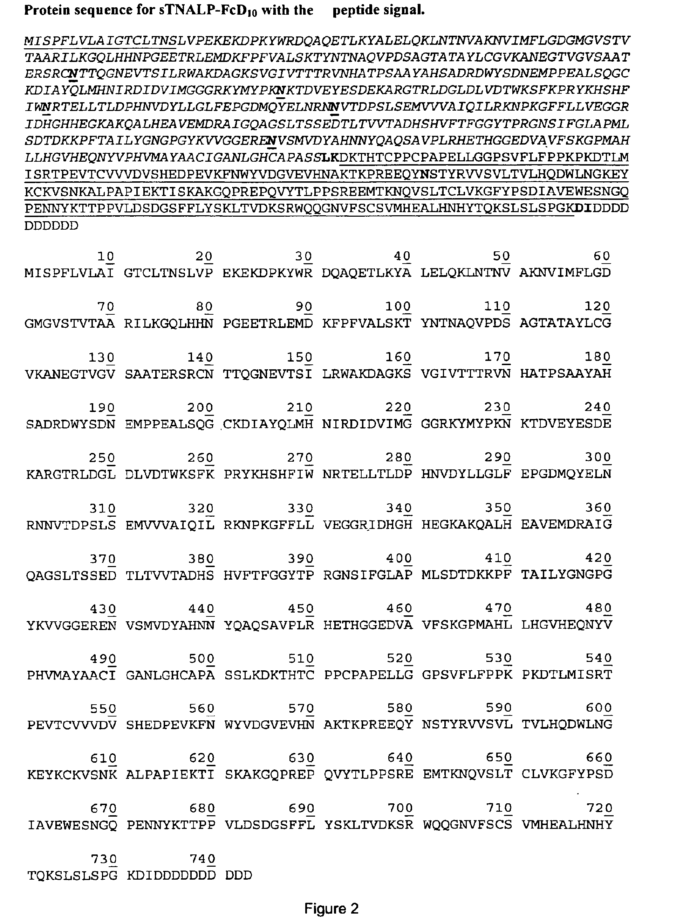

In order to facilitate the expression and purification of recombinant TNALP, the hydrophobic C-terminal sequence that specifies GPI-anchor attachment in TNALP was eliminated to make it a soluble secreted enzyme (Di Mauro et al. 2002). The coding sequence of the TNALP ectodomain was also extended with the Fc region of the human IgG (γ¹ form (IgG1), Swiss-Prot P01857). This allowed rapid purification of the recombinant enzyme on Protein A chromatography and surprisingly, its increased expression. Furthermore, to target the recombinant TNALP to bone tissue, a deca-aspartate (D10) sequence was attached to the C-terminal of the Fc region. This chimeric form of TNALP, designated sTNALP-Fc010, retains full enzymatic activity both when assayed at pH 9.8 using the artificial substrate p-nitrophenylphosphate and when assayed at pH 7.4 using inorganic pyrophosphate (PPi), as the physiological substrate. As in the naturally occurring form of TNALP the N-terminal signal peptide is cleaved off during the cotranslational translocation of the protein across the rough endoplasmic reticulum. Its design and structure is schematically illustrated in Figure 1. The amino acid sequence of the fusion protein (including the signal peptide) is shown in Figure 2. The amino acid sequence of the fusion protein as secreted (i.e. without the signal peptide) is shown in Figure 3.

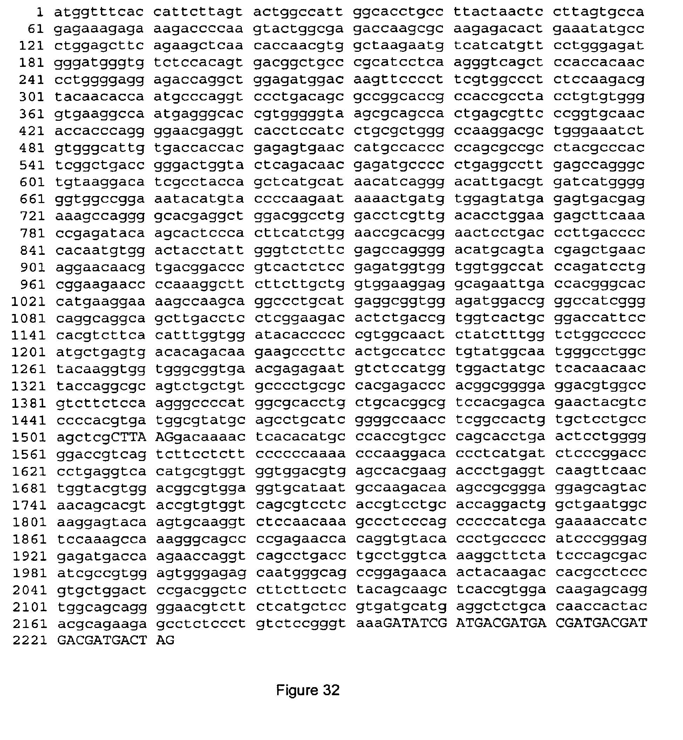

The method that was used to construct this fusion protein is as follows. The cDNA encoding the fusion protein (See Figure 32) was inserted in the pIRES vector (Clontech™) in the first multiple cloning site located upstream of the IRES using Nhel and BamHI endonuclease restriction sites. The dihydrofolate reductase (DHFR) gene was inserted in the second multiple cloning site located downstream of the IRES using SmaI and XbaI endonuclease restriction sites. The resulting vector was transfected into Chinese Hamster Ovary (CHO-DG44) cells lacking both DHFR gene alleles (Urlaub et al. 1983, obtained from Dr Lawrence A. Chasin, Columbia University) using the Lipofectamine™ transfection kit (Invitrogen). Two days after transfection, media was changed and the cells were maintained in a nucleotide free medium (IMDM supplemented with 5% dialyzed FBS) for 15 days to isolate stable transfectants for plaque cloning.

Cells from the three best clones or the five originally selected growing in the nucleotide-free medium were pooled and further cultivated in media (IMDM + 5% dialyzed FBS) containing increasing concentration of methotrexate (MTX). Cultures resistant to 50 nM MTX were further expanded in Cellstacks™ (Corning) containing IMDM medium supplemented with 5% FBS. Upon reaching confluency, the cell layer was rinsed with Phosphate Buffered Saline (PBS) and cells were incubated for three additional days with IMDM containing 3.5 mM sodium butyrate to increase protein expression. At the end of the culture the concentration of sTNALP-FcD10 in the spent medium was 3.5 mg/l as assessed by measuring TNALP enzymatic activity.

Levels of sALP-FcD10 in spent medium were quantified using a colorimetric assay for ALP activity where absorbance of released p-nitrophenol is proportional to the reaction products. The reaction occurred in 100 µl of ALP buffer (20 mM Bis Tris Propane (HCl) pH 9, 50 mM NaCl, 0.5 mM MgCl₂, and 50 µM ZnCl₂) containing 10 µl of diluted spent medium and 1 mM pNPP. The latter compound was added last to initiate the reaction. Absorbance was recorded at 405 nm every 45 seconds over 20 minutes using a spectrophotometric plate reader. sTNALP-FcD10 catalytic activity, expressed as an initial rate, was assessed by fitting the steepest slope for 8 sequential values. Standards were prepared with varying concentrations of sALP-FcD10, and ALP activity was determined as above. The standard curve was generated by plotting Log of the initial rate as a function of the Log of the standard concentrations. sTNALP-FcD10 concentration in the different samples was read from the standard curve using their respective ALP absorbance. Activity measures were transformed intro concentrations of sALP-FcD10 by using a calibration curve obtained by plotting the activity of known concentrations of purified recombinant enzyme.

Culture supernatant was then concentrated and dialyzed against PBS using tangential flow filtration and loaded on MabSelect SuRe™ column (GE Health Care) equilibrated with 150 mM NaCl, 10 mM sodium PO₄. Bound proteins were eluted with 50 mM Tris pH 11, pH 11.0 buffer. Collected fractions were adjusted to pH 8-9 with 200 mM Tris-HCl pH 5.5. Fractions containing most of the eluted material were dialyzed against 150 mM NaCl, 25 mM sodium PO₄ pH 7.4 buffer containing 0.1 mM MgCl₂, 20 µM ZnCl₂ and filtered on a 0.22 µm (Millipore, Millex-GP™) membrane under sterile conditions. The overall yield of the purification procedure was 50% with a purity above 95% as assessed by Sypro™ ruby stained SDS-PAGE. Purified sTNALP-FcD10 preparation was stored at 4°C and remained stable for several months.

The following purification technique was also tested with success. Culture supernatant was concentrated and dialyzed against PBS using tangential flow filtration and loaded on Protein A-Sepharose™ column (Hi-Trap™ 5 ml, GE Health Care) equilibrated with PBS. Bound proteins were eluted with 100 mM citrate pH 4.0 buffer. Collected fractions were immediately adjusted to pH 7.5 with 1 M Tris pH 9.0. Fractions containing most of the eluted material were dialyzed against 150 mM NaCl, 25 mM sodium PO₄ pH 7.4 buffer containing 0.1 mM MgCl₂, 20 µM ZnCl₂ and filtered on a 0.22 µm (Millipore, Millex-GP™) membrane under sterile conditions. The overall yield of the purification procedure was 50% with a purity above 95% as assessed by Sypro™ ruby stained SDS-PAGE. Purified sTNALP-FcD10 preparation was stored at 4°C and remained stable for several months.

The number of copies of the sTNALP-FcD10 gene was increased by culturing transfected CHO-DG44194 cells in the presence of increasing concentration of methotrexate. Clones of cells resistant to 100 nM methotrexate were isolated and evaluated for their capacity to produce sTNALP-FcD10 at a high yield. The best producers were adapted to culture in suspension in Hyclone media™ SFM4CHO™ (cat # SH30549) in absence of fetal bovine serum. Cultures that maintained a high production yield under those conditions were transferred to disposable Wave™ bioreactor bags. The medium (25 L total volume) was seeded at a density of 0.4 x 10⁶ cells per ml. Temperature of the culture was maintained at 37°C until the cell density reached 2 x 10⁶ cells/ml. The temperature was then reduced to 30°C and the culture was supplemented with 125 ml of CHO generic feed (Sigma, C1615). Those conditions were found to slow down cell division and increase product secretion in the culture medium. These conditions were maintained for 6 days before harvesting cell culture supernatant containing secreted sTNALP-FcD10.

### EXAMPLE 2

### Comparative expression of sTNALP-D10 and sTNALP-FcD10

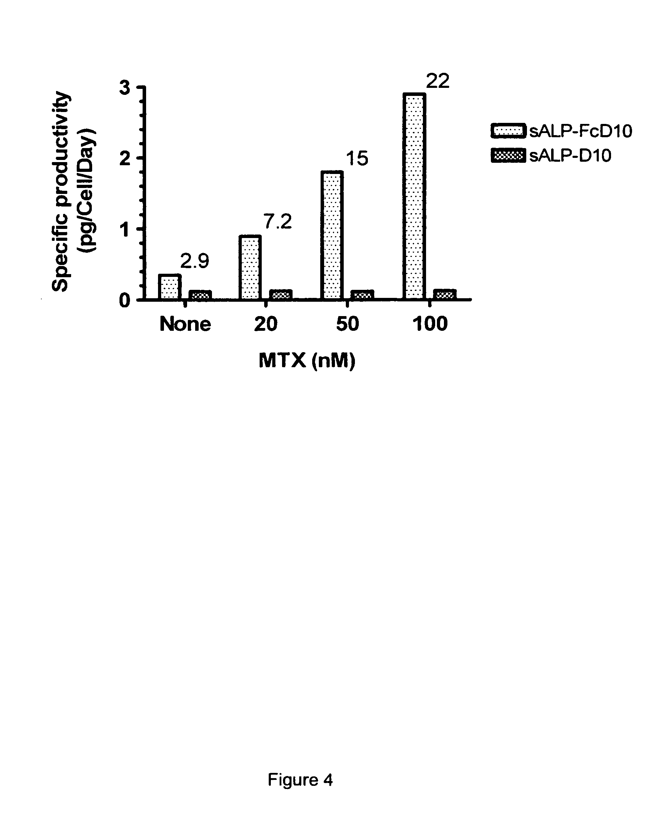

Plasmid vectors encoding either sTNALP-FcD10 or sTNALP-D10 were transfected in CHO-DG44 cells using Lipofectamine™ and grown in selective media (i.e. devoid of nucleotides) designed to promote survival of cells expressing the DHFR gene as described in Example 1 above. Stable transfectants were isolated by plaque cloning and ranked according to their level of protein expression using the alkaline phosphatase enzymatic assay also described in Example 1 above. Screening allowed the identification of one clone only for sTNALP-D10 (0.120 pg/cell/day) and five clones for sTNALP-FcD10 (0.377, 0.258, 0.203, 0.099 and 0.088 pg/cell/day). Methotrexate (MTX) gene amplification was performed as described in Example 1 above (MTX ranging from 0 to 100mM) and allowed an 8-fold expression increase for sTNALP-FcD10 while no amplification was observed with the sTNALP-D10 cultures (see Figure 4). Using a similar process for cell line development, it was unexpectedly found that the sTNALP-FcD10 protein was easier to express compared to sTNALP-D10 (see Figure 4).

### EXAMPLE 3

### sTNALP-FcD10 characterization

sTNALP-FcD10 was first purified on Protein-A Sepharose™ and was analyzed on SDS-PAGE under reducing and non-reducing conditions.

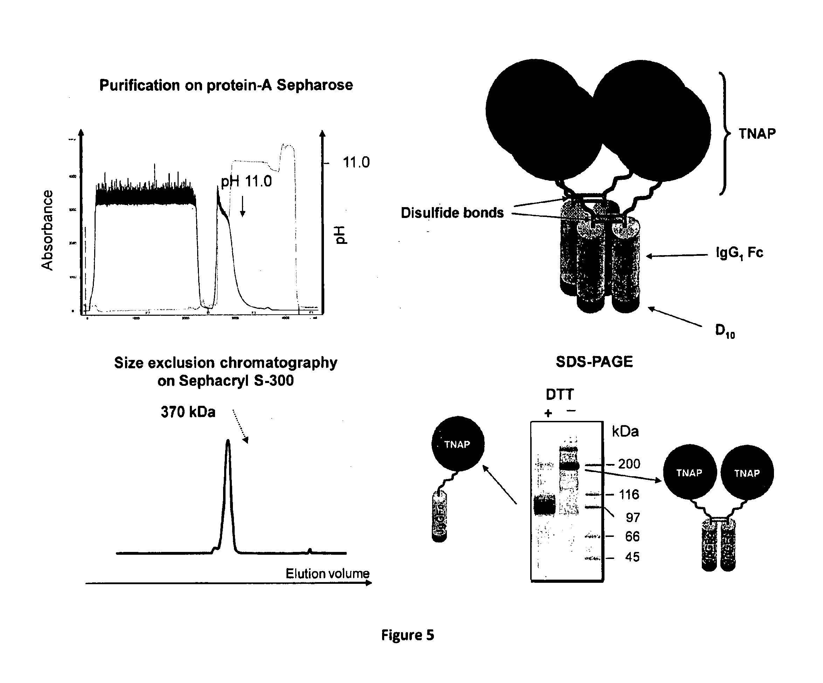

Under reducing conditions, it migrated as a broad band with an apparent molecular mass of ∼ 90,000 Da (DTT+ in Figure 5). Digestion with peptide N-Glycosidase F (PNGAse F) reduced the apparent molecular mass of the protein to about 80,000 which closely approximates the calculated mass of 80,500 Da for the non-glycosylated sTNALP-FcD10 monomer shown in Figure 1. Soluble TNALP in serum, like TNALP present as a GPI anchored protein on the outer surface of osteoblasts, is a highly glycosylated protein with carbohydrates comprising ≤ 20% of the total mass of the enzyme (Farley & Magnusson 2005). Although the specific carbohydrate structures on TNALP have not been identified, sequence studies indicate that the enzyme possesses five putative sites for N-linked glycosylation, and biochemical studies have shown evidence for both N- and O-linked carbohydrates (Nosjean et al. 1997). In agreement with these earlier observations, the electrophoretic migration of sTNALP-Fc010 and its sensitivity to PNGAse F suggests it is also a heavily N-glycosylated protein. Soluble TNALP in serum, like TNALP present as a GPI anchored protein on the outer surface of osteoblasts, is a highly glycosylated protein with carbohydrates comprising ≤ 20% of the total mass of the enzyme (Farley & Magnusson 2005).

When SDS-PAGE was repeated under non reducing conditions the apparent molecular mass of sTNALP-Fc010 was found to be 200,000 (DTT- in Figure 5) consistent with that of a homodimer as in native unaltered TNALP (Millán 2006). This homodimer likely results from the formation of two disulfide bridges between two monomeric Fc regions (upper right panel, Figure 5).

The molecular mass of purified sTNALP-FcD10 under native conditions was next evaluated using size exclusion FPLC chromatography on a column of Sephacryl™ S-300 (GE Health Care) equilibrated in 150 mM NaCl, 20 mM Tris pH 7.5 buffer. The column was previously calibrated with a standard protein kit (HMW calibration kit, GE Health care) (lower left panel, Figure 5).

Collected chromatography fractions were assayed for alkaline phosphatase enzymatic activity and the material in each peak. Surprisingly, 78% of the material eluted at a position corresponding to proteins of 370kDa (lower left panel, Figure 5) suggesting a tetrameric form for the native sTNALP-FcD10 recombinant enzyme produced in CHO cells. When fractions from the Sephacryl S-300 column were tested for activity, all of the enzymatic activity was associated with the 370 kDa fraction. The remaining material was of a much higher molecular weight indicating the formation of some sTNALP-FcD10 aggregates. Both the tetrameric forms, which may not be observed on the SDS-page because the latter destroys the non covalent binding maintaining the tetramer together, and aggregate forms were resolved as sTNALP-FcD10 monomers with an apparent molecular weight of 90,000 by SDS-PAGE under reducing conditions (DTT+, lower right panel in Figure 5) and as dimers with an apparent molecular weight of 200,000 in non reducing conditions (DTT-, lower right panel in Figure 5). Recombinant sTNALP-FcD10 appears to consist mainly of enzymatically functional homotetramers formed by non covalent association of two sTNALP-FcD10 disulfide-linked dimers.

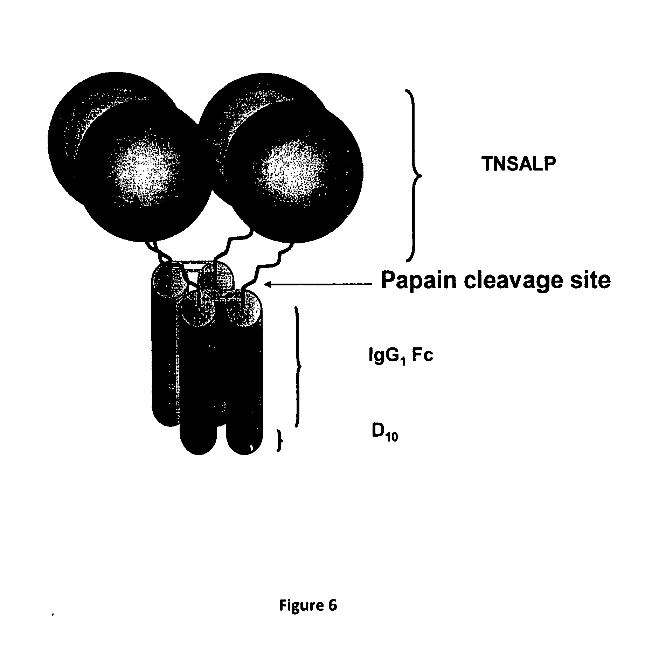

The tetrameric structure of sTNALP-FcD10 was further tested by limited papain digestion (Figures 6-8). This protease is known to cleave IgG heavy chains close to the hinge region and on the N-terminal side of the disulfide bonds, thereby generating whole monomeric Fab fragments and dimeric disulfide-linked Fc dimers. Digestion of sTNALP-FcD10 should thus liberate enzymatically active sTNALP dimers from the intact Fc domains (see Figure 6).

Aliquots containing 400 µg of sTNALP-FcD10 were digested with 208 mU of papain-agarose (Sigma) in a 20 mM phosphate buffer (pH 7.0) containing 250 µM dithiothreitol. Digestion was left to proceed at 37°C for 1 h under gentle agitation. The reaction was stopped by removing the papain-agarose beads by centrifugation. In those conditions, there was no significant loss of sTNALP-FcD10 enzymatic activity during the first 4 h of incubation. sTNALP-FcD10 incubated for one h in the presence or absence of papain-agarose was next analyzed by SEC-HPLC on a TSK-Gel G3000WXL (Tosoh Bioscience) in non denaturing conditions.

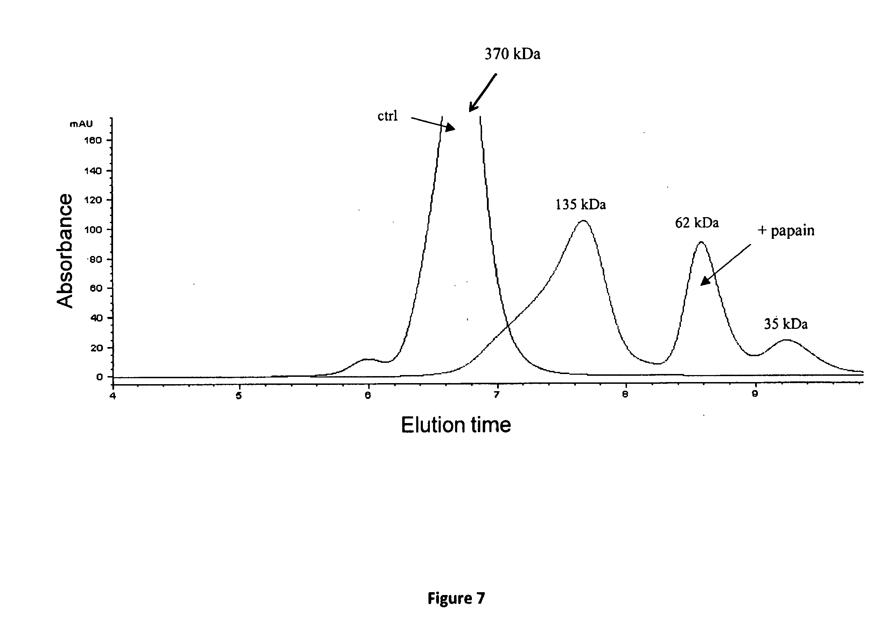

Figure 7 shows that the main product eluting with an apparent Mr of 370 kDa was no longer observed after a 1 h papain digestion. In those conditions papain digestion generates two main fragments of 135 kDa and 62 kDa respectively. A minor peak with Mr of 35 kDa was also observed.

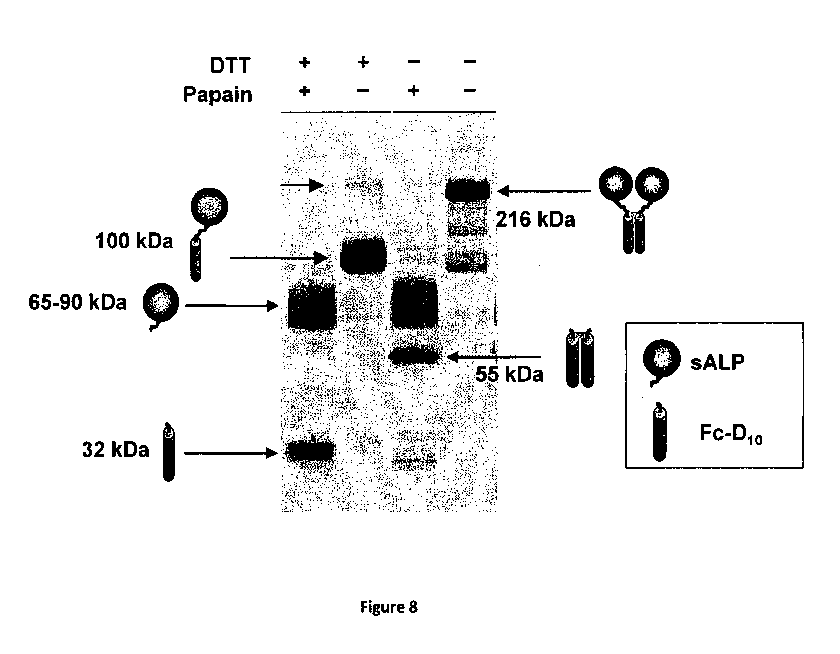

Under reducing SDS-PAGE conditions (DTT+, Figure 8) the product of the non papain treated sample was resolved into a major band (102 kDa) (DTT+, papain-), which was previously shown to correspond to monomeric sTNALP-FcD10. In Western blots this band can indeed be stained with antibodies for both TNALP and the Fc domain of the IgG₁ molecule (not shown). After papain digestion this band is cleaved into two major fragments: 1) The 32 kDa band, which binds the anti-Fc but not the anti-TNALP antibody and is proposed to correspond to the FcD10 fragment; and 2) The broad and diffuse protein band (66 - 90 kDa) which can be stained with the anti-ALP antibody but not with anti-Fc antibody and is thus thought to correspond to TNALP ectodomain monomers. The heterogeneity of this material is presumably due to its glycosylation as it can be reduced by digestion with Peptide-N-Glycosidase F, which also decreases its apparent molecular mass to 52 kDa (results not shown);

Under non-reducing conditions (DTT-, Figure 8), sTNALP-FcD10 incubated without papain was found to migrate in SDS-Page as a 216 kDa protein (DTT-, papain-, Figure 8). Western blotting also demonstrates that this protein contains epitopes for both the TNALP and Fc moieties (results not shown). This molecular species was previously proposed to consist of disulfide-bonded sTNALP-FcD10 dimers. As under reducing conditions, papain cleavage under non-reducing conditions (DTT-, papain +) generates two main fragments. In Western blots, the 55 kDa fragment can be stained with the anti-Fc but not with the anti-TNALP antibody. This fragment is most probably identical to the 62 kDa species observed on SEC-HPLC in native conditions and is proposed to correspond to disulfide-bonded Fc dimers. The other major species comigrates with the major protein band (66-90 kDa) observed under reducing conditions. This is consistent with it being composed of TNALP ectodomain monomers. When analyzed by HPLC in non denaturing conditions these monomers are non-covalently associated in the enzymatically active TNALP dimers eluting from the SEC column as the 135 kDa species.

### EXAMPLE 4

### Compared affinity for hydroxyapatite of sTNALP-FcD10 protein and bovine kidney sALP

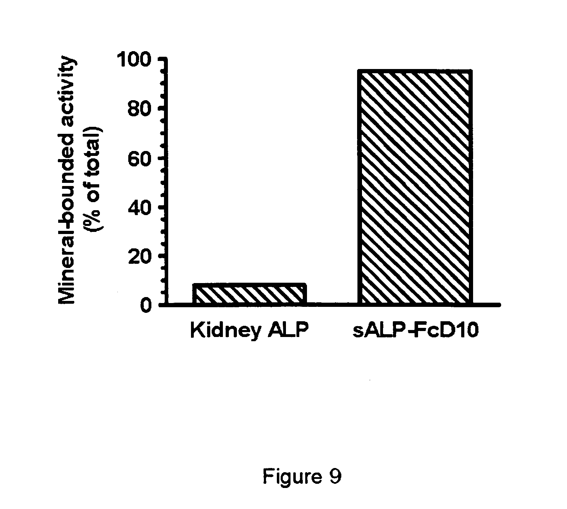

The affinity of the purified sTNALP-Fc010 protein for hydroxyapatite was also compared to that of bovine kidney (tissue non specific) soluble alkaline phosphatase (Calzyme) using the following procedure. Bovine kidney TNALP was used instead of human bone TNALP because it was commercially available. Hydroxyapatite ceramic beads (Biorad) were first solubilized in 1 M HCl and the mineral was precipitated by bringing back the solution to pH to 7.4 with 10 N NaOH. Binding to this reconstituted mineral was performed by incubating aliquots of the mineral suspension containing 750 µg of mineral with 5 µg of protein in 100 µl of 150 mM NaCl, 80 mM sodium phosphate pH 7.4, buffer. The samples were kept at 21 ± 2°C for 30 minutes on a rotating wheel. Mineral was spun down by low speed centrifugation and total enzymatic activity recovered in both the mineral pellet and the supernatant was measured. Figure 9 clearly shows that sTNALP-FcD10 binds more efficiently to reconstituted hydroxyapatite mineral than bovine kidney TNALP. Furthermore, most of the recombinant sTNALP-FcD10 protein introduced in the assay was recovered by summing up the enzymatic activity recovered in both the bound and non bound fractions. This indicates that binding of the recombinant protein to the reconstituted mineral phase does not significantly alter its enzymatic activity.

### EXAMPLE 5

### Mouse model

The Akp2^{-/-} mice were created by insertion of the Neo cassette into exon VI of the mouse TNALP gene (Akp2) via homologous recombination (Narisawa et al. 1997; Fedde et al. 1999). This mutation caused the functional inactivation of the Akp2 gene and no mRNA or TNALP protein is detectable in these knockout mice (Narisawa et al. 1997). Phenotypically, the Akp2^{-/-} mice mimic severe infantile HPP. These mice have no obvious hypophosphatasia phenotype at birth, skeletal defects usually appearing at or around day 6, and worsen thereafter. They have stunted growth with rickets, develop epileptic seizures and apnea, and were reported to die between postnatal days 12-16. Like HPP patients, Akp2^{-/-} mice feature hypophosphatasemia due to global deficiency of TNALP activity, endogenous accumulation of the ALP substrates, PPi, PLP and PEA and suffer impaired skeletal matrix mineralization leading to rickets or osteomalacia (Fedde et al. 1999).

To understand how defects in alkaline phosphatase can lead to neurological manifestations of the disease in both human and mice, one has to review the role and metabolism of Vitamin B6 in the CNS. Vitamin B6 is an important nutrient that serves as a cofactor for at least 110 enzymes, including those involved in the biosynthesis of the neurotransmitters γ-aminobutyric acid (GABA), dopamine and serotonin. Vitamin B6 can be found in three free forms (or vitamers), i.e., pyridoxal (PL), pyridoxamine (PM), and pyridoxine (PN), all of which can be phosphorylated to the corresponding 5'-phosphated derivatives, PLP, PMP and PNP (Jansonius 1998). Removal of the phosphate group is a function of ALP, and primarily that of the TNALP isozyme (Whyte 2001). Since only dephosphorylated vitamers can be transported into the cells, decreased TNALP activity in Hypophosphatasia results in marked increases in plasma PLP (Whyte et al. 1985; Whyte 2001) and intracellular deficiency of PLP in peripheral tissues and the central nervous system where it leads to reduced brain levels of GABA. It has also been hypothesized that the epileptic seizures observed in these mice result from glutamic acid decarboxylase dysfunction due to shortage of PLP (Waymire et al. 1995).

Pyridoxine supplementation suppresses the epileptic seizures of Akp2^{-/-} mice but extends their lifespan only a few days, till postnatal days 18-22 (Narisawa et al. 2001). Therefore, all animals in this study (breeders, nursing moms, pups and weanlings) were given free access to a modified laboratory rodent diet 5001 with increased levels (325 ppm) of pyridoxine.

Akp2^{-/-} mice (12.5 % C57BL/6 - 87.5%129J hybrid background) were maintained by heterozygote breeding. Animals, breeder pairs or nursing moms with their pups, were housed in a ventilated solid bottom plastic cage equipped with an automatic watering system. All animals had free access to a modified laboratory rodent diet 5001 with 325 ppm pyridoxine (#48057, TestDiet™). Maximum allowable concentrations of contaminants in the diet (e.g. heavy metals, aflatoxin, organophosphate, chlorinated hydrocarbons, PCBs) were assured by the manufacturers. No known contaminants were present in the dietary material to influence the toxicity of the test article.

### EXAMPLE 6

### Pharmacokinetics and tissue distribution of injected sTNALP-FcD10 in WT mice

### Blood sample collection

Blood samples were collected into heparin lithium tube (VWR, #CBD365958), put on ice for a maximum of 20 minutes before centrifugation at 2500g for 10 min at room temperature. At least, 15 µl of plasma was transferred into 0.5 ml tube (Sarstedt, #72.699), frozen in liquid nitrogen and kept at -80°C until assayed. If available, another ≤ 50 µl of plasma was transferred into 0.5 ml tube, inactivated at 65°C for 10 minutes, frozen in liquid nitrogen and kept at -80°C until assayed. Any remaining plasma, was pooled with the 15 µl aliquot, frozen in liquid nitrogen and kept at -80°C until assayed.

### Determination of plasma sTNALP-FcD10

Presence of sTNALP-FcD10 in plasma samples was assessed upon completion of treatment using a colorimetric enzymatic assay. Enzymatic activity was determined using a chromogenic substrate where increase of absorbance is proportional to substrate conversion to products. The reaction was carried out in 100 µl of buffer 50 mM NaCl, 20 mM Bis Tris Propane (HCl) pH 9 buffer containing 0.5 mM MgCl₂ and 50 µM ZnCl₂ to which was added 10 µl of diluted plasma sample. The ALP substrate p-nitrophenyl was added last at a final concentration of 1 mM to initiate the reaction. The absorbance was recorded at 405 nm every 45 seconds over a twenty minutes period using a Spectramax™ 190 (Molecular devices) plate reader. sTNALP-FcD10 enzymatic activity expressed as an initial rate of reaction was assessed by fitting the steepest slope over 8 adjacent reading values. Standards were prepared with varying concentrations of test article and the enzymatic activity was determined as described above in Example 1. The standard curve was generated by plotting Log of the initial speed rate as a function of the Log of the standard quantities. sTNALP-FcD10 concentration of the different plasma samples was read directly from the standard curve using their respective enzymatic activity.

### Determination of plasma PPi

Circulating levels of PPi were measured in serum obtained from cardiac puncture using differential adsorption on activated charcoal of UDP-D-[6-³H]glucose (Amersham Pharmacia) from the reaction product of 6-phospho[6-³H]gluconate, as previously described (Johnson et al. 1999).

### Half-life and tissue distribution of sTNALP-FcD10

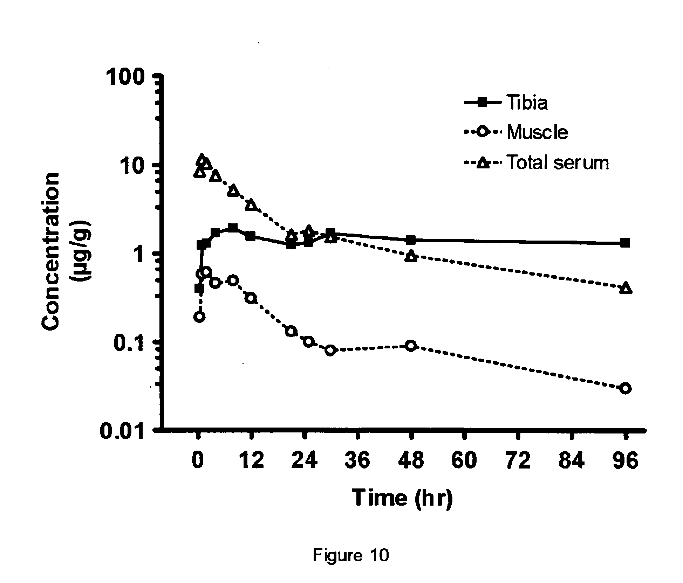

**In adult WT mice**, the half-life and tissue distribution of sTNALP-FcD10 injected into mice were determined. Figure 10 summarizes its pharmacokinetics and tissue distribution after a single, bolus intravenous injection of 5 mg/kg into adult WT mice.

The half-life was 34 h in blood with an accumulation of the [¹²⁵I]-labeled sTNALP-FcD10 in bone of up to 1 µg/g of bone (wet weight). This half-life is comparable to that observed previously in unsuccessful reported clinical trials. Levels of bone-targeted material seemed quite stable, as no significant decrease in radiolabeled sTNALP-FcD10 was observed during the experiment. No accumulation of sTNALP-FcD10 was observed in muscle, as the amount of radiolabeled enzyme in that tissue decreased in parallel with that of sTNALP-FcD10 enzymatic activity in blood.

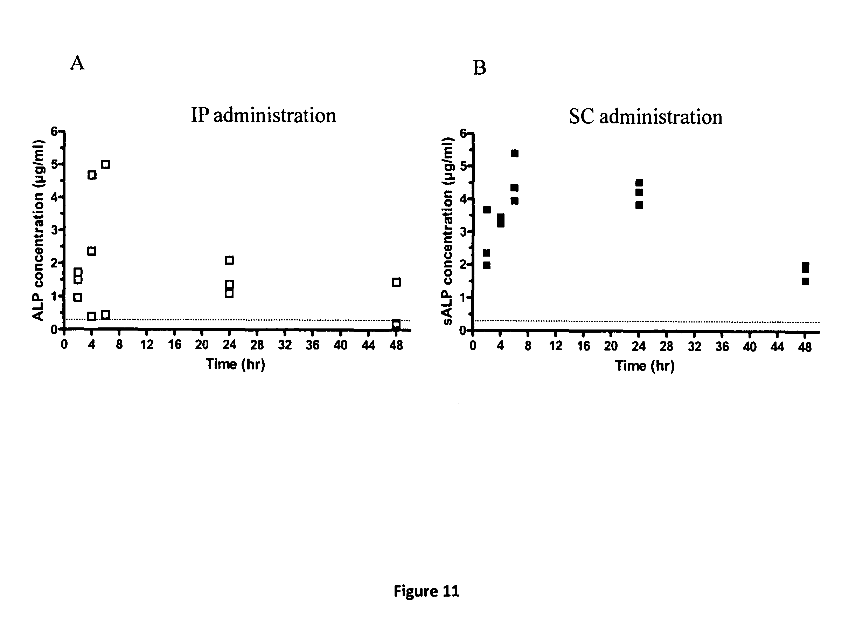

**In newborn mice.** Because Akp2^{-/-} mice die between days 12-16 and i.v. injection is not feasible in such young mice, the pharmacokinetic analysis of sTNALP-FcD10 in serum was repeated using the i.p. and s.c. routes in WT newborn mice using a dose of 3.7 mg/Kg. The i.p. route was found inadequate due to the high pressure in the abdominal cavity leading to unpredictable losses through the injection site (Figure 11A). The s.c. route was more reproducible in newborn mice, as seen in the PK experiment of Figure 11B. The pharmacokinetic parameters of sTNALP-FcD10 in newborn and adult mice is reported in Table 2 below.

**Table 2: Pharmacokinetic parameters of sTNALP-FcD10 in newborn WT mice.**

| Parameter | Newborn | |
|---|---|---|
| | s.c. | i.p. |
| T1/2 (h) | 31 | 19 |
| Tmax (h) | 6 | 6 |
| Cmax (mg/L) | 5 | 3 |
| AUCinf (mg/L/h) | 257 | 92 |

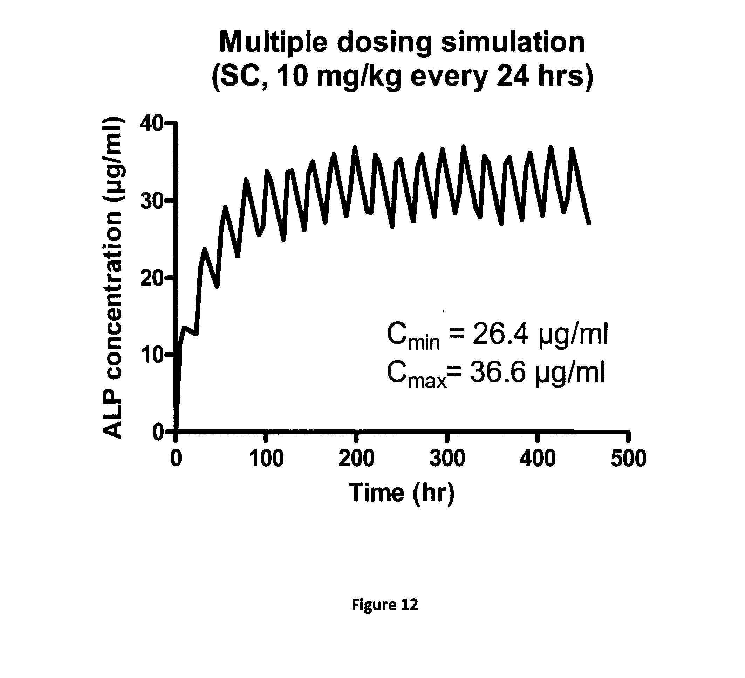

These PK data, analyzed by WinNonlin™ software (Pharsight Corporation, Mountain View, CA), were used to predict circulating blood levels of sTNALP-FcD10 achieved after repeated daily s.c. injections. Circulating sTNALP-FcD10 reached steady state serum concentrations oscillating between Cmin and Cmax values of 26.4 and 36.6 µg/ml, respectively (Figure 12). Steady state was achieved after 5 to 6 daily doses of 10 mg/kg.

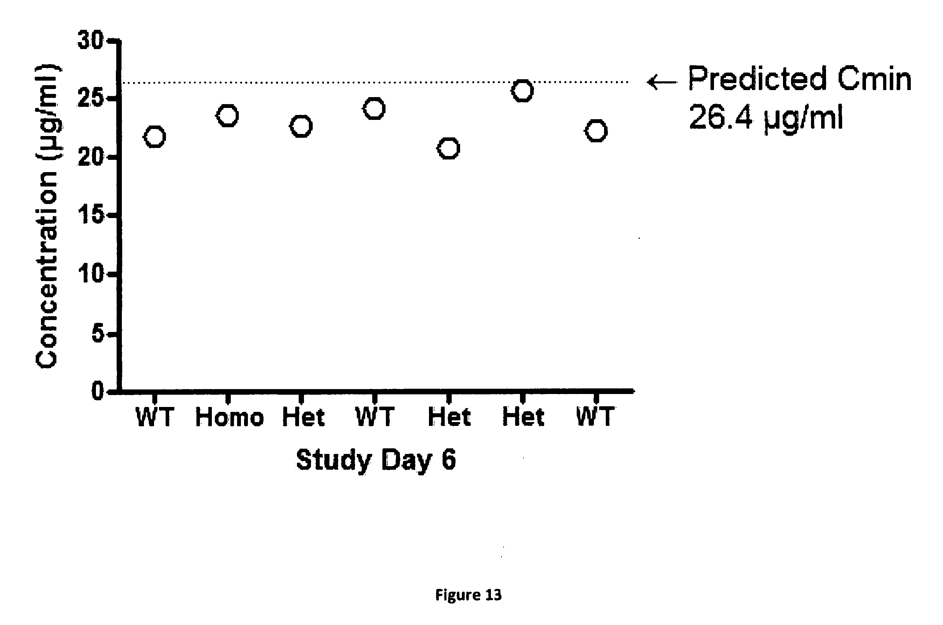

Prediction validity was tested experimentally after 5 daily injections of 10 mg/kg of sTNALP-FcD10. At the day of injection, the mice genotype could not be distinguished. It was later determined which amongst the mice tested were heterozygous or homozygous. There was no difference in the behavior of all the different genotypes. When circulating ALP activity was measured 24 h after the last injection, namely on day 6, (Cmin), good agreement was observed between experimental and predicted concentrations (Figure 13). In these non treated 5 day old animals, serum TNALP levels were 0.58 µg/ml. These levels will decrease with age. Thus, it was calculated that the injection regimen allowed building up to steady state serum concentrations of sTNALP-FcD10 approximately 50 times higher than normal TNALP concentrations.

### EXAMPLE 7

### Concentration of sTNALP-FcD10 in adult WT mice bones after bolus intravenous administration

A 5 mg/kg sTNALP-FcD10 dose was administered i.v. in 129J adult WT mice. The sTNALP-FcD10 concentration in bone at T=25 hours was as follows: 0.64 µg/g calvaria; 1.33 µg/g tibias; and 1.37 µg/g femurs, for a mean concentration of 1.11 µg/g. In rat, bone tissues represent 16.3% of total mass. It is expected that this percentage is also found in mice. The body weight of mice used for this experiment was 18.4 g. The calculated bone tissue weight of these mice was thus about 18.4 g x 0.163 = 3.00 g. The calculated quantity of sTNALP-FcD10 in bone tissues was of 3.33 µg. The percentage of the injected dose in bone tissues was thus of (3.33µg/(5µg/g * 18.4g))*100 = 4%.

The sTNALP-FcD10 concentration in bone at T=96 hours was as follows: 0.83 µg/g calvaria; 1.33 µg/g tibias and1.63 µg/g femurs, for a mean concentration of 11.26 µg/g. The body weight of mice used for this experiment was 17.8 g. The calculated bone tissue weight of these mice was thus about 17.8 g x 0.163 = 2.90 g. The quantity ofsTNALP-FcD10 in mice bone tissues was thus about 3.66 µg. The percentage of the injected dose in bone tissues was thus of (3.66µg/(5µg/g * 17.8g))*100 = 4%.

### EXAMPLE 8

### Concentration of sTNALP-FcD10 in newborn WT mice bones after 15 days bolus subcutaneous injection

A 4.3 mg/kg sTNALP-FcD10 dose was administered subcutaneously in 129J newborn WT mice every day for 15 days for a total administered amount of 65 mg/kg. The sTNALP-FcD10 concentration in bone at T=24 hours was as follows: 6.45 µg/g calvaria; 3.05 µg/g tibias; and 3.71 µg/g femurs, for a mean concentration of 4.40 µg/g. The body weight of mice used for this experiment was 9.83 g. The calculated bone tissue weight of these mice was thus about 9.83 g x 0.163 = 1.60 g. The quantity of sTNALP-FcD10 in mice bone tissues at that time was thus about 7.04 µg. The percentage of the injected dose in bone tissues was thus of (7.04µg/(65µg/g * 9.83g))*100 = 1%.

The sTNALP-FcD10 concentration in bone at T=168 hours was as follows: 5.33 µg/g calvaria; 1.37 µg/g tibias; and 1.88 µg/g femurs, for a mean concentration of 2.86 µg/g. The body weight of mice used for this experiment was 14.0 g. The calculated bone tissue weight of these mice was thus about 14.0 g x 0.163 = 2.28 g. The quantity of sTNALP-FcD10 in mice bone tissues at that time was thus about 6.52 µg. The percentage of the injected dose in bone tissues was thus of (6.52µg/(65µg/g * 14g))*100 = 0.7%. Table 3 below summarizes results of Examples 7 and 8.

**Table 3: Mean concentration of sTNALP-FcD10 and percentage of injected dose in bones**

| Experiment | Injection regimen | Mean concentration in bones (µg/g wet tissue) | | % of injected dose in bones | |
|---|---|---|---|---|---|
| IV Bolus | 1 x 5 mg/kg (bolus) | T = 25 h⁽¹⁾ | T = 96 h⁽¹⁾ | T = 25 h⁽¹⁾ | T = 96 h⁽¹⁾ |
| | | 1.11 | 1.26 | 4% | 4% |
| SC Bolus over 15 days | 15x4.3 mg/kg (daily) | T = 24 h⁽¹⁾ | T = 168 h⁽¹⁾ | T = 24 h⁽¹⁾ | T = 168 h⁽¹⁾ |
| | | 4.40 | 2.86 | 1% | 0.7% |

| | | | | | |
|---|---|---|---|---|---|
| ⁽¹⁾ Times indicated are from the last injection. | | | | | |

### EXAMPLE 9

### Short-term (15 days) efficacy of low doses (1 mg/kg) of sTNALP-FcD10 for HPP in Akp2^{-/-} mice

Daily s.c. injection of sTNALP-FcD₁₀ were performed for 15 days in Akp2^{-/-} mice using 1 mg/kg. Treatment groups were constituted from 19 litters. Akp2^{-/-} mice received vehicle (N=13) or sTNALP-FcD10 (N=12).Controls consisted of 15 WT mice (one per litter). Controls were not submitted to injections. Blood was taken 24 h after the last injection as described in Example 6.

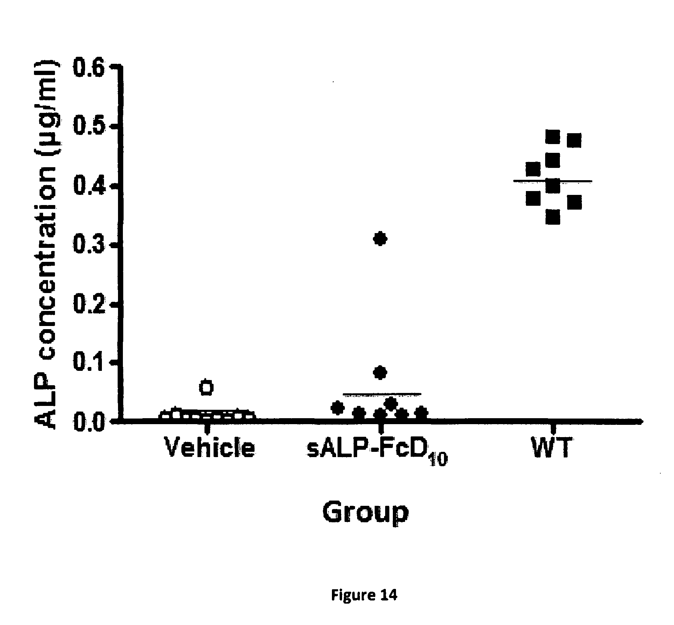

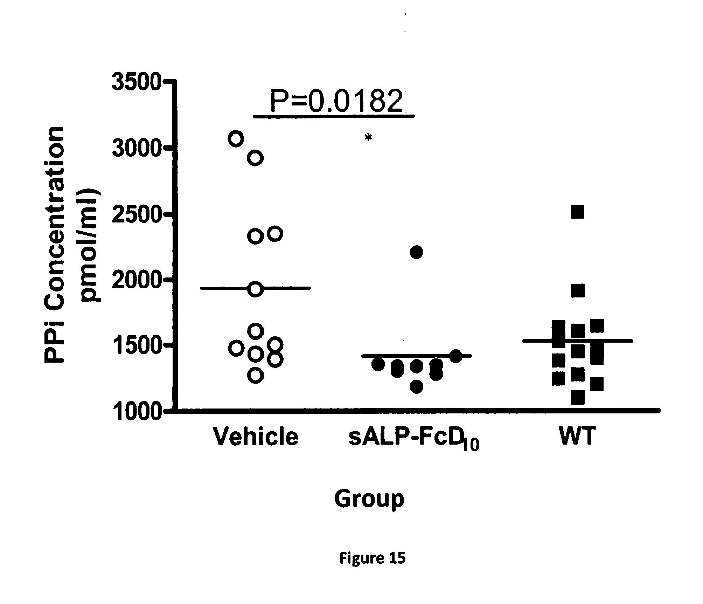

Figure 14 shows that enzyme activities in serum at day 16 were barely above the detection level. Despite low serum values for sTNALP-FcD10, serum PPi levels were corrected (Figure 15). Untreated Akp2^{-/-} mice had serum PPi concentrations of 1.90 ± 0.64 µmol/ml, whereas treated Akp2^{-/-} mice had levels of 1.41 ± 0.30 µmol/ml, comparable to those of WT mice (1.52 ± 0.35 µmol/ml).

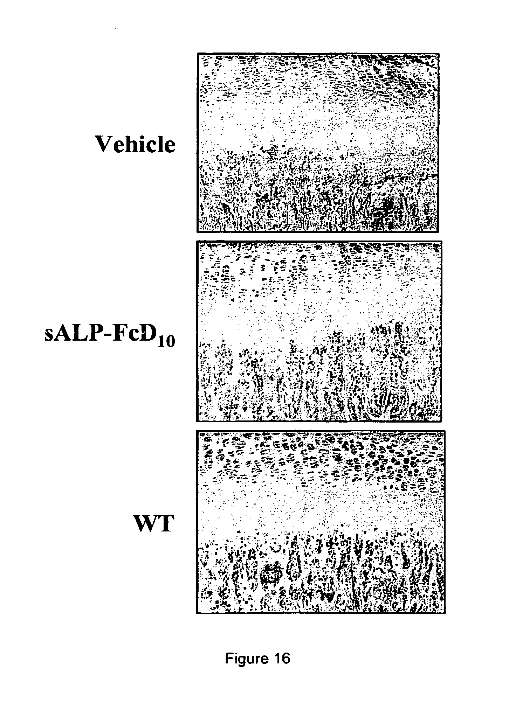

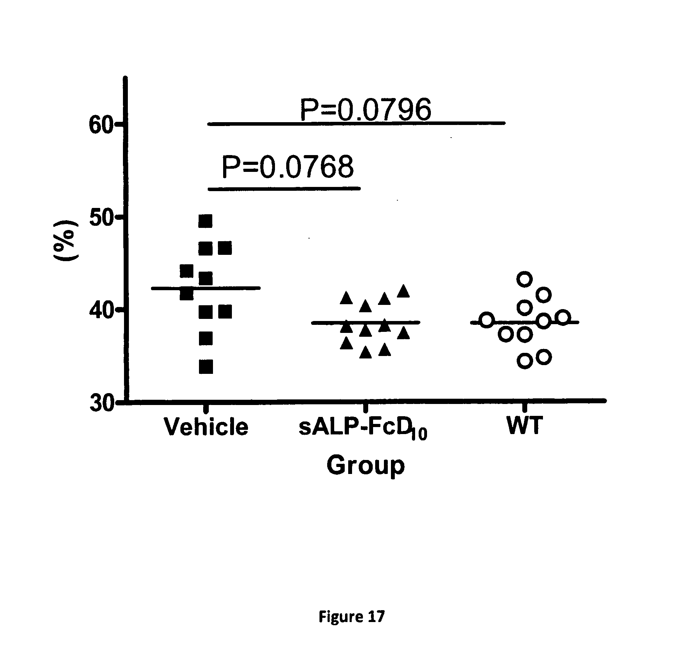

Proximal tibial growth plates showed some widening of the hypertrophic zone in Akp2^{-/-} animals compared WT animals (compare vehicle with wild-type in Figure 16). The same observation made earlier in this strain of Akp2^{-/-} mice (Hessle et al. 2002) is consistent with rickets. A trend toward normalization of the physeal morphology was observed in animals treated with sTNALP-FcD10 for 15 days (Figure 17) compared to vehicle (untreated).

### EXAMPLE 10

### Short-term (15 days) efficacy of high doses (8.2 mg/kg) of sTNALP-FcD10 for HPP in Akp2^{-/-} mice

To evaluate 15 days of daily s.c. injections using a significantly higher dose of sTNALP-FcD10 (8.2 mg/kg) on growth and bone mineralization, mice from 20 litters (141 mice total) were used. They were distributed to two groups: 1) Akp2^{-/-} mice given vehicle (N = 19); 2) Akp2^{-/-} mice treated with sTNALP-FcD10 (N = 20); additionally, there was one WT mouse per litter, non treated (N = 18).

### Body weight

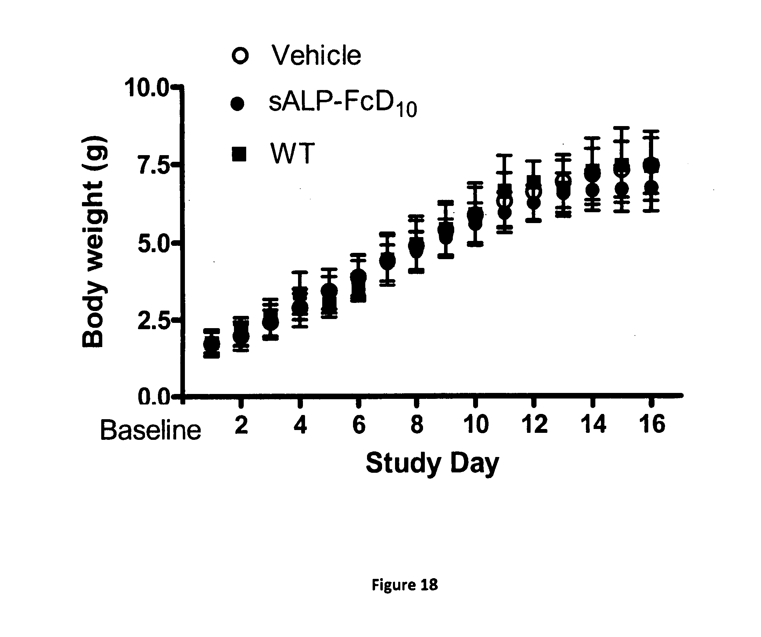

Akp2^{-/-} mice grew more slowly than WT mice. At day 1, no statistical difference in body weights was observed among the vehicle, sTNALP-FcD10, and WT animals. However, daily mean body weights diverged at day 6 (Figure 18). The difference between WT (4.2 ± 0.6 g) and vehicle (3.7 ± 0.7 g) achieved statistical significance (p=0.0217) at day 6; but the difference between vehicle (5.9 ± 1.0 g) and sTNALP-FcD10 treated values (6.7 ± 1.0 g) achieved statistical significance at day 11 (p=0.04), with the treated group paradoxically heavier than WT. At day 16, mean body weight of treated animals (8.2 ± 1.1 g) and WT (8.4 ± 0.8 g) were not statistically different. Animals treated with sTNALP-FcD10 had body weights statistically greater (p=0.026) than those treated with vehicle (6.6 ± 1.4 g). No significant difference between the ERT and WT groups was observed for body weight at any time point.

### Bone length

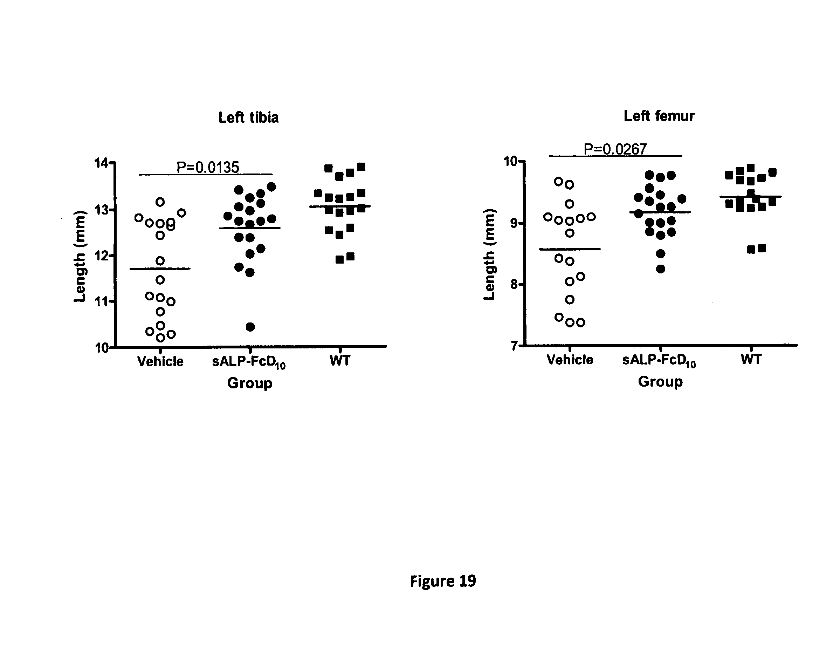

At the end of this experiment (day 16), tibial length provided an additional measure of skeletal benefit for Akp2^{-/-} mice. The tibia length with ERT was 12.6 ± 0.7 mm and longer (p=0.0135) compared to animals given vehicle (11.7 ± 1.1 mm) (Figure 19). A statistical difference (p=0.0267) was also obtained when femur length was compared between the sTNALP-FcD10 (9.2 ± 0.4 mm) and vehicle (8.6 ± 0.8 mm) groups. No statistical difference was noted for tibia or femur length of the ERT compared to WT mice. A partial preservation (i.e. partial prevention of reduction in bone growth that becomes apparent around two weeks of age) of tibia and femur growth was observed by measures of length at necropsy (Figure 19).

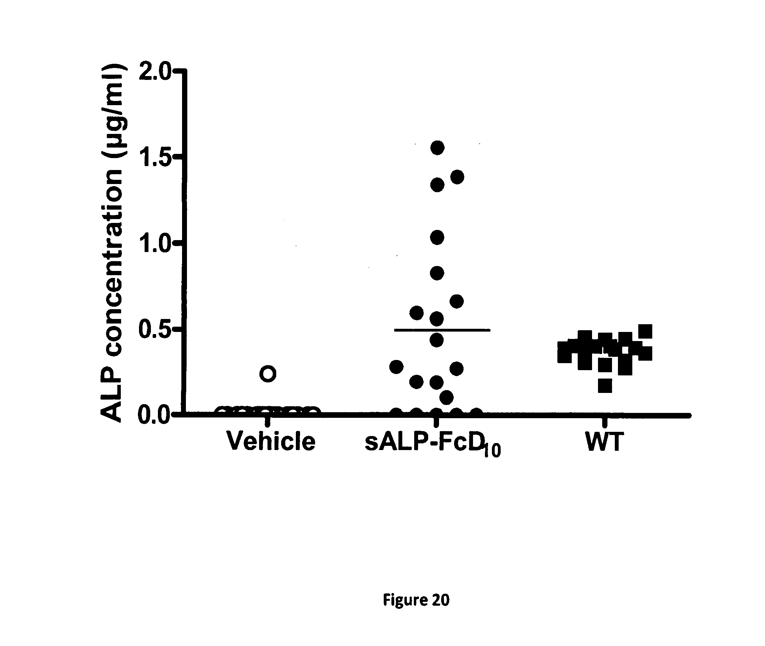

In all but 5 animals, detectable, but highly variable, levels of sTNALP-FcD10 were found in the plasma of treated Akp2^{-/-} mice at day 16 (Figure 20). Circulating TNALP concentrations in normal animals are given for comparison purposes.

### Bone mineralization

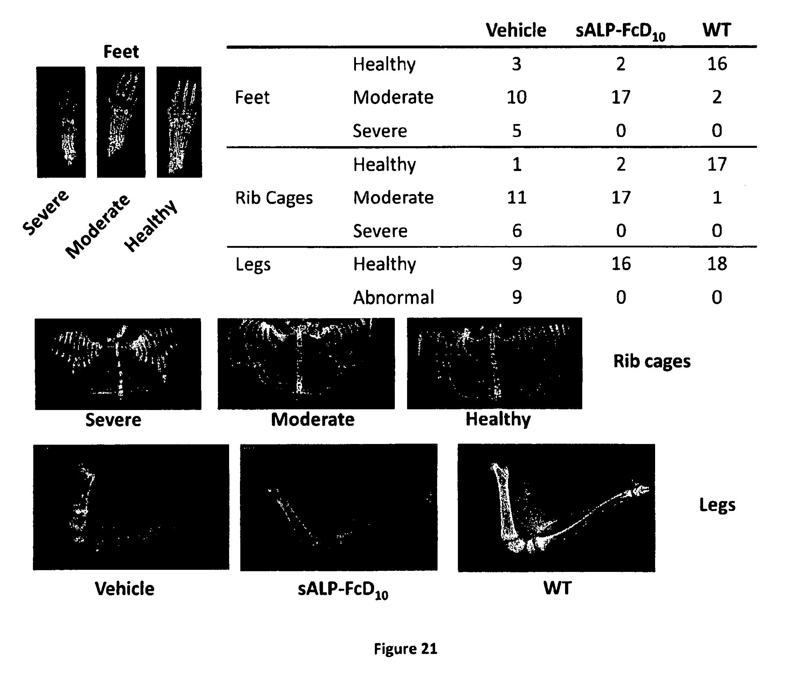

Blinded evaluations of Faxitron™ images of the feet and rib cages distinguished two degrees of severity of mineralization defects in the Akp2^{-/-} mice (Figure 21). Severely affected mice (Severe) had an absence of digital bones (phalanges) and secondary ossification centers. Moderately affected (Moderate) mice had abnormal secondary ossification centers, but all digital bones were present. WT mice (Healthy) had all bony structures present with normal architecture. Radiographic images of the hind limbs were similarly classified as abnormal if evidence of acute or chronic fractures was present, or healthy in the absence of any abnormal findings (Figure 21). ERT minimized mineralization defects in the feet documented by the number of Akp2-/- mice with severe defects, consisting of 5 in the untreated group yet 0 in the ERT group (Table in Figure 21). Chi-Square was significant (p ≤ 0.05), indicating ERT decreased the severity of the acquired bone defects. Because severely affected infantile HPP patients often die from undermineralized and fractured ribs incapable of supporting respiration, the thoraces were also closely examined. ERT also reduced the incidence of severely dysmorphic rib cages (Table in Figure 21). Chi-Square analysis was significant at p ≤ 0.025. Similarly, the hind limbs appeared healthy in all treated animals (Table in Figure 21). Chi-Square analysis was significant at p ≤ 0.025.

### Dental defects

Mandibles from 16-day-old mice were immersion-fixed overnight in sodium cacodylatebuffered aldehyde solution and cut into segments containing the first molar, the underlying incisor, and the surrounding alveolar bone. Samples were dehydrated through a graded ethanolseries and infiltrated with either acrylic (LR White) or epoxy (Epon 812) resin, followed by polymerization of the tissue-containing resin blocks at 55°C for 2 days. Thin sections (1 µm) were cut on an ultramicrotome using a diamond knife, and glass slide-mounted sections were stained for mineral using 1% silver nitrate (von Kossa staining, black) and counterstained with 1% toluidine blue. Frontal sections through the mandibles (at the same level of the most mesial root of the first molar) provided longitudinally sectioned molar and cross-sectioned incisor for comparative histological analyses.

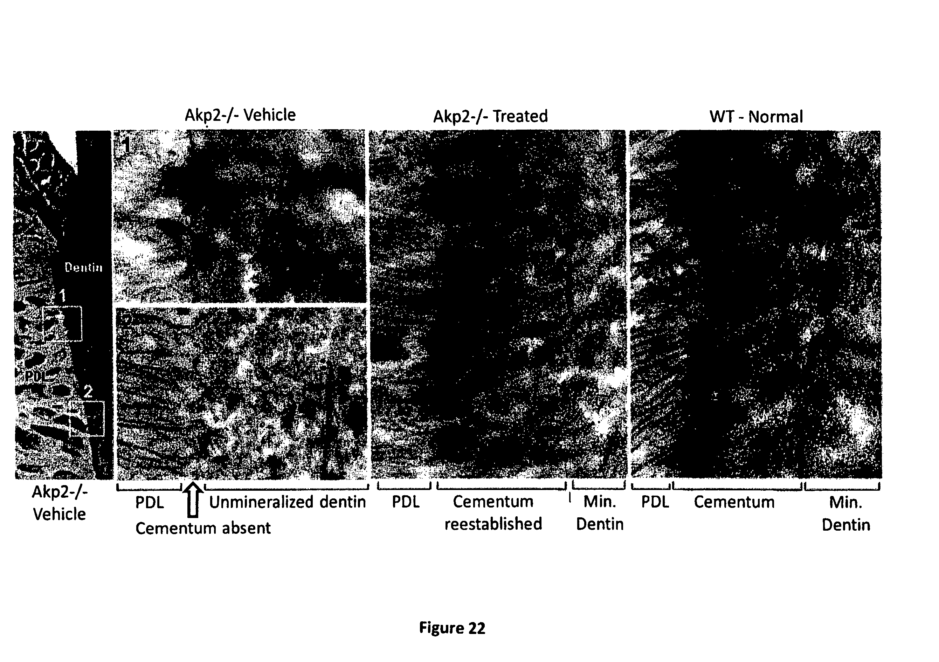

Histological examination of teeth from Akp2^{-/-} mice, shows poorly mineralized dentin tissue and very little cementum between the periodontal ligament and the dentin as compared to wild-type animals (Figure 22, compare Akp2^{-/-} Vehicle and WT-Normal). Restored dentin mineralization and the formation of the cementum is also shown in Figure 22 (Akp2^{-/-} Treated vs. WT-Normal).

### EXAMPLE 11

### Long-term (52 days) efficacy of high doses (8.2 mg/kg) of sTNALP-FcD10 for HPP in Akp2^{-/-} mice

Finally, to assess long-term survival and bone mineralization in Akp2^{-/-} mice, either sTNALP-FcD10 (8.2 mg/kg) or vehicle was given daily for 52 days (s.c. injections).

### Mice survival, activity and appearance

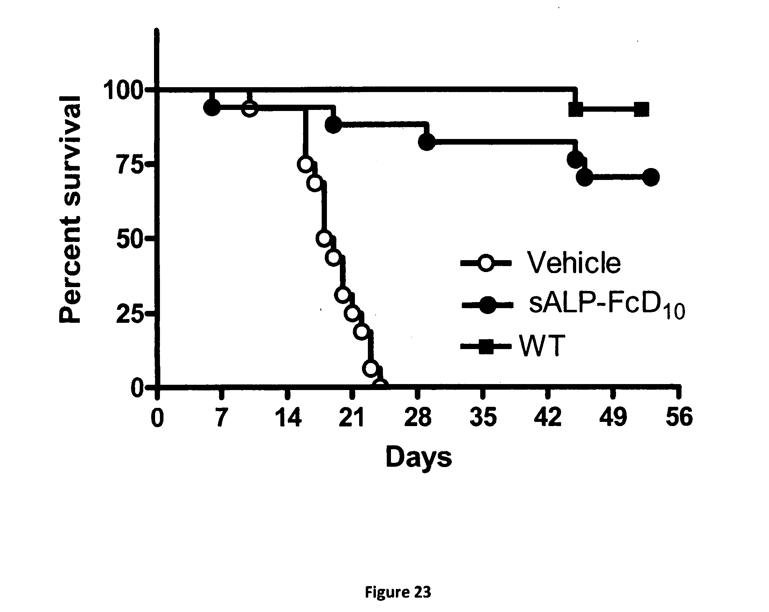

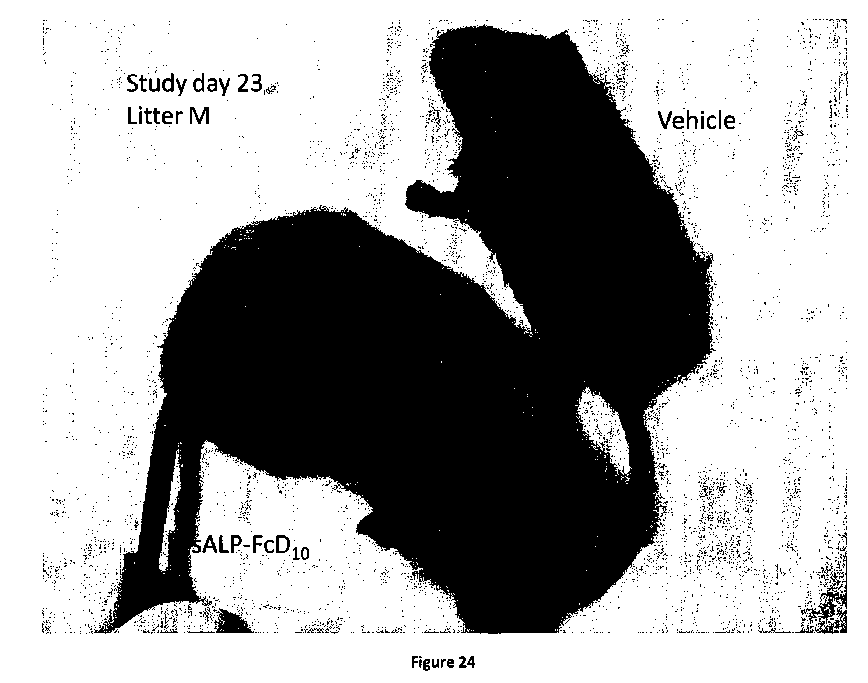

Untreated mice had a median survival of 18.5 days (Figure 23) whereas survival was dramatically increased with ERT and this treatment also preserved the normal activity and healthy appearance (Figure 24) of the treated mice.

### Bone mineralization

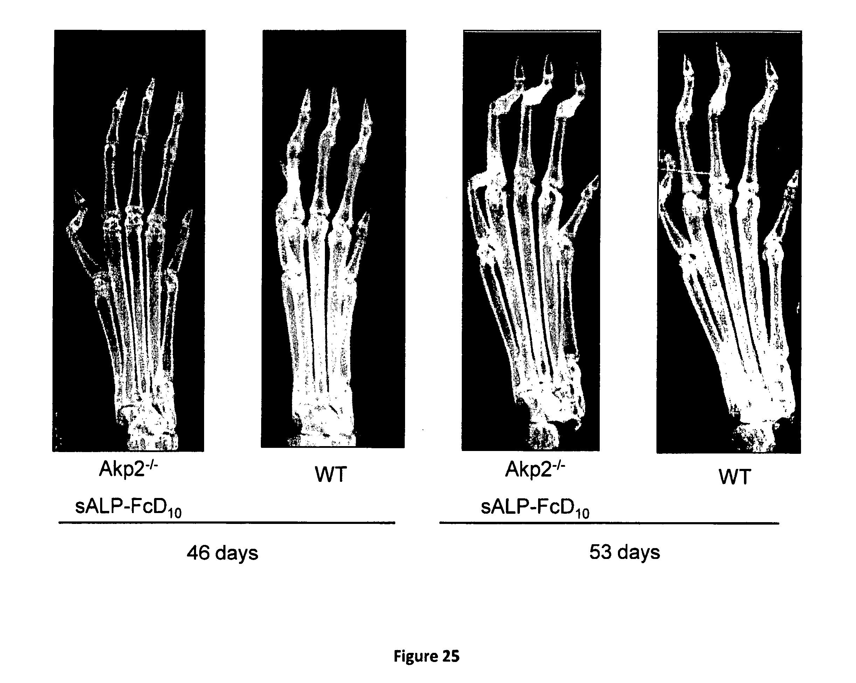

Radiographs of the feet of 16 day-old Akp2^{-/-} mice showed secondary ossification defects that are a hallmark of the disease (see Figure 25). These defects were prevented in all treated mice by daily doses of sTNALP-FcD10 for 46 or 53 days (Figure 25).

### ALP activity

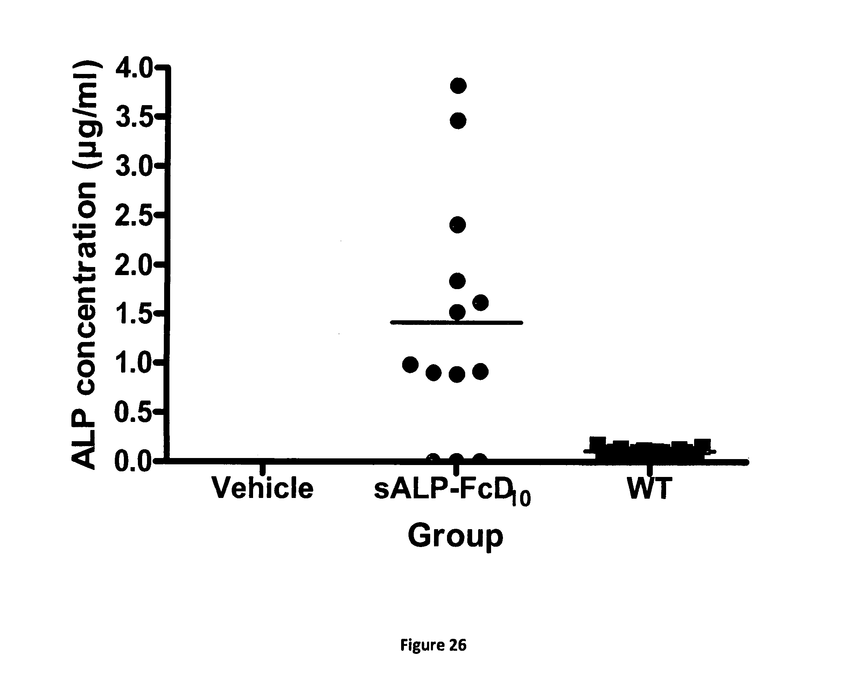

Plasma ALP activity levels were measured in treated Akp2^{-/-} mice after 53 days. Figure 26 shows that most of the values were between 1 and 4 µg/ml of ALP activity. Three animals, however, had undetectable ALP levels.

Interestingly, unlike WT mice where a steady-state serum concentration of sTNALP-FcD10 was achievable, great variability in the serum levels of ALP was measured in the treated Akp2^{-/-} mice.

### EXAMPLE 12

### Long-term efficacy of different dosage intervals of sTNALP-FcD10 in Akp2^{-/-} mice

Newborn Akp2^{-/-} mice were injected with 4.3 mg/kg daily (Tx-1), 15.2 mg/kg every 3 days (Tx-3) or 15.2 mg/kg every 7 days (Tx-7) of sTNALP-FcD10. Treatment was pursued for 43 days and mice were sacrificed on day 44, namely 24 hours after the last injection. They were monitored to evaluate any improvement of their survival and skeletal mineralization.

### Mice survival

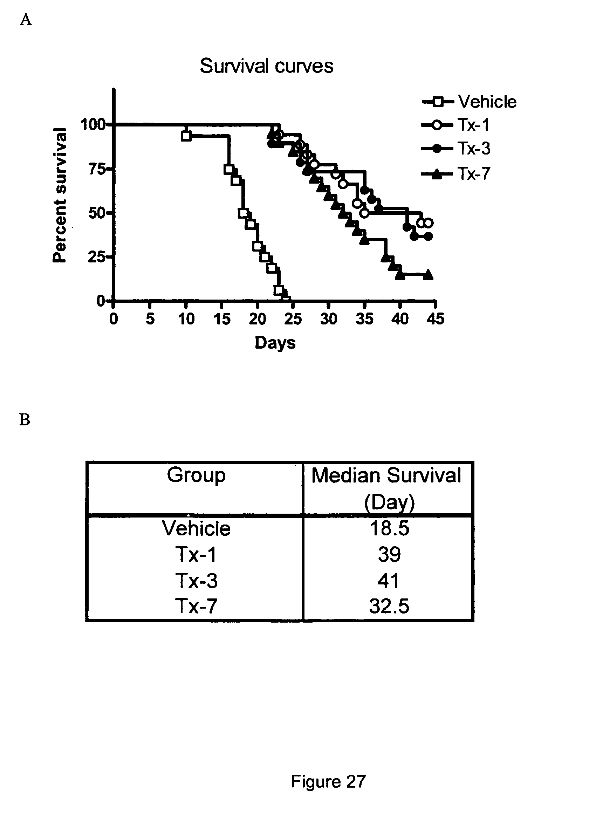

The survival of treated mice was increased compared to the mice that were injected vehicle (Figure 27). This increase was statistically significant (p<0.0001).There was no statistically significant difference when the survival curves of treated groups were compared between themselves.

### Bone mineralization

A) For each treatment, the radiographs of the feet were analyzed and distributed between normal and abnormal. Numbers and percentages (in parentheses) appear in Table 4 below. The bone mineralization defects were evaluated at day 23 and at the end of the study (Day 23-45).

**B) Table 4: Distribution of radiographs of feet**

| Mid-Study (D23) | | |
|---|---|---|
| **Group** | **Abnormal (%)** | **Normal (%)** |
| **Tx-1 (N=18)** | 6(33) | 12 (67) |
| **Tx-3 (N=19)** | 4(21) | 15(79) |
| **Tx-7 (N=20)** | 10 (50) | 10 (50) |
| **WT (N=32)** | 0(0) | 32 (100) |
| **B)** | | |

| D23-45 | | |
|---|---|---|
| **Group** | **Abnormal (%)** | **Normal (%)** |
| **Tx-1 (N=18)** | 3(17) | 15 (83) |
| **Tx-3 (N=19)** | 0(0) | 19 (100) |
| **Tx-7 (N=20)** | 3 (15) | 17(85) |
| **WT (N=31)** | 0(0) | 31 (100) |

At mid study, sTNALP-FcD10 administered at 15.2 mg/kg every 3 days normalized bone mineralization defects in 79% of mice. This rate of normalization approached statistical significance when compared to the 50% rate of normalization evaluated in the mice treated with 15.2 mg/kg every 7 days (Chi Square; p= 0.0596). No other inter treatment comparisons were statistically significant or approached significance.

At end of study, the percent of normalization improved in all treated groups compared to the percent normalization evaluated at day 23. The Chi Square test comparing the distribution among all sTNALP-FcD10 treatments was not significant (p=0.1844). The 100% rate of normalization observed in the mice treated with every 3 days approached statistical significance when compared to the rate in mice treated daily (83%, p=0.0634) or every 7 days (85%, p=0.0789).

However, in all treatment groups a significant proportion of the animals classified as abnormal at day 23 improved and became normal at end of the study. In the daily treatment group, 3 out of 6 animals normalized; in the mice treated every 3 days, 4 out of 4 improved, and finally in the weekly treatment group, 7 out of 10 became normal. Although dosage intervals provide satisfying results, the best results were obtained when the resulting daily amount administered was the highest.

### EXAMPLE 13

### Long-term efficacy of high doses (8.2 mg/kg) of sTNALP-FcD10 in 15 day old Akp2^{-/-} mice

Efficacy studies as described in Example 11 were conducted in 15 day old mice which have started to manifest skeletal defects as observed on X-ray pictures of feet (see Example 11, Figure 25). sTNALP-FcD10 was administered until the end of the study. The animals were monitored to evaluate any improvement of their survival, body weight and skeletal mineralization.

### Mice survival

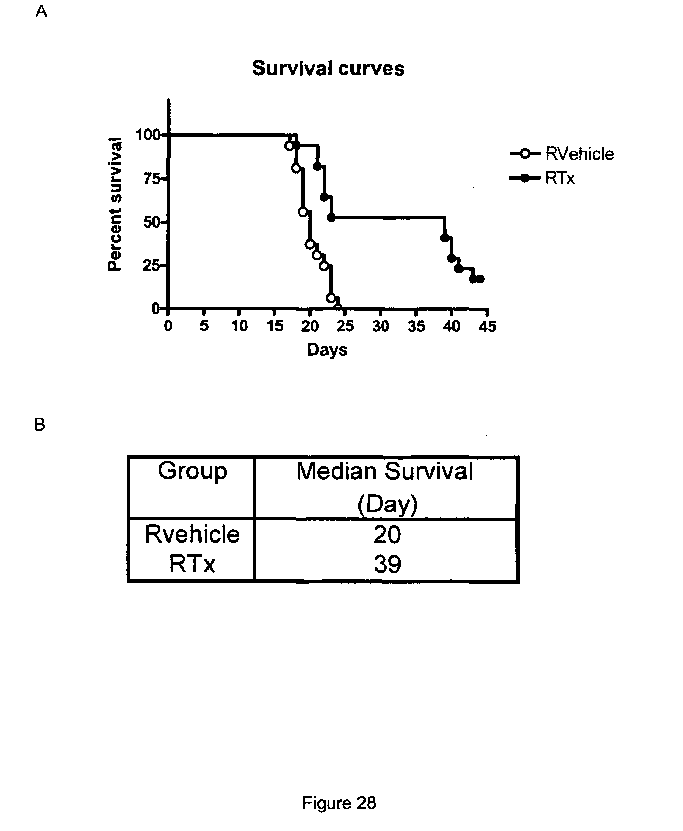

Daily injections, starting at day 15, of 8.2 mg/kg sTNALP-FcD10 to Akp2^{-/-} mice increased their survival compared to the mice that were injected vehicle (Figure 28). This increase was statistically significant (p<0.05).

### Body weight

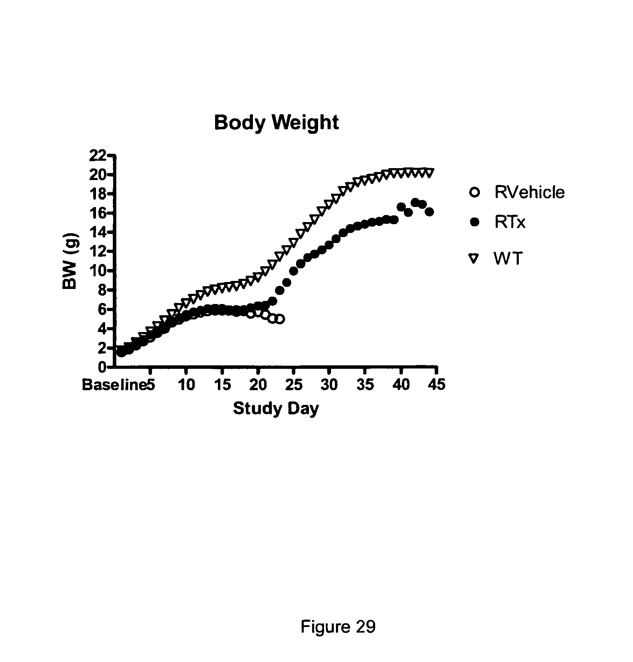

At the start of the study, no significant difference in body weight was noticed between groups (Figure 29). At the beginning of treatment (day 15), the body weight of Akp2^{-/-} mice was smaller than that of wild-type animals. While the body weight of animals injected vehicle continued to decrease, Akp2^{-/-} mice treated with sTNALP-FcD10 started to gain weight 4 to 5 days after initiation of treatment and kept gaining weight until the end of the study, without however reaching the values of the wild-type animals. This weight gain suggests improvement in the well being of the animals treated with sTNALP-FcD10.

### Bone mineralization

For each treatment, the radiographs of the feet were analyzed and distributed between normal and abnormal. Numbers and percentages (in parentheses) appear in Table 5. The radiographs were taken at necropsy.

Treatment of Akp2^{-/-} mice with 8.2 mg/kg sTNALP-FcD10 daily, starting at day 15 after birth improved mineralization as seen from the radiography of the feet taken at necropsy. The sTNALP-FcD10-treated group showed 41% normal animals compared to 12% in vehicle-injected group of Akp2^{-/-} mice. This difference almost reached statistical significance (p= 0.0645 in Chi square test).

**Table 5: Distribution of radiographs of feet**

| **Group** | **Abnormal** | **Normal** |
|---|---|---|
| **RVehicle (N=16)** | 14 (88) | 2 (12) |
| **RTx-1 (N=17)** | 10 (59) | 7(41) |
| **WT (N=30)** | 0(0) | 30 (100) |

### EXAMPLE 14

### Long-term efficacy of different dosage intervals of sTNALP-FcD10 on the rescue of Akp2^{-/-} mice

Mice were initiated on the treatment at day 12 and injected s.c. with vehicle (RV), 8.2 mg/Kg daily to days 46/47 (RTx-1) or injected with 8.2.mg/Kg daily for 7 days followed by 24.6 mg/Kg every 3 day (RTx-3) or followed by 57.4 mg/Kg every 7 days (RTx-7). The median survival was 19.5 days for the RV mice, 21.0 days for the RTx-7 mice, 30.5 days for the RTx-3 mice and 37.5 days for the RTx-1 mice. In all cases, survival was statistically increased when compared to that of the vehicle-treated group. There is a clear benefit of ERT in *Akp2*^{*-*/*-*} mice with well-established hypophosphatasia. Dosing less frequently than daily also appears to statistically increase survival.

### EXAMPLE 15

### A Maximum Tolerated Dose Intravenous Injection Toxicity Study in Juvenile Sprague-Dawley Rats

The objective of the study was to determine the maximum tolerated dose (MTD) and toxicity of the test article, sTNALP-FcD10, following repeated administration to juvenile Sprague-Dawley rats by intravenous injection. In Examples 15 to 18, the sALP-FcD10 used is that specifically described in Figure 3.

sTNALP-FcD10 was administered to juvenile Sprague-Dawley rats (aged at initiation between 22 and 24 days) once weekly for four weeks by intravenous injection as described in Table 6 below:

**Table 6: Study design**

| Group Numbers | Treatment | Dose Level (mg/kg/occasion) | Dose Concentration (mg/mL) | Number of Animals | |
|---|---|---|---|---|---|
| | | | | Male | Female |
| 1 | Dose 1 | 10 | 2.0 | 3 | 3 |
| 2 | Dose 2 | 30 | 6.0 | 3 | 3 |
| 3 | Dose 3 | 90 | 18.0 | 3 | 3 |
| 4 | Dose 4 | 180 | 36.0 | 3 | 3 |

Throughout the study, the animals were monitored for mortality, body weight, and clinical condition. Hematology, coagulation and clinical chemistry assessments were performed on all animals. Terminally, the rats were euthanized and subjected to necropsy. For each animal, samples of selected tissues were retained and were subjected to histological processing and microscopic examination.

There was no mortality in this study and there were no test article-related changes in coagulation parameters or organ weights. The body weights of the High Dose males, in particular, were about 10% below the Low Dose suggesting a treatment-related effect.

No clinical signs were observed in Groups 1 and 2 animals on the first dosing occasion. In Groups 3 and 4 animals, however, the animals appeared weak immediately following dosing and some Group 4 animals showed slight to moderate decrease in activity. Slight swelling of limbs, pinna and muzzle with skin discoloration (red or blue in appearance) at the extremities were also observed in the two groups. Other clinical signs observed in Group 4 animals included excessive scratching, piloerection and hyperpnea.

Clinical signs recorded for Groups 1 and 2 animals on the second dosing occasion (Day 8), were swelling of limbs, pinna and muzzle with skin discoloration (red or blue in appearance) at the extremities. Similar clinical signs of skin swelling were also recorded on the third and fourth dosing occasions (Days 15 and 22) for the same groups of animals. On the fourth dosing occasion (Day 22) slight hyperactivity was observed in Group 1 females whereas hypoactivity was observed in Group 2 males. For Group 3 and 4 animals, the clinical signs of reduced motor activity, piloerection, hyperpnea and swelling of limbs, pinna and muzzle with skin coloration became more evident as dosing progressed from the first dosing occasion to the fourth. It is considered that these clinical signs were treatment-related. On Days 16 to 19 and on Day 23, slight swelling and skin coloration of pinna (red in appearance) were observed in one animal (Group 1). Similar clinical signs were observed in another animal (Group 2) on Day 23.

The clinical signs were acute and the severity increased as dosing progressed but they were transient. All clinical signs appeared within 50 minutes after administration of test article, sTNALP-FcD10, with some animals recovering within, approximately, thirty minutes to 2 hours. For other animals, recovery was complete the next morning (the next scheduled observation time).

There was a treatment-related decrease in platelet counts (PLT) for males and females from all treatment groups, measured after the last dose, compared to background values. There was an increase in predominantly the percentage but also absolute reticulocytes that was noted generally in animals treated at the three highest dose levels.

Levels of alkaline phosphatase in serum were higher than could be quantified by the analytical instrument even following dilution. The results that were available for the Low Dose females were dramatically higher than the background range. This was expected as the test article is an active modified ALP.

Macroscopically, dark focus/area and/or depressed area of the glandular stomach were observed in 3 of 6 Group 3 animals (2 males/1 female) and 4 of 6 Group 4 animals (2 males/2 females).

Microscopically, minimal to mild erosion/ulcer of the glandular stomach, occasionally associated with submucosal edema was noted in 3 of 6 Group 3 animals (2 males/1 females) and 4 of 6 Group 4 animals (2 males/2 females), correlating gross findings.

In conclusion, intravenous injection of sTNALP-FcD10 to juvenile Sprague-Dawley rats once weekly for 4 weeks did not cause death at any of the dose levels tested but did cause adverse clinical signs, minor haematological changes and erosion/ulceration of the glandular stomach, occasionally associated with submucosal edema at dose levels of 90 and 180 mg/kg.

Changes related to administration of the test article at the two lowest dose levels tested (10 and 30 mg/kg) were limited to transient clinical signs apparent on the day of dosing only. The clinical signs were more severe at the 90 mg/kg dose level but they were also transient. The clinical signs noted in the animals treated with 180 mg/kg were so severe as to prevent this dose level from being used in future studies. Consequently the highest recommended dose level for subsequent longer term studies is 90 mg/kg.

### EXAMPLE 16

### An Intravenous Injection and Infusion Maximum Tolerated Dose Toxicity Study in Juvenile Cynomolgus Monkeys

The purpose of this study was to determine the maximum tolerated dose for sTNALP-FcD10, when administered once by intravenous injection or infusion to juvenile Cynomolgus monkeys. The test article dosing formulations were administered once in an incremental fashion, as indicated in Table 7 below.

**Table 7: Study design**

| | | | | | | | | | |
|---|---|---|---|---|---|---|---|---|---|
| * Only the Main Study animals were dosed on Day 46. | | | | | | | | | |

| Study Day | Treatment | Dose Level (mg/kg) | Dose Volume (mL/kg) | Dose Rate (mL/kg/hr) | Dose Conc. (mg/mL) | Number of Animals | | | |
|---|---|---|---|---|---|---|---|---|---|
| | | | | | | Main Study | | Toxicokinetic | |
| | | | | | | Males | Femal es | Males | Females |
| 1 | IV Injection | 5 | 4 | N/A | 1.25 | 2 | 2 | 1 | 1 |
| 8 | IV Injection | 15 | 4 | N/A | 3.75 | | | | |
| 15 | IV Infusion | 45 | 4 | 80 | 11.25 | | | | |
| 22 | IV Infusion | 90 | 4 | 40 | 22.5 | | | | |
| 29 | IV Infusion | 180 | 4 | 20 | 45 | | | | |
| 46 | IV Injection | 45 | 4 | N/A | 11.25 | | | | |

After the last treatment, the animals were released from the study. Parameters monitored during the study were mortality, clinical observations, body weights, appetence, toxicokinetics, hematology and clinical chemistry.

No mortality, adverse clinical signs or effect on body weights were observed during the study.

A marked dose proportional increase in alkaline phosphatase was observed in all animals throughout the study. Since the test article was a synthetic alkaline phosphatase, this increase was principally due to the presence of the drug in the bloodstream of the animals after each dosing.

Increases in alanine aminotransferase and aspartate aminotransferase were observed in three animals during the study but in the absence of a necropsy, the toxicological significance of this finding is uncertain.

The pharmacokinetic of sTNALP-FcD10 was well characterized following a single IV administration of 5, 15, 45, 90 and 180 mg/kg to monkeys. For the IV injections mean AUC∞ values ranged from 797 to 2950 mg•h/L and mean Cmax values ranged from 65 to 396 mg/L over the dose range studied. For the infusions, mean AUC∞ ranged from 9410 to 48400 mg•h/L and Cmax ranged from 1230 to 7720 mg/L over the dose range studied.

Mean t1/2 values of sTNALP-FcD10 appeared to decrease with increasing dose levels of sTNALP-FcD10. Although systemic clearance of sTNALP-FcD10 was relatively consistent across dose levels, the 90 mg/kg dose group appeared to be a pharmacokinetic outlier with a substantially lower clearance when compared to the other dose levels (approximately five fold). No obvious gender related trends were noted.

In summary, although some reversible blood chemistry changes were observed during the study, the intravenous injection/infusion of sTNALP-FcD10 at up to 180 mg/kg was well tolerated by the juvenile Cynomolgus monkeys. Therefore, under the conditions of this study, the Maximum Tolerated Dose was considered to be at least 180 mg/kg.

### EXAMPLE 17

### A 4-Week (Once Weekly) Intravenous Injection Toxicity Study of sTNALP-FcD10 in the Juvenile Albino Rat Followed by a 28-Day Recovery Period

The objective of this study was to investigate the potential toxicity of sTNALP-FcD10 given once weekly by intravenous injection to the juvenile rat for a minimum of 4 consecutive weeks (total of 4 doses) followed by 28 days of recovery. The animals were dosed on study days 1, 8, 15 and 22 and the recovery period began on study day 29. The study design is detailed in Table 8 below.

**Table 8: Study design**

| | | | | Main | Main Study | | Recovery Study | |
|---|---|---|---|---|---|---|---|---|
| Groups | Target Dose Level (mg/kg/dose) | Actual Dose Level (mg/kg/dose) | Target Concentration (mg/mL) | Actual Concentration (mg/mL) | Males | Females | Males | Females |
| 1-Vehicle Control | 0 | 0 | 0 | 0 | 10 | 10 | 5 | 5 |
| 2- Low Dose | 3 | 2.5 | 0.6 | 0.5 | 10 | 10 | 5 | 5 |
| 3- Mid Dose | 30 | 26 | 6 | 5.1 | 10 | 10 | 5 | 5 |
| 4- High Dose | 90 | 77 | 18 | 15.3 | 10 | 10 | 5 | 5 |

The following were evaluated: clinical signs (twice daily), body weight (once during acclimation period and weekly starting on Day 21 post *partum*), food consumption (weekly), ophthalmology (end of treatment and end of recovery period), hematology (at necropsy), serum chemistry (at necropsy), urinalysis (Day 29 and at the end of recovery period), biochemical markers of bone turnover: osteocalcin (bone formation marker) and C-telopeptide (bone resorption marker) (the morning prior to schedule necropsy), antibody assessment (Day -1 and at necropsy), test article blood concentration evaluation (Day 16 and Day 23), bone densitometry (by DXA *in vivo* Day -1, 28-main and recovery study animals and Day 14 and 56-recovey study animals and pQCT *ex vivo*), radiography and macroscopic observations at necropsy, organ weights and histopathology.

One male given 90 mg/kg/dose was found dead on study Day 25. As no consequential histological observations (pulmonary and thymic hemorrhages graded slight and minimal, respectively) were made for this rat, its cause of death was undetermined based on pathological investigations. On Day 23, this animal was bled for test article blood concentration evaluation and this procedure may have contributed to its death since there was no evidence of toxicity on Day 22. There were no sTNALP-FcD10-related mortality or effects on ophthalmology, urinalysis, bone formation marker (osteocalcin), organ weights, gross pathology, radiology or microscopy examination.

sTNALP-FcD10-related clinical signs observed at 3, 30 and/or 90 mg/kg/dose groups are considered to be acute infusion reaction. These included partly closed eyes, decreased muscle tone, lying on the side, hunched posture, cold to touch, uncoordinated movements, decreased activity, abnormal gait and/or blue, red and/or firm swollen hindpaws and/or forepaws during cage-side observations at 5, 15, 30 and/or 60 minutes post dose. These observations were transient and did not occur on nondosing days or during the recovery period.

Generally, a trend for slightly decreased body weight and body weight gain was noted for males in the 3, 30 and/or 90 mg/kg/dose groups during the recovery period. The effect on bone size on two bones of appendicular skeleton (femur and at tibia) correlated with decreased body weights. Decreases in food consumption were generally consistent with the decreased body weights.

### Body weights were comparable to controls for sTNALP-FcD10-treated females.

sTNALP-FcD10 administered at 90 mg/kg/dose was generally associated with slight decreases in absolute neutrophils, monocytes and/or eosinophils compared to the control group. Additionally, slight increases in lymphocytes, platelets and absolute reticulocytes were observed compared to the control group. At the end of the recovery period, these slight changes were still apparent in the animals treated with 90 mg/kg.

sTNALP-FcD10 was generally associated with statistically significant dose-related increases in alkaline phosphatase in all treated groups compared to controls. Considering the nature of the test article (alkaline phosphatase), the absence of any changes in other liver enzymes and absence of histopathological correlates, these increases are likely attributed to circulating levels of sTNALP-FcD10. Slight statistically significant increases in phosphorus were observed in males treated with sTNALP-FcD10 at 90 mg/kg/dose during Week 4, associated with a non-significant increases in serum total calcium. At the end of the recovery, these changes, including those statistically significant, returned to control values.

There were no organ weight, radiological, macroscopic or microscopic changes that were related to sTNALP-FcD10 in juvenile rats treated intravenously once weekly at up to 90 mg/kg/dose for 4 consecutive weeks. There were no delayed effects identified in a subset of these animals allowed a 28-day recovery after completion of the treatment.

Slightly lower mean CTx values were observed for treated females compared to controls (attaining statistical significance at 90 mg/kg/dose). These lower values were not consistent with the bone density analysis and also with the results obtained for males, therefore the incidental nature for these decreases cannot be excluded.

High variability in bone densitometry and bone geometry parameters noted between groups was attributed to the rapid growth phase. At the end of recovery, area and BMC (assessed by DXA and pQCT) were generally lower for treated males, suggesting smaller bones for these animals. The effect on bone size was noted on two bones of appendicular skeleton (femur and at tibia) by two different techniques, however no consistent effect was noted for axial skeleton (suggesting no effect on crown to rump length). Although area and BMC were decreased, the mean BMD values were generally comparable to controls, suggesting the effect on BMC and area was secondary to the effect on growth. Lower body weights and lower food consumption for treated males relative to controls are consistent with these data. However small group size at recovery, lack of consistency with respect to gender as well as the variability confounded these results, therefore an incidental nature for these decreases cannot be completely excluded.

In conclusion, once weekly intravenous injection to the juvenile rat for a minimum of 4 consecutive weeks followed by 28-day of recovery at doses of 3, 30 and 90 mg/kg/dose resulted in clinical signs associated with transient injection related effects including uncoordinated and reduced activity and paw swelling observed up to 60 minutes post-dose. Males treated at 90 mg/kg/dose showed slight decreases in body weight and food consumption which correlated with slightly smaller tibiae and femurs assessed by densitometry techniques. For females, slightly lower mean values were obtained for C-telopeptide levels compared to controls. Serum phosphorus levels were slightly, although significantly, increased in the 90 mg/kg/dose group. Elevated serum alkaline phosphatase levels were likely attributed to circulating levels of sTNALP-FcD10. sTNALP-FcD10 had no meaningful or consistent effects on bone densitometry and bone geometry for females during treatment and recovery period. For males no biologically significant effects were noted on bone densitometry or bone geometry during the treatment period. In general, slight decreases in bone densitometry (bone mineral content and/or area assessed by DXA and pQCT) and bone geometry parameters with a corresponding lower mean body weight were noted for males relative to controls at the end of the recovery period. All findings resolved after a 28-day treatment-free period with the exception of the effects on body weight and bone size for high dose males which persisted. There were no evidence of ectopic calcification at the end of treatment or the end of the recovery period. There were no radiological, macroscopic or microscopic findings as well as any organ weight changes associated with sTNALP-FcD10 treatment at any dose level. Because the injection reaction was transient and did not result in any effect on any parameters used to assess toxicity in the 3 and 30 mg/kg/dose groups, it was not considered to be adverse. In the 90 mg/kg/dose group, this reaction was more severe and accompanied by decreases in body weight gain, reduced food consumption, and potentially decrease in bone growth and therefore the effects in this group were considered to be adverse. Consequently, the no observable adverse effect level (NOAEL) was considered to be 30 mg/kg/dose in this study.

### EXAMPLE 18

### A 4-Week Intravenous Injection Toxicity Study in Juvenile Cynomolgus Monkeys Followed by a 28-Day Recovery Period

The purpose of this study was to determine the toxicity and toxicokinetics of sTNALP-FcD10 in juvenile Cynomolgus monkeys, when administered once weekly by slow bolus intravenous injection for 4 weeks and to assess reversibility of any changes following a 28-day recovery period.

The control and test article dosing formulations were administered to juvenile Cynomolgus monkeys by slow intravenous bolus injection once weekly for 4 weeks followed by a 28-day recovery period, as indicated in the Table 9 below:

**Table 9: Study design**

| **Group** | **Dose Level (mg/kg)** | **Dose Volume (mL/kg)** | **Dose Conc. (mg/mL)** | **Number of Animals** | | | |
|---|---|---|---|---|---|---|---|
| | | | | **Main Study** | | **Recovery** | |
| | | | | **Males** | **Females** | **Males** | **Females** |
| 1 Control * | 0 | 4 | 0 | 3 | 3 | 2 | 2 |
| 2 Low Dose | 5 | 4 | 1.25 | 3 | 3 | 2 | 2 |
| 3 Mid Dose | 15 | 4 | 3.75 | 3 | 3 | 2 | 2 |
| 4 High Dose | 45 | 4 | 11.25 | 3 | 3 | 2 | 2 |

| | | | | | | | |
|---|---|---|---|---|---|---|---|
| * The Group 1 animals received the vehicle/control article, 25 mM sodium phosphate pH 7.4, 150 mM NaCl. | | | | | | | |

After the last treatment (Day 22), the Main Study animals were euthanized on Day 29, while the remaining Recovery animals were observed for an additional 28 days, following which they were euthanized on Day 57. All Main and Recovery animals were subjected to a necropsy examination.

Evaluations conducted during the study or at its conclusion included mortality, clinical condition, body weight, appetence, body measurements, radiographic assessments of bone development, ophthalmology, electrocardiography, toxicokinetics, immunogenicity, hematology, coagulation, clinical chemistry, urinalysis, biomarkers of bone turnover, organ weights, ex-vivo bone mineral density analyses, and gross and histopathology.

No mortality or adverse treatment-related clinical observations were noted during the study.

Based on the body measurements recorded at the end of the treatment and recovery period, there were no noteworthy inter-group differences for cranial circumference, or humerus, forearm, tibia or pelvic limb lengths.

There were no body weight or food consumption changes related to treatment with the test article at any dose level. There were no ophthalmological or electrocardographic findings related to the test article at any dose level. There were no haematological, red cell morphological, coagulation or urinalysis changes related to treatment with the test article at any dose level. There were no toxicologically significant changes among clinical biochemistry parameters during the treatment or recovery periods. A slight to pronounced dose related increase in alkaline phosphatase was observed in all test article treated animals at most assessment occasions throughout the treatment period. Alkaline phosphatase levels were generally more comparable to control values by the end of the recovery period. Since the test article is a synthetic alkaline phosphatase, this increase was principally due to the presence of the drug in the bloodstream of the animals after each dose, and thus the increases were considered to be non-adverse.

At the end of the treatment and recovery periods, there were no noteworthy inter-group differences in absolute or relative organ weights, nor were there any test article-related macroscopic or microscopic findings. Histological changes noted were considered to be either incidental findings, common background findings in this species, or findings related to some aspect of experimental manipulation. Reproductive organs were generally immature but considered normal for this age monkey.

In conclusion, weekly intravenous injection of sTNALP-FcD10, to male and female Cynomolgus monkeys for 4 weeks, at dose levels of 0, 5, 15 and 45 mg/kg, and followed by a 4-week recovery period, was without evidence of toxicity at any dose level. Therefore the high dose level, 45 mg/kg, was considered to be the No Observed Adverse Effect Level (NOAEL) in this study.

### EXAMPLE 19

### Determination of Maximum Recommended Starting Dose for Human

The maximum recommended starting dose (MRSD) for human is calculated by establishing the No Observed Adverse Effect Level (NOAEL, see Guidance for Industry and Reviewers. December 2002). Various concentrations of the formulation described above have been tested on mice, rat and monkeys including 1 mg/kg, 5 mg/kg, and 8.2 mg/kg daily subcutaneously; 3 mg/kg, 5 mg/kg, 10 mg/kg, 30 mg/kg, 45 mg/kg, 90 mg/kg and 180 mg/kg. The NOAEL for the most sensitive species, namely for rat, was 30 mg/kg.

This dose was scaled up to a human equivalent dose (HED) using published conversion tables which provide a conversion factor from rat to human of 6. A NOAEL of 30 mg/kg for that species is equivalent to 5 mg/kg in human.

This value (5 mg/kg) was divided by a security factor of ten. The calculated MRSD is thus 0.5 mg/kg. For an average human weighting 60 kg, a weekly dose of 30 mg or daily dose of 4.28 mg daily could thus be injected to start clinical trials.

### REFERENCES

1. Ali, S.Y., Sajdera, S.W. & Anderson, H.C. Isolation and characterization of calcifying matrix vesicles from epiphyseal cartilage. Proc Natl Acad Sci U S A 67, 1513-20 (1970).
2. Anderson, HC, Hsu, H.H., Morris, D.C., Fedde, K.N. & Whyte, M.P. 1997 Matrix vesicles in osteomalacic hypophosphatasia bone contain apatite-like mineral crystals. Am J Pathol 151 1555-61.
3. Anderson HC, Garimella R & Tague SE 2005a The role of matrix vesicles in growth plate development and biomineralization. Frontiers in Bioscience 10 822-837.
4. Anderson HC, Harmey D, Camacho NP, Garimella R, Sipe JB, Tague S, Bi XH, Johnson K, Terkeltaub R & Millan JL 2005b Sustained osteomalacia of long bones despite major improvement in other hypophosphatasia-related mineral deficits in tissue nonspecific alkaline phosphatase/nucleoticle pyrophosphatase phosphodiesterase 1 double-deficient mice. American Journal of Pathology 166 1711-1720.
5. Anderson HC & Reynolds JJ 1973 Pyrophosphate stimulation of calcium uptake into cultured embryonic bones. Fine structure of matrix vesicles and their role in calcification. Developmental Biology 34 211-227.
6. Anderson HC, Sipe JB, Hessle L, Dhamyamraju R, Atti E, Camacho NP & Millan JL 2004 Impaired Calcification Around Matrix Vesicles of Growth Plate and Bone in Alkaline Phosphatase-Deficient Mice. American Journal of Pathology 164 841-847.
7. Bernard, G.W. 1978Ultrastructural localization of alkaline phosphatase in initial intramembranous osteogenesis. Clin Orthop, 218-25.
8. Di Mauro S, Manes T, Hessle L, Kozlenkov A, Pizauro JM, Hoylaerts MF & Millan JL 2002 Kinetic characterization of hypophosphatasia mutations with physiological substrates. Journal of Bone and Mineral Research 17 1383-1391.
9. Farley JR & Magnusson P 2005 Effects of Tunicamycin, Mannosamine, and Other Inhibitors of Glycoprotein Processing on Skeletal Alkaline Phosphatase in Human Osteoblast-Like Cells. Calcified Tissue International 76 63-74.
10. Fedde KN, Blair L, Silverstein J, Coburn SP, Ryan LM, Weinstein RS, Waymire K, Narisawa S, Millan JL, MacGregor GR & Whyte MP 1999 Alkaline phosphatase knock-out mice recapitulate the metabolic and skeletal defects of infantile hypophosphatasia. Journal of Bone and Mineral Research 14 2015-2026.
11. Greenberg, C.R. et al. 1993 A homoallelic Gly317-->Asp mutation in ALPL causes the perinatal (lethal) form of hypophosphatasia in Canadian mennonites. Genomics 17, 215-7.
12. Harmey D, Johnson KA, Zelken J, Camacho NP, Hoylaerts MF, Noda M, Terkeltaub R & Millan JL 2006 Elevated skeletal osteopontin levels contribute to the hypophosphatasia phenotype in Akp2(-/-) mice. Journal of Bone and Mineral Research 21 1377-1386.
13. Harmey D, Hessle L, Narisawa S, Johnson KA, Terkeltaub R & Millan JL 2004 Concerted Regulation of Inorganic Pyrophosphate and Osteopontin by Akp2, Enpp1, and Ank: An Integrated Model of the Pathogenesis of Mineralization Disorders. American Journal of Pathology 164 1199-1209.
14. Hawrylak K & Stinson RA 1988 The solubilization of tetrameric alkaline phosphatase from human liver and its conversion into various forms by phosphatidylinositol phospholipase C or proteolysis. Journal of Biological Chemistry 263 14368-14373.
15. Henthorn, P.S., Raducha, M., Fedde, K.N., Lafferty, M.A. & Whyte, M.P. 1992a Different missense mutations at the tissue-nonspecific alkaline phosphatase gene locus in autosomal recessively inherited forms of mild and severe hypophosphatasia. Proc Natl Acad Sci U S A 89, 9924-8.
16. Henthorn, P.S. & Whyte, M.P. 1992b Missense mutations of the tissue-nonspecific alkaline phosphatase gene in hypophosphatasia. Clin Chem 38, 2501-5.
17. Hessle L, Johnson KA, Anderson HC, Narisawa S, Sali A, Goding JW, Terkeltaub R & Millan JL 2002 Tissue-nonspecific alkaline phosphatase and plasma cell membrane glycoprotein-1 are central antagonistic regulators of bone mineralization. Proceedings of the National Academy of Sciences of the United States of America 99 9445-9449.
18. Ikezawa H 2002 Glycosylphosphatidylinositol (GPI)-Anchored Proteins. Biol Pharm. Bull. 25(4) 409-417.
19. Jansonius JN 1998 Structure, evolution and action of vitamin B6-dependent enzymes. Current Opinion in Structural Biology 8 759-769.
20. Johnson K, Moffa A, Chen Y, Pritzker K, Goding J & Terkeltaub R 1999 Matrix vesicle plasma cell membrane glycoprotein-1 regulates mineralization by murine osteoblastic MC3T3 cells. Journal of Bone and Mineral Research 14 883-892.
21. Mahmood I, Green MD, & Fisher JE 2003 Selection of the First-Time Dose in Humans: Comparison of Different Approaches Based on Interspecies Scaling of Clearance. J. Clin. Pharmacol., 43 (7), 692-7.
22. Meyer, J.L. 1984 Can biological calcification occur in the presence of pyrophosphate? Arch Biochem Biophys 231 1-8.
23. Millán, J.L. 2006 Mammalian Alkaline Phosphatases. From Biology to Applications in Medicine and Biotechnology, Wiley-VCH Verlag GmbH & Co., Weinheim, Germany 1-322.
24. Morris, D.C., Masuhara, K., Takaoka, K., Ono, K. & Anderson, H.C. 1992 Immunolocalization of alkaline phosphatase in osteoblasts and matrix vesicles of human fetal bone. Bone Miner 19 287-98.
25. Murshed M, Harmey D, Millan JL, McKee MD & Karsenty G 2005 Unique coexpression in osteoblasts of broadly expressed genes accounts for the spatial restriction of ECM mineralization to bone. Genes and Development 19 1093-1104.
26. Nasu M, Ito M, Ishida Y, Numa N, Komaru K, Nomura S and Oda K 2006 Aberrant interchain disulfide bridge of tissue-nonspecific alkalinephosphatase with an Arg433 . Cys substitution associated with severe hypophosphatasia FEBS Journal 273 5612-5624.
27. Narisawa S, Frohlander N & Millan JL 1997 Inactivation of two mouse alkaline phosphatase genes and establishment of a model of infantile hypophosphatasia. Developmental Dynamics 208 432-446.
28. Narisawa S, Wennberg C & Millan JL 2001 Abnormal vitamin B6 metabolism in alkaline phosphatase knock-out mice causes multiple abnormalities, but not the impaired bone mineralization. Journal of Pathology 193 125-133.
29. Nishioka T, Tomatsu S, Gutierrez MA, Miyamoto K, Trandafirescu GG, Lopez PLC, Grubb JH, Kanai R, Kobayashi H, Yamaguchi S, Gottesman GS, Cahill R, Noguchi A & Sly WS 2006 Enhancement of drug delivery to bone: Characterization of human tissue-non specific alkaline phosphatase tagged with an acidic oligopeptide. Molecular Genetics and Metabolism 88 244-255.
30. Nosjean O, Koyama I, Goseki M, Roux B & Komoda T 1997 Human tissue non-specific alkaline phosphatases: Sugar-moiety-induced enzymic and antigenic modulations and genetic aspects. Biochemical Journal 321 297-303.
31. Oda et al. 1999 J. Biochem 126: 694-699.
32. Rezende LA, Ciancaglini P, Pizauro JM & Leone FA 1998 Inorganic pyrophosphate-phosphohydrolytic activity associated with rat osseous plate alkaline phosphatase. Cellular and Molecular Biology 44 293-302.
33. Urlaub G, Kas E, Carothers AM & Chasin LA 1983 Deletion of the Diploid Dihydrofolate-Reductase Locus from Cultured Mammalian-Cells. Cell 33 405-412.
34. Waymire KG, Mahuren JD, Jaje JM, Guilarte TR, Coburn SP & MacGregor GR 1995 Mice Lacking Tissue Nonspecific Alkaline-Phosphatase Die from Seizures Due to Defective Metabolism of Vitamin-B-6. Nature Genetics 11 45-51.
35. Weiss, M.J. et al. 1988 A missense mutation in the human liver/bone/kidney alkaline phosphatase gene causing a lethal form of hypophosphatasia. Proc Natl Acad Sci U S A 85, 7666-9.
36. Weninger M, Stinson RA, Plenk H, Bock P & Pollak A 1989 Biochemical and Morphological Effects of Human Hepatic Alkaline-Phosphatase in A Neonate with Hypophosphatasia. Acta Paediatrica Scandinavica 154-160.
37. Whyte MP 1994 Hypophosphatasia and the Role of Alkaline-Phosphatase in Skeletal Mineralization. Endocrine Reviews 15 439-461.
38. Whyte M.P. 1995 Alkaline Phosphatase: Placental and Tissue-nonspecific Isoenzymes hydrolyze Phosphoethanolamine, Inorganic Pyrophosphate, and Pyridoxal 5'-phosphate J. Clin. Invest.. 95:1440-1445.
39. Whyte MP 2001 Hypophosphatasia. In The Metabolic and Molecular Bases of Disease, edn 8th, pp 5313-5329. Eds CL Scriver, AL Beaudet, WS Sly, D Valle & B Vogelstein. New York: McGraw-Hill Book Company.
40. Whyte MP 2002 Hypophosphatasia. Nature's window on alkaline phosphatase function in man. In Principle of Bone Biology, edn Second, pp 1229-1248. Eds JP Bilezikian, LG Raisz & GA Rodan. London: Academic Press.
41. Whyte MP, Kurtzberg J, McAlister WH, Mumm S, Podgornik MN, Coburn SP, Ryan LM, Miller CR, Gottesman GS, Smith AK, Douville J, Waters P, Armstrong RD & Martin PL 2003 Marrow cell transplantation for infantile hypophosphatasia. J Bone Miner Res 18 624-636.
42. Whyte MP, Mahuren JD, Vrabel LA & Coburn SP 1985 Markedly Increased Circulating Pyridoxal-5'-Phosphate Levels in Hypophosphatasia - Alkaline- Phosphatase Acts in Vitamin-B6 Metabolism. Journal of Clinical Investigation 76 752-756.
43. Whyte MP, McAlister WH, Patton LS, Magill HL, Fallon MD, Lorentz WB & Herrod HG 1984 Enzyme replacement therapy for infantile hypophosphatasia attempted by intravenous infusions of alkaline phosphatase-rich Paget plasma: results in three additional patients. J Pediatr 105 926-933.
44. Whyte MP, Valdes R, Ryan LM & McAlister WH 1982 Infantile hypophosphatasia: enzyme replacement therapy by intravenous infusion of alkaline phosphatase-rich plasma from patients with Paget bone disease. J Pediatr 101 379-386.
45. Zurutuza L, Muller F, Gibrat JF, Taillandier A, Simon-Bouy B, Serre JL & Mornet E 1999 Correlations of genotype and phenotype in hypophosphatasia. Human Molecular Genetics 8 1039-1046.
46. Beertsen W., Van den Bos T, Everts V, 1999, Root development in mice lacking functional tissue non-specific alkaline phosphatase gene: Inhibition of acellular cementum formation. J. Dent. Res. 78:1221-1229.

## Claims

1. A bone targeted alkaline phosphatase comprising a polypeptide having the structure:
Z-sALP-Y-spacer-X-Wₙ-V,
wherein sALP consists of amino acid residues 18-502 of SEQ ID NO: 8;
wherein V is absent or is an amino acid sequence of at least one amino acid;
X is absent or is an amino acid sequence of at least one amino acid;
Y is absent or is an amino acid sequence of at least one amino acid;
Z is absent or is an amino acid sequence of at least one amino acid;
Wₙ is a polyaspartate or a polyglutamate wherein n = 10 to 16; and the spacer comprises a fragment crystallizable region (Fc).

2. The alkaline phosphatase of claim 1, wherein the Fc comprises a CH2 domain, a CH3 domain and a hinge region, or wherein the Fc is a constant domain of an immunoglobulin selected from the group consisting of IgG-1, IgG-2, IgG-3, IgG-3 and IgG-4, preferably wherein the Fc is a constant domain of an immunoglobulin IgG-1.

3. The alkaline phosphatase of claim 2, wherein the Fc is a constant domain of human IgG-1.

4. The alkaline phosphatase of claim 3, wherein the Fc is as set forth in SEQ ID NO: 3.

5. The alkaline phosphatase of any one of claims 1 to 4, wherein Wₙ is a polyaspartate, preferably wherein n = 10.

6. The alkaline phosphatase of any one of claims 1 to 5, wherein Z is absent, and/or wherein V is absent.

7. The alkaline phosphatase of any one of claims 1 to 6, wherein
a) Y is two amino acid residues, preferably wherein Y is leucine-lysine; and/or
b) X is two amino acid residues, preferably wherein X is aspartate-isoleucine.

8. The alkaline phosphatase of claim 1, wherein the polypeptide is as set forth in SEQ ID NO: 4.

9. The alkaline phosphatase of any one of claims 1 to 8, comprising the polypeptide in a form comprising a dimer.

10. The alkaline phosphatase of any one of claims 1 to 9, wherein said alkaline phosphatase consists of said polypeptide.

11. The alkaline phosphatase of any one of claims 1 to 10, wherein the alkaline phosphatase is in a pharmaceutically acceptable carrier, preferably wherein the pharmaceutically acceptable carrier is a saline, more preferably wherein the alkaline phosphatase is in a lyophilized form.

12. The alkaline phosphatase of any of claims 1 to 11 for use as a medicament.

13. The alkaline phosphatase of claim 12 in a daily dosage of about 0.2 to about 20 mg/kg, or a weekly dosage of about 1.4 to about 140 mg/kg.

14. An isolated nucleic acid comprising or consisting of a sequence that encodes the polypeptide defined in any one of claims 1 to 8.

15. A recombinant expression vector comprising the nucleic acid of claim 14.

16. A recombinant adeno-associated virus vector comprising the nucleic acid of claim 14.

17. An isolated recombinant host cell transformed or transfected with the vector of claim 15 or 16.

18. A method of producing the alkaline phosphatase of any one of claims 1 to 8, comprising culturing the host cell of claim 17, under conditions suitable to effect expression of the alkaline phosphatase, and recovering the alkaline phosphatase from the culture medium.

19. The method of claim 18, wherein the host cell is a L cell, C127 cell, 3T3 cell, CHO cell, BHK cell, COS-7 cell or a Chinese Hamster Ovary (CHO) cell, preferably wherein the host cell is a Chinese Hamster Ovary (CHO) cell, more preferably wherein the host cell is a CHO-DG44 cell.

20. A kit comprising the alkaline phosphatase as defined in any one of claims 1 to 11, and instructions for use in a method of correcting or preventing a hypophosphatasia (HPP) phenotype.

21. An alkaline phosphatase as defined in any one of claims 1 to 11 for use in a method of correcting or preventing at least one hypophosphatasia (HPP) phenotype in a subject in need thereof.

22. The alkaline phosphatase of claim 21, wherein
a) the subject has at least one HPP phenotype, preferably wherein
i) the at least one HPP phenotype comprises HPP-related seizure, or
ii) the at least one HPP phenotype comprises premature loss of deciduous teeth, or
iii) the at least one HPP phenotype comprises incomplete bone mineralization, preferably wherein incomplete bone mineralization is incomplete femoral bone mineralization or incomplete tibial bone mineralization, or incomplete metatarsal bone mineralization, or incomplete ribs bone mineralization, or
iv) the at least one HPP phenotype comprises elevated blood and/or urine levels of inorganic pyrophosphate (PPi), or
v) the at least one HPP phenotype comprises elevated blood and/or urine levels of phosphoethanolamine (PEA), or
vi) the at least one HPP phenotype comprises elevated blood and/or urine levels of pyridoxal 5'-phosphate (PLP), or
vii) the at least one HPP phenotype comprises inadequate weight gain; or
viii) the at least one HPP phenotype comprises rickets, or
ix) the at least one HPP phenotype comprises bone pain, or
x) the at least one HPP phenotype comprises calcium pyrophosphate dihydrate crystal deposition, or
xi) the at least one HPP phenotype comprises aplasia, hypoplasia or dysplasia of dental cementum.

23. The alkaline phosphatase of claim 21 or 22, wherein the subject in need thereof has
a) infantile HPP; or
b) childhood HPP; or
c) perinatal HPP; or
d) adult HPP; or
e) odontohypophosphatasia HPP.

24. The alkaline phosphatase of any of claims 21 to 23, wherein the use comprises transfecting a cell in the subject with a nucleic acid encoding the alkaline phosphatase, in particular wherein the transfecting the cell is performed *in vitro* such that the alkaline phosphatase is expressed and secreted in an active form and administered to the subject with said cell.

25. The alkaline phosphatase of any one of claims 21 to 23, wherein the use comprises subcutaneous administration of the alkaline phosphatase to the subject.

26. The alkaline phosphatase of any one of claims 21 to 23, wherein the use comprises intravenous administration of the alkaline phosphatase to the subject.

## Patentansprüche

1. Eine auf Knochen gezielte Alkalische Phosphatase, umfassend ein Polypeptid mit der Struktur:
Z-sALP-Y-spacer-X-Wₙ-V,
wobei sALP aus den Aminosäureresten 18-502 der SEQ ID NO: 8 besteht;
wobei V fehlt oder eine Aminosäuresequenz von mindestens einer Aminosäure ist; X nicht vorhanden ist oder eine Aminosäuresequenz von mindestens einer Aminosäure ist;
Y nicht vorhanden ist oder eine Aminosäuresequenz von mindestens einer Aminosäure ist;
Z nicht vorhanden ist oder eine Aminosäuresequenz von mindestens einer Aminosäure ist;
Wₙ ein Polyaspartat oder ein Polyglutamat ist, wobei n = 10 bis 16; und
der Spacer eine Fragment-kristallisierbare Region (Fc) umfasst.

2. Die Alkalische Phosphatase nach Anspruch 1, wobei die Fc eine CH2-Domäne, eine CH3-Domäne und eine Scharnier-Region umfasst, oder wobei die Fc eine konstante Domäne von einem Immunglobulin ist, das aus der Gruppe ausgewählt ist, bestehend aus IgG-1, IgG-2, IgG-3, IgG-3 und IgG-4, vorzugsweise wobei die Fc eine konstante Domäne von einem Immunglobulin IgG-1 ist.

3. Die Alkalische Phosphatase nach Anspruch 2, wobei die Fc eine konstante Domäne von menschlichem IgG-1 ist.

4. Die Alkalische Phosphatase nach Anspruch 3, wobei die Fc wie in SEQ ID NO: 3 ausgeführt ist.

5. Die Alkalische Phosphatase nach einem der Ansprüche 1 bis 4, wobei Wₙ ein Polyaspartat ist, bevorzugt wobei n = 10.

6. Die Alkalische Phosphatase nach einem der Ansprüche 1 bis 5, wobei Z nicht vorhanden ist, und/oder wobei V nicht vorhanden ist.

7. Die Alkalische Phosphatase nach einem der Ansprüche 1 bis 6, wobei
a) Y zwei Aminosäurereste ist, vorzugsweise wobei Y Leucin-Lysin ist; und/oder
b) X zwei Aminosäurereste ist, vorzugsweise wobei X Aspartat-Isoleucin ist.

8. Die Alkalische Phosphatase nach Anspruch 1, wobei das Polypeptid wie in SEQ ID NO: 4 ausgeführt ist.

9. Die Alkalische Phosphatase nach einem der Ansprüche 1 bis 8, umfassend das Polypeptid in einer Form, die ein Dimer umfasst.

10. Die Alkalische Phosphatase nach einem der Ansprüche 1 bis 9, wobei die Alkalische Phosphatase aus dem Polypeptid besteht.

11. Die Alkalische Phosphatase nach einem der Ansprüche 1 bis 10, wobei die Alkalische Phosphatase in einem pharmazeutisch annehmbaren Träger ist, bevorzugt wobei der pharmazeutisch annehmbare Träger eine Kochsalzlösung ist, stärker bevorzugt wobei die alkalische Phosphatase in einer lyophilisierten Form vorliegt.

12. Die Alkalische Phosphatase nach einem der Ansprüche 1 bis 11 zur Verwendung als ein Medikament.

13. Die Alkalische Phosphatase nach Anspruch 12 in einer täglichen Dosis von etwa 0,2 bis etwa 20 mg/kg oder einer wöchentlichen Dosis von etwa 1,4 bis etwa 140 mg/kg.

14. Eine isolierte Nukleinsäure umfassend oder bestehend aus einer Sequenz, die für das in einem der Ansprüche 1 bis 8 definierte Polypeptid kodiert.

15. Ein rekombinanter Expressionsvektor umfassend die Nukleinsäure nach Anspruch 14.

16. Ein rekombinanter adeno-assoziierter Virusvektor umfassend die Nukleinsäure nach Anspruch 14.

17. Eine isolierte rekombinante Wirtszelle transformiert oder transfiziert mit dem Vektor nach Anspruch 15 oder 16.

18. Ein Verfahren zur Herstellung der Alkalischen Phosphatase nach einem der Ansprüche 1 bis 8, umfassend Kultivieren der Wirtszelle nach Anspruch 17, unter Bedingungen geeignet, um die Expression der Alkalischen Phosphatase zu bewirken, und Gewinnen der Alkalischen Phosphatase aus dem Kulturmedium.

19. Das Verfahren nach Anspruch 18, wobei die Wirtszelle eine L-Zelle, C127-Zelle, 3T3-Zelle, CHO-Zelle, BHK-Zelle, COS-7-Zelle oder eine Chinesischer-Hamster-Ovarien- (CHO) -Zelle ist, bevorzugt wobei die Wirtszelle eine Chinesischer-Hamster-Ovarienzelle (CHO) ist, stärker bevorzugt, wobei die Wirtszelle eine CHO-DG44-Zelle ist.

20. Ein Kit umfassend die Alkalische Phosphatase wie definiert in einem der Ansprüche 1 bis 11, und Anweisungen zur Verwendung in einem Verfahren zur Korrektur oder Prävention eines Hypophosphatasie- (HPP) -Phänotyps.

21. Eine Alkalische Phosphatase wie definiert in einem der Ansprüche 1 bis 11 zur Verwendung in einem Verfahren zur Korrektur oder Prävention von mindestens einem Hypophosphatasie- (HPP) -Phänotyp in einem Individuum, welches dessen bedarf.

22. Die Alkalische Phosphase nach Anspruch 21, wobei
a) das Individuum mindestens einen HPP-Phänotyp aufweist, vorzugsweise wobei
i) der mindestens eine HPP-Phänotyp HPP-verwandten Schlaganfall umfasst, oder
ii) der mindestens eine HPP-Phänotyp vorzeitigen Verlust von Milchzähnen umfasst, oder
iii) der mindestens eine HPP-Phänotyp unvollständige Knochenmineralisation umfasst, wobei die unvollständige Knochenmineralisation vorzugsweise die unvollständige femurale Knochenmineralisation oder die unvollständige tibiale Knochenmineralisation oder die unvollständige metatarsale Knochenmineralisation oder die unvollständige Rippen-Knochenmineralisation ist, oder
iv) der mindestens eine HPP-Phänotyp erhöhte Blut- und/oder Urinspiegel von anorganischem Pyrophosphat (PPi) umfasst, oder
v) der mindestens eine HPP-Phänotyp erhöhte Blut- und/oder Urinspiegel von Phosphoethanolamin (PEA) umfasst, oder
vi) der mindestens eine HPP-Phänotyp erhöhte Blut- und/oder Urinspiegel von Pyridoxal-5'-Phosphat (PLP) umfasst, oder
vii) der mindestens eine HPP-Phänotyp unzureichende Gewichtszunahme umfasst; oder
viii) der mindestens eine HPP-Phänotyp Rachitis umfasst, oder
ix) der mindestens eine HPP-Phänotyp Knochenschmerz umfasst, oder
x) der mindestens eine HPP-Phänotyp Calciumpyrophosphatdihydrat-Kristallablagerung umfasst, oder
xi) der mindestens eine HPP-Phänotyp Aplasie, Hypoplasie oder Dysplasie von Dentalzement umfasst.

23. Die Alkalische Phosphatase nach Anspruch 21 oder 22, wobei das Individuum, welches dessen bedarf
a) frühkindliche HPP; oder
b) kindliche HPP; oder
c) perinatale HPP; oder
d) adulte HPP; oder
e) Odontohypophosphatasie-HPP
aufweist.

24. Die Alkalische Phosphatase nach einem der Ansprüche 21 bis 23, wobei die Verwendung die Transfektion einer Zelle in dem Individuum mit einer Nukleinsäure kodierend für die Alkalische Phosphatase umfasst, insbesondere wobei die Transfektion der Zelle *in vitro* durchgeführt wird, derart, dass die Alkalische Phosphatase in einer aktiven Form exprimiert und sekretiert wird und dem Individuum mit dieser Zelle verabreicht wird.

25. Die Alkalische Phosphatase nach einem der Ansprüche 21 bis 23, wobei die Verwendung die subkutane Gabe der Alkalischen Phosphatase an das Individuum umfasst.

26. Die Alkalische Phosphatase nach einem der Ansprüche 21 bis 23, wobei die Verwendung die intravenöse Gabe der Alkalischen Phosphatase an das Individuum umfasst.

## Revendications

1. Phosphatase alcaline ciblant les os, comprenant un polypeptide ayant la structure:
Z-sALP-Y-espaceur-X-Wₙ-V,
dans laquelle sALP est constitué des résidus d'acide aminé 18 à 502 de SEQ ID NO : 8 ;
dans laquelle V est absent ou est une séquence d'acides aminés contenant au moins un acide aminé ;
X est absent ou est une séquence d'acides aminés contenant au moins un acide aminé ;
Y est absent ou est une séquence d'acides aminés contenant au moins un acide aminé ;
Z est absent ou est une séquence d'acides aminés contenant au moins un acide aminé ;
Wₙ est un polyaspartate ou un polyglutamate dans lequel n = 10 à 16 ; et l'espaceur comprend une région de fragment cristallisable (Fc).

2. Phosphatase alcaline selon la revendication 1, dans laquelle le Fc comprend un domaine CH2, un domaine CH3 et une région charnière, ou dans lequel le Fc est un domaine constant d'une immunoglobuline choisie dans le groupe constitué par IgG-1, IgG-2, IgG-3, IgG-3 et IgG-4, de préférence dans lequel le Fc est un domaine constant d'une immunoglobuline IgG-1.

3. Phosphatase alcaline selon la revendication 2, dans laquelle le Fc est un domaine constant d'IgG-1 humaine.

4. Phosphatase alcaline selon la revendication 3, dans laquelle le Fc est tel que représenté par SEQ ID NO : 3.

5. Phosphatase alcaline selon l'une quelconque des revendications 1 à 4, dans laquelle Wₙ est un polyaspartate, de préférence dans lequel n = 10.

6. Phosphatase alcaline selon l'une quelconque des revendications 1 à 5, dans laquelle Z est absent et/ou dans laquelle V est absent.

7. Phosphatase alcaline selon l'une quelconque des revendications 1 à 6, dans laquelle
a) Y est deux résidus d'acide aminé, Y étant de préférence une leucine-lysine ; et/ou
b) X est deux résidus d'acide aminé, X étant de préférence un aspartate-isoleucine.

8. Phosphatase alcaline selon la revendication 1, dans laquelle le polypeptide est tel que représenté par SEQ ID NO : 4.

9. Phosphatase alcaline selon l'une quelconque des revendications 1 à 8, comprenant le polypeptide sous une forme comprenant un dimère.

10. Phosphatase alcaline selon l'une quelconque des revendications 1 à 9, dans laquelle ladite phosphatase alcaline est constituée dudit polypeptide.

11. Phosphatase alcaline selon l'une quelconque des revendications 1 à 10, dans laquelle la phosphatase alcaline est dans un support pharmaceutiquement acceptable, de préférence dans laquelle le support pharmaceutiquement acceptable est une solution physiologique salée, et de manière davantage préférée dans laquelle la phosphatase alcaline est sous une forme lyophilisée.

12. Phosphatase alcaline selon l'une quelconque des revendications 1 à 11, pour un usage en tant que médicament.

13. Phosphatase alcaline selon la revendication 12, en une dose quotidienne d'environ 0,2 mg/kg à environ 20 mg/kg, ou une dose hebdomadaire d'environ 1,4 mg/kg à environ 140 mg/kg.

14. Acide nucléique isolé comprenant ou consistant en une séquence qui code le polypeptide défini dans l'une quelconque des revendications 1 à 8.

15. Vecteur recombiné d'expression, comprenant l'acide nucléique selon la revendication 14.

16. Vecteur recombiné de virus adéno-associé, comprenant l'acide nucléique selon la revendication 14.

17. Cellule hôte recombinée isolée, transformée ou transfectée avec le vecteur selon la revendication 15 ou 16.

18. Procédé de production de la phosphatase alcaline selon l'une quelconque des revendications 1 à 8, comprenant la culture de la cellule hôte selon la revendication 17 dans des conditions appropriées pour effectuer l'expression de la phosphatase alcaline, et la récupération de la phosphatase alcaline dans le milieu de culture.

19. Procédé selon la revendication 18, dans lequel la cellule hôte est une cellule L, une cellule C127, une cellule 3T3, une cellule CHO, une cellule BHK, une cellule COS-7 ou une cellule d'ovaire de hamster chinois (CHO), de préférence dans lequel la cellule hôte est une cellule d'ovaire de hamster chinois (CHO), et de manière davantage préférée dans lequel la cellule hôte est une cellule CHO-DG44.

20. Trousse comprenant la phosphatase alcaline telle que définie dans l'une quelconque des revendications 1 à 11, et des instructions pour l'utiliser dans un procédé de correction ou de prévention d'un phénotype d'hypophosphatasie (HPP).

21. Phosphatase alcaline selon l'une quelconque des revendications 1 à 11, pour un usage dans un procédé de correction ou de prévention d'au moins un phénotype d'hypophosphatasie (HPP) chez un sujet en ayant besoin.

22. Phosphatase alcaline selon la revendication 21, dans laquelle
a) le sujet présente au moins un phénotype HPP, de préférence dans lequel
i) l'au moins un phénotype HPP comprend une attaque associée à HPP, ou
ii) l'au moins un phénotype HPP comprend la perte prématurée d'une dent temporaire, ou
iii) l'au moins un phénotype HPP comprend la minéralisation incomplète des os, de préférence dans lequel la minéralisation incomplète des os est une minéralisation incomplète de l'os fémoral ou une minéralisation incomplète de l'os tibial, ou une minéralisation incomplète de l'os métatarsien, ou une minéralisation incomplète des os des côtes, ou
iv) l'au moins un phénotype HPP comprend des taux élevés de pyrophosphate inorganique (PPi) dans le sang et/ou les urines, ou
v) l'au moins un phénotype HPP comprend des taux élevés de phosphoéthanolamine (PEA) dans le sang et/ou les urines, ou
vi) l'au moins un phénotype HPP comprend des taux élevés de pyridoxal-5'-phosphate (PLP) dans le sang et/ou les urines, ou
vii) l'au moins un phénotype HPP comprend un gain de poids inadéquate ; ou
viii) l'au moins un phénotype HPP comprend le rachitisme, ou
ix) l'au moins un phénotype HPP comprend une douleur osseuse, ou
x) l'au moins un phénotype HPP comprend un dépôt de cristaux de pyrophosphate de calcium dihydraté, ou
xi) l'au moins un phénotype HPP comprend une aplasie, une hypoplasie ou une dysplasie du ciment dentaire.

23. Phosphatase alcaline selon la revendication 21 ou 22, dans laquelle le sujet en ayant besoin présente
a) une HPP infantile ; ou
b) une HPP juvénile ; ou
c) une HPP périnatale ; ou
d) une HPP de l'adulte ; ou
e) une HPP d'odontohypophosphatasie.

24. Phosphatase alcaline selon l'une quelconque des revendications 21 à 23, dans laquelle l'utilisation comprend la transfection d'une cellule du sujet avec l'acide nucléique codant la phosphatase alcaline, en particulier dans laquelle la transfection de la cellule est réalisée *in vitro* de manière à ce que la phosphatase alcaline soit exprimée et sécrétée sous une forme active et administrée au sujet avec ladite cellule.

25. Phosphatase alcaline selon l'une quelconque des revendications 21 à 23, dans laquelle l'utilisation comprend l'administration sous-cutanée de la phosphatase alcaline au sujet.

26. Phosphatase alcaline selon l'une quelconque des revendications 21 à 23, dans laquelle l'utilisation comprend l'administration intraveineuse de la phosphatase alcaline au sujet.


### SEQUENCE LISTING

<110> ENOBIA PHARMA INC.
   Crine, Philippe
   Boileau, Guy
   Loisel Thomas P.
   Lemire, Isabelle
   Leonard, Pierre
   Heft, Robert
   Landy, Hal
<120> BONE TARGETED ALKALINE PHOSPHATASE, KITS AND METHODS OF USE THEREOF
<130> 765/12987.80
<150> US 60/917,589
   <151> 2007-05-11
<160> 19
<170> Patent In version 3.3
<210> 1
   <211> 743
   <212> PRT
   <213> Artificial
<220>
   <223> hTNALP-Fc-d10 with peptide signal
<400> 1
<210> 2
   <211> 502
   <212> PRT
   <213> Artificial
<220>
   <223> hTNALP 1-502
<400> 2
   Met Ile Ser Pro Phe Leu Val Leu Ala Ile Gly Thr Cys Leu Thr Asn
   1 5 10 15
   Ser Leu Val Pro Glu Lys Glu Lys Asp Pro Lys Tyr Trp Arg Asp Gln
   20 25 30
   Ala Gln Glu Thr Leu Lys Tyr Ala Leu Glu Leu Gln Lys Leu Asn Thr
   35 40 45
   Asn Val Ala Lys Asn Val Ile Met Phe Leu Gly Asp Gly Met Gly Val
   50 55 60
   Ser Thr Val Thr Ala Ala Arg Ile Leu Lys Gly Gln Leu His His Asn
   65 70 75 80
   Pro Gly Glu Glu Thr Arg Leu Glu Met Asp Lys Phe Pro Phe Val Ala
   85 90 95
   Leu Ser Lys Thr Tyr Asn Thr Asn Ala Gln Val Pro Asp Ser Ala Gly
   100 105 110
   Thr Ala Thr Ala Tyr Leu Cys Gly Val Lys Ala Asn Glu Gly Thr Val
   115 120 125
   Gly Val Ser Ala Ala Thr Glu Arg Ser Arg Cys Asn Thr Thr Gln Gly
   130 135 140
   Asn Glu Val Thr Ser Ile Leu Arg Trp Ala Lys Asp Ala Gly Lys Ser
   145 150 155 160
   Val Gly Ile Val Thr Thr Thr Arg Val Asn His Ala Thr Pro Ser Ala
   165 170 175
   Ala Tyr Ala His Ser Ala Asp Arg Asp Trp Tyr Ser Asp Asn Glu Met
   180 185 190
   Pro Pro Glu Ala Leu Ser Gln Gly Cys Lys Asp Ile Ala Tyr Gln Leu
   195 200 205
   Met His Asn Ile Arg Asp Ile Asp Val Ile Met Gly Gly Gly Arg Lys
   210 215 220
   Tyr Met Tyr Pro Lys Asn Lys Thr Asp Val Glu Tyr Glu Ser Asp Glu
   225 230 235 240
   Lys Ala Arg Gly Thr Arg Leu Asp Gly Leu Asp Leu Val Asp Thr Trp
   245 250 255
   Lys Ser Phe Lys Pro Arg Tyr Lys His Ser His Phe Ile Trp Asn Arg
   260 265 270
   Thr Glu Leu Leu Thr Leu Asp Pro His Asn Val Asp Tyr Leu Leu Gly
   275 280 285
   Leu Phe Glu Pro Gly Asp Met Gln Tyr Glu Leu Asn Arg Asn Asn Val
   290 295 300
   Thr Asp Pro Ser Leu Ser Glu Met Val Val Val Ala Ile Gln Ile Leu
   305 310 315 320
   Arg Lys Asn Pro Lys Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Arg Ala Ile Gly Gln Ala Gly Ser Leu Thr Ser Ser
   355 360 365
   Glu Asp Thr Leu Thr Val Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Leu Ser Asp Thr Asp Lys Lys Pro Phe Thr Ala Ile Leu Tyr Gly
   405 410 415
   Asn Gly Pro Gly Tyr Lys Val Val Gly Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Ser Lys
   450 455 460
   Gly Pro Met Ala His Leu Leu His Gly Val His Glu Gln Asn Tyr Val
   465 470 475 480
   Pro His Val Met Ala Tyr Ala Ala Cys Ile Gly Ala Asn Leu Gly His
   485 490 495
   Cys Ala Pro Ala Ser Ser
   500
<210> 3
   <211> 227
   <212> PRT
   <213> Artificial
<220>
   <223> IgG-1 Fc fragment
<400> 3
   Asp Lys Thr His Thr Cys Pro Pro Cys Pro Ala Pro Glu Leu Leu Gly
   1 5 10 15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Asn Ser Thr Tyr
   65 70 75 80
   Arg Val Val Ser Val Leu Thr Val Leu His Gln Asp Trp Leu Asn Gly
   85 90 95
   Lys Glu Tyr Lys Cys Lys Val Ser Asn Lys Ala Leu Pro Ala Pro Ile
   100 105 110
   Glu Lys Thr Ile Ser Lys Ala Lys Gly Gln Pro Arg Glu Pro Gln Val
   115 120 125
   Tyr Thr Leu Pro Pro Ser Arg Glu Glu Met Thr Lys Asn Gln Val Ser
   130 135 140
   Leu Thr Cys Leu Val Lys Gly Phe Tyr Pro Ser Asp Ile Ala Val Glu
   145 150 155 160
   Trp Glu Ser Asn Gly Gln Pro Glu Asn Asn Tyr Lys Thr Thr Pro Pro
   165 170 175
   Val Leu Asp Ser Asp Gly Ser Phe Phe Leu Tyr Ser Lys Leu Thr Val
   180 185 190
   Asp Lys Ser Arg Trp Gln Gln Gly Asn Val Phe Ser Cys Ser Val Met
   195 200 205
   His Glu Ala Leu His Asn His Tyr Thr Gln Lys Ser Leu Ser Leu Ser
   210 215 220
   Pro Gly Lys
   225
<210> 4
   <211> 726
   <212> PRT
   <213> Artificial
<220>
   <223> hsTNALP-Fc-d10 without signal peptide
<400> 4
   Leu Val Pro Glu Lys Glu Lys Asp Pro Lys Tyr Trp Arg Asp Gln Ala
   1 5 10 15
   Gln Glu Thr Leu Lys Tyr Ala Leu Glu Leu Gln Lys Leu Asn Thr Asn
   20 25 30
   Val Ala Lys Asn Val Ile Met Phe Leu Gly Asp Gly Met Gly Val Ser
   35 40 45
   Thr Val Thr Ala Ala Arg Ile Leu Lys Gly Gln Leu His His Asn Pro
   50 55 60
   Gly Glu Glu Thr Arg Leu Glu Met Asp Lys Phe Pro Phe Val Ala Leu
   65 70 75 80
   Ser Lys Thr Tyr Asn Thr Asn Ala Gln Val Pro Asp Ser Ala Gly Thr
   85 90 95
   Ala Thr Ala Tyr Leu Cys Gly Val Lys Ala Asn Glu Gly Thr Val Gly
   100 105 110
   Val Ser Ala Ala Thr Glu Arg Ser Arg Cys Asn Thr Thr Gln Gly Asn
   115 120 125
   Glu Val Thr Ser Ile Leu Arg Trp Ala Lys Asp Ala Gly Lys Ser Val
   130 135 140
   Gly Ile Val Thr Thr Thr Arg Val Asn His Ala Thr Pro Ser Ala Ala
   145 150 155 160
   Tyr Ala His Ser Ala Asp Arg Asp Trp Tyr Ser Asp Asn Glu Met Pro
   165 170 175
   Pro Glu Ala Leu Ser Gln Gly Cys Lys Asp Ile Ala Tyr Gln Leu Met
   180 185 190
   His Asn Ile Arg Asp Ile Asp Val Ile Met Gly Gly Gly Arg Lys Tyr
   195 200 205
   Met Tyr Pro Lys Asn Lys Thr Asp Val Glu Tyr Glu Ser Asp Glu Lys
   210 215 220
   Ala Arg Gly Thr Arg Leu Asp Gly Leu Asp Leu Val Asp Thr Trp Lys
   225 230 235 240
   Ser Phe Lys Pro Arg Tyr Lys His Ser His Phe Ile Trp Asn Arg Thr
   245 250 255
   Glu Leu Leu Thr Leu Asp Pro His Asn Val Asp Tyr Leu Leu Gly Leu
   260 265 270
   Phe Glu Pro Gly Asp Met Gln Tyr Glu Leu Asn Arg Asn Asn Val Thr
   275 280 285
   Asp Pro Ser Leu Ser Glu Met Val Val Val Ala Ile Gln Ile Leu Arg
   290 295 300
   Lys Asn Pro Lys Gly Phe Phe Leu Leu Val Glu Gly Gly Arg Ile Asp
   305                 310                 315                 320
   His Gly His His Glu Gly Lys Ala Lys Gln Ala Leu His Glu Ala Val
                   325                 330                 335
   Glu Met Asp Arg Ala Ile Gly Gln Ala Gly Ser Leu Thr Ser Ser Glu
               340                 345                 350
   Asp Thr Leu Thr Val Val Thr Ala Asp His Ser His Val Phe Thr Phe
           355                 360                 365
   Gly Gly Tyr Thr Pro Arg Gly Asn Ser Ile Phe Gly Leu Ala Pro Met
       370                 375                 380
   Leu Ser Asp Thr Asp Lys Lys Pro Phe Thr Ala Ile Leu Tyr Gly Asn
   385                 390                 395                 400
   Gly Pro Gly Tyr Lys Val Val Gly Gly Glu Arg Glu Asn Val Ser Met
                   405                 410                 415
   Val Asp Tyr Ala His Asn Asn Tyr Gln Ala Gln Ser Ala Val Pro Leu
               420                 425                 430
   Arg His Glu Thr His Gly Gly Glu Asp Val Ala Val Phe Ser Lys Gly
           435                 440                 445
   Pro Met Ala His Leu Leu His Gly Val His Glu Gln Asn Tyr Val Pro
       450                 455                 460
   His Val Met Ala Tyr Ala Ala Cys Ile Gly Ala Asn Leu Gly His Cys
   465                 470                 475                 480
   Ala Pro Ala Ser Ser Leu Lys Asp Lys Thr His Thr Cys Pro Pro Cys
                   485                 490                 495
   Pro Ala Pro Glu Leu Leu Gly Gly Pro Ser Val Phe Leu Phe Pro Pro
               500                 505                 510
   Lys Pro Lys Asp Thr Leu Met Ile Ser Arg Thr Pro Glu Val Thr Cys
           515                 520                 525
   Val Val Val Asp Val Ser His Glu Asp Pro Glu Val Lys Phe Asn Trp
       530                 535                 540
   Tyr Val Asp Gly Val Glu Val His Asn Ala Lys Thr Lys Pro Arg Glu
   545                 550                 555                 560
   Glu Gln Tyr Asn Ser Thr Tyr Arg Val Val Ser Val Leu Thr Val Leu
                   565                 570                 575
   His Gln Asp Trp Leu Asn Gly Lys Glu Tyr Lys Cys Lys Val Ser Asn
               580                 585                 590
   Lys Ala Leu Pro Ala Pro Ile Glu Lys Thr Ile Ser Lys Ala Lys Gly
           595                 600                 605
   Gln Pro Arg Glu Pro Gln Val Tyr Thr Leu Pro Pro Ser Arg Glu Glu
       610                 615                 620
   Met Thr Lys Asn Gln Val Ser Leu Thr Cys Leu Val Lys Gly Phe Tyr
   625                 630                 635                 640
   Pro Ser Asp Ile Ala Val Glu Trp Glu Ser Asn Gly Gln Pro Glu Asn
                   645                 650                 655
   Asn Tyr Lys Thr Thr Pro Pro Val Leu Asp Ser Asp Gly Ser Phe Phe
               660                 665                 670
   Leu Tyr Ser Lys Leu Thr Val Asp Lys Ser Arg Trp Gln Gln Gly Asn
           675                 680                 685
   Val Phe Ser Cys Ser Val Met His Glu Ala Leu His Asn His Tyr Thr
       690                 695                 700
   Gln Lys Ser Leu Ser Leu Ser Pro Gly Lys Asp Ile Asp Asp Asp Asp
   705                 710                 715                 720
   Asp Asp Asp Asp Asp Asp
                   725
<210> 5
   <211> 485
   <212> PRT
   <213> Artificial
<220>
   <223> hsTNALP (18-502)
<400> 5
   Leu Val Pro Glu Lys Glu Lys Asp Pro Lys Tyr Trp Arg Asp Gln Ala
   1               5                   10                  15
   Gln Glu Thr Leu Lys Tyr Ala Leu Glu Leu Gln Lys Leu Asn Thr Asn
               20                  25                  30
   Val Ala Lys Asn Val Ile Met Phe Leu Gly Asp Gly Met Gly Val Ser
           35                  40                  45
   Thr Val Thr Ala Ala Arg Ile Leu Lys Gly Gln Leu His His Asn Pro
       50                  55                  60
   Gly Glu Glu Thr Arg Leu Glu Met Asp Lys Phe Pro Phe Val Ala Leu
   65                  70                  75                  80
   Ser Lys Thr Tyr Asn Thr Asn Ala Gln Val Pro Asp Ser Ala Gly Thr
                   85                  90                  95
   Ala Thr Ala Tyr Leu Cys Gly Val Lys Ala Asn Glu Gly Thr Val Gly
               100                 105                 110
   Val Ser Ala Ala Thr Glu Arg Ser Arg Cys Asn Thr Thr Gln Gly Asn
           115                 120                 125
   Glu Val Thr Ser Ile Leu Arg Trp Ala Lys Asp Ala Gly Lys Ser Val
       130                 135                 140
   Gly Ile Val Thr Thr Thr Arg Val Asn His Ala Thr Pro Ser Ala Ala
   145                 150                 155                 160
   Tyr Ala His Ser Ala Asp Arg Asp Trp Tyr Ser Asp Asn Glu Met Pro
                   165                 170                 175
   Pro Glu Ala Leu Ser Gln Gly Cys Lys Asp Ile Ala Tyr Gln Leu Met
               180                 185                 190
   His Asn Ile Arg Asp Ile Asp Val Ile Met Gly Gly Gly Arg Lys Tyr
           195                 200                 205
   Met Tyr Pro Lys Asn Lys Thr Asp Val Glu Tyr Glu Ser Asp Glu Lys
       210                 215                 220
   Ala Arg Gly Thr Arg Leu Asp Gly Leu Asp Leu Val Asp Thr Trp Lys
   225                 230                 235                 240
   Ser Phe Lys Pro Arg Tyr Lys His Ser His Phe Ile Trp Asn Arg Thr
                   245                 250                 255
   Glu Leu Leu Thr Leu Asp Pro His Asn Val Asp Tyr Leu Leu Gly Leu
               260                 265                 270
   Phe Glu Pro Gly Asp Met Gln Tyr Glu Leu Asn Arg Asn Asn Val Thr
           275                 280                 285
   Asp Pro Ser Leu Ser Glu Met Val Val Val Ala Ile Gln Ile Leu Arg
       290                 295                 300
   Lys Asn Pro Lys Gly Phe Phe Leu Leu Val Glu Gly Gly Arg Ile Asp
   305                 310                 315                 320
   His Gly His His Glu Gly Lys Ala Lys Gln Ala Leu His Glu Ala Val
                   325                 330                 335
   Glu Met Asp Arg Ala Ile Gly Gln Ala Gly Ser Leu Thr Ser Ser Glu
               340                 345                 350
   Asp Thr Leu Thr Val Val Thr Ala Asp His Ser His Val Phe Thr Phe
           355                 360                 365
   Gly Gly Tyr Thr Pro Arg Gly Asn Ser Ile Phe Gly Leu Ala Pro Met
       370                 375                 380
   Leu Ser Asp Thr Asp Lys Lys Pro Phe Thr Ala Ile Leu Tyr Gly Asn
   385                 390                 395                 400
   Gly Pro Gly Tyr Lys Val Val Gly Gly Glu Arg Glu Asn Val Ser Met
                   405                 410                 415
   Val Asp Tyr Ala His Asn Asn Tyr Gln Ala Gln Ser Ala Val Pro Leu
               420                 425                 430
   Arg His Glu Thr His Gly Gly Glu Asp Val Ala Val Phe Ser Lys Gly
   435 440 445
   Pro Met Ala His Leu Leu His Gly Val His Glu Gln Asn Tyr Val Pro
   450 455 460
   His Val Met Ala Tyr Ala Ala Cys Ile Gly Ala Asn Leu Gly His Cys
   465 470 475 480
   Ala Pro Ala Ser Ser
   485
<210> 6
   <211> 524
   <212> PRT
   <213> bos taurus
<400> 6
   Met Ile Ser Pro Phe Leu Leu Leu Ala Ile Gly Thr Cys Phe Ala Ser
   1 5 10 15
   Ser Leu Val Pro Glu Lys Glu Lys Asp Pro Lys Tyr Trp Arg Asp Gln
   20 25 30
   Ala Gln Gln Thr Leu Lys Asn Ala Leu Arg Leu Gln Thr Leu Asn Thr
   35 40 45
   Asn Val Ala Lys Asn Val Ile Met Phe Leu Gly Asp Gly Met Gly Val
   50 55 60
   Ser Thr Val Thr Ala Ala Arg Ile Leu Lys Gly Gln Leu His His Ser
   65 70 75 80
   Pro Gly Glu Glu Thr Lys Leu Glu Met Asp Lys Phe Pro Tyr Val Ala
   85 90 95
   Leu Ser Lys Thr Tyr Asn Thr Asn Ala Gln Val Pro Asp Ser Ala Gly
   100 105 110
   Thr Ala Thr Ala Tyr Leu Cys Gly Val Lys Ala Asn Glu Gly Thr Val
   115 120 125
   Gly Val Ser Ala Ala Thr Gln Arg Ser Gln Cys Asn Thr Thr Gln Gly
   130 135 140
   Asn Glu Val Thr Ser Ile Leu Arg Trp Ala Lys Asp Ala Gly Lys Ser
   145 150 155 160
   Val Gly Ile Val Thr Thr Thr Arg Val Asn His Ala Thr Pro Ser Ala
   165 170 175
   Ser Tyr Ala His Ser Ala Asp Arg Asp Trp Tyr Ser Asp Asn Glu Met
   180 185 190
   Pro Pro Glu Ala Leu Ser Gln Gly Cys Lys Asp Ile Ala Tyr Gln Leu
   195 200 205
   Met Tyr Asn Ile Lys Asp Ile Glu Val Ile Met Gly Gly Gly Arg Lys
   210 215 220
   Tyr Met Phe Pro Lys Asn Arg Thr Asp Val Glu Tyr Glu Leu Asp Glu
   225 230 235 240
   Lys Ala Arg Gly Thr Arg Leu Asp Gly Leu Asn Leu Ile Asp Ile Trp
   245 250 255
   Lys Ser Phe Lys Pro Lys His Lys His Ser His Tyr Val Trp Asn Arg
   260 265 270
   Thr Asp Leu Leu Ala Leu Asp Pro His Ser Val Asp Tyr Leu Leu Gly
   275 280 285
   Leu Phe Glu Pro Gly Asp Met Gln Tyr Glu Leu Asn Arg Asn Asn Ala
   290 295 300
   Thr Asp Pro Ser Leu Ser Glu Met Val Glu Met Ala Ile Arg Ile Leu
   305 310 315 320
   Asn Lys Asn Pro Lys Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Gln Ala Ile Gly Gln Ala Gly Ala Met Thr Ser Val
   355 360 365
   Glu Asp Thr Leu Thr Val Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Val Ser Asp Thr Asp Lys Lys Pro Phe Thr Ala Ile Leu Tyr Gly
   405 410 415
   Asn Gly Pro Gly Tyr Lys Val Val Gly Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Ala Lys
   450 455 460
   Gly Pro Met Ala His Leu Leu His Gly Val Gln Glu Gln Asn Tyr Ile
   465 470 475 480
   Pro His Val Met Ala Tyr Ala Ala Cys Ile Gly Ala Asn Arg Asp His
   485 490 495
   Cys Ala Ser Ala Ser Ser Ser Gly Ser Pro Ser Pro Gly Pro Leu Leu
   500 505 510
   Leu Leu Leu Ala Leu Leu Pro Leu Gly Ser Leu Phe
   515 520
<210> 7
   <211> 524
   <212> PRT
   <213> felis catus
<400> 7
   Met Ile Ser Pro Phe Leu Val Leu Ala Ile Gly Thr Cys Leu Thr Asn
   1 5 10 15
   Ser Leu Val Pro Glu Lys Glu Lys Asp Pro Lys Tyr Trp Arg Asp Gln
   20 25 30
   Ala Gln Gln Thr Leu Lys Asn Ala Leu Arg Leu Gln Lys Leu Asn Thr
   35 40 45
   Asn Val Val Lys Asn Val Ile Met Phe Leu Gly Asp Gly Met Gly Val
   50 55 60
   Ser Thr Val Thr Ala Ala Arg Ile Leu Lys Gly Gln Leu His His Asn
   65 70 75 80
   Pro Gly Glu Glu Thr Arg Leu Glu Met Asp Lys Phe Pro Tyr Val Ala
   85 90 95
   Leu Ser Lys Thr Tyr Asn Thr Asn Ala Gln Val Pro Asp Ser Ala Gly
   100 105 110
   Thr Ala Thr Ala Tyr Leu Cys Gly Val Lys Ala Asn Glu Gly Thr Val
   115 120 125
   Gly Val Ser Ala Ala Thr Gln Arg Thr Gln Cys Asn Thr Thr Gln Gly
   130 135 140
   Asn Glu Val Thr Ser Ile Leu Arg Trp Ala Lys Asp Ser Gly Lys Ser
   145 150 155 160
   Val Gly Ile Val Thr Thr Thr Arg Val Asn His Ala Thr Pro Ser Ala
   165 170 175
   Ala Tyr Ala His Ser Ala Asp Arg Asp Trp Tyr Ser Asp Asn Glu Met
   180 185 190
   Pro Pro Glu Ala Leu Ser Gln Gly Cys Lys Asp Ile Ala Tyr Gln Leu
   195 200 205
   Met His Asn Val Arg Asp Ile Glu Val Ile Met Gly Gly Gly Arg Lys
   210 215 220
   Tyr Met Phe Pro Lys Asn Arg Thr Asp Val Glu Tyr Glu Met Asp Glu
   225 230 235 240
   Lys Ala Arg Gly Thr Arg Leu Asp Gly Leu Asn Leu Val Asp Ile Trp
   245 250 255
   Lys Ser Phe Lys Pro Arg His Lys His Ser His Tyr Val Trp Asn Arg
   260 265 270
   Thr Glu Leu Leu Thr Leu Asp Pro Tyr Gly Val Asp Tyr Leu Leu Gly
   275 280 285
   Leu Phe Glu Pro Gly Asp Met Gln Tyr Glu Leu Asn Arg Asn Ser Thr
   290 295 300
   Thr Asp Pro Ser Leu Ser Glu Met Val Glu Ile Ala Ile Lys Ile Leu
   305 310 315 320
   Ser Lys Asn Pro Lys Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Gln Ala Ile Gly Arg Ala Gly Ala Met Thr Ser Val
   355 360 365
   Glu Asp Thr Leu Thr Ile Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Val Ser Asp Thr Asp Lys Lys Pro Phe Thr Ser Ile Leu Tyr Gly
   405 410 415
   Asn Gly Pro Gly Tyr Lys Val Val Gly Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Ala Lys
   450 455 460
   Gly Pro Met Ala His Leu Leu His Gly Val His Glu Gln Asn Tyr Ile
   465 470 475 480
   Pro His Val Met Ala Tyr Ala Ala Cys Ile Gly Ala Asn Leu Asp His
   485 490 495
   Cys Ala Ser Ala Ser Ser Ala Gly Gly Pro Ser Pro Gly Pro Leu Phe
   500 505 510
   Leu Leu Leu Ala Leu Pro Ser Leu Gly Ile Leu Phe
   515 520
<210> 8
   <211> 524
   <212> PRT
   <213> homo sapiens
<400> 8
   Met Ile Ser Pro Phe Leu Val Leu Ala Ile Gly Thr Cys Leu Thr Asn
   1 5 10 15
   Ser Leu Val Pro Glu Lys Glu Lys Asp Pro Lys Tyr Trp Arg Asp Gln
   20 25 30
   Ala Gln Glu Thr Leu Lys Tyr Ala Leu Glu Leu Gln Lys Leu Asn Thr
   35 40 45
   Asn Val Ala Lys Asn Val Ile Met Phe Leu Gly Asp Gly Met Gly Val
   50 55 60
   Ser Thr Val Thr Ala Ala Arg Ile Leu Lys Gly Gln Leu His His Asn
   65 70 75 80
   Pro Gly Glu Glu Thr Arg Leu Glu Met Asp Lys Phe Pro Phe Val Ala
   85 90 95
   Leu Ser Lys Thr Tyr Asn Thr Asn Ala Gln Val Pro Asp Ser Ala Gly
   100 105 110
   Thr Ala Thr Ala Tyr Leu Cys Gly Val Lys Ala Asn Glu Gly Thr Val
   115 120 125
   Gly Val Ser Ala Ala Thr Glu Arg Ser Arg Cys Asn Thr Thr Gln Gly
   130 135 140
   Asn Glu Val Thr Ser Ile Leu Arg Trp Ala Lys Asp Ala Gly Lys Ser
   145 150 155 160
   Val Gly Ile Val Thr Thr Thr Arg Val Asn His Ala Thr Pro Ser Ala
   165 170 175
   Ala Tyr Ala His Ser Ala Asp Arg Asp Trp Tyr Ser Asp Asn Glu Met
   180 185 190
   Pro Pro Glu Ala Leu Ser Gln Gly Cys Lys Asp Ile Ala Tyr Gln Leu
   195 200 205
   Met His Asn Ile Arg Asp Ile Asp Val Ile Met Gly Gly Gly Arg Lys
   210 215 220
   Tyr Met Tyr Pro Lys Asn Lys Thr Asp Val Glu Tyr Glu Ser Asp Glu
   225 230 235 240
   Lys Ala Arg Gly Thr Arg Leu Asp Gly Leu Asp Leu Val Asp Thr Trp
   245 250 255
   Lys Ser Phe Lys Pro Arg Tyr Lys His Ser His Phe Ile Trp Asn Arg
   260 265 270
   Thr Glu Leu Leu Thr Leu Asp Pro His Asn Val Asp Tyr Leu Leu Gly
   275 280 285
   Leu Phe Glu Pro Gly Asp Met Gln Tyr Glu Leu Asn Arg Asn Asn Val
   290 295 300
   Thr Asp Pro Ser Leu Ser Glu Met Val Val Val Ala Ile Gln Ile Leu
   305 310 315 320
   Arg Lys Asn Pro Lys Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Arg Ala Ile Gly Gln Ala Gly Ser Leu Thr Ser Ser
   355 360 365
   Glu Asp Thr Leu Thr Val Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Leu Ser Asp Thr Asp Lys Lys Pro Phe Thr Ala Ile Leu Tyr Gly
   405 410 415
   Asn Gly Pro Gly Tyr Lys Val Val Gly Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Ser Lys
   450 455 460
   Gly Pro Met Ala His Leu Leu His Gly Val His Glu Gln Asn Tyr Val
   465 470 475 480
   Pro His Val Met Ala Tyr Ala Ala Cys Ile Gly Ala Asn Leu Gly His
   485 490 495
   Cys Ala Pro Ala Ser Ser Ala Gly Ser Leu Ala Ala Gly Pro Leu Leu
   500 505 510
   Leu Ala Leu Ala Leu Tyr Pro Leu Ser Val Leu Phe
   515 520

<210> 9
   <211> 524
   <212> PRT
   <213> mus musculis
<400> 9
   Met Ile Ser Pro Phe Leu Val Leu Ala Ile Gly Thr Cys Leu Thr Asn
   1 5 10 15
   Ser Phe Val Pro Glu Lys Glu Arg Asp Pro Ser Tyr Trp Arg Gln Gln
   20 25 30
   Ala Gln Glu Thr Leu Lys Asn Ala Leu Lys Leu Gln Lys Leu Asn Thr
   35 40 45
   Asn Val Ala Lys Asn Val Ile Met Phe Leu Gly Asp Gly Met Gly Val
   50 55 60
   Ser Thr Val Thr Ala Ala Arg Ile Leu Lys Gly Gln Leu His His Asn
   65 70 75 80
   Thr Gly Glu Glu Thr Arg Leu Glu Met Asp Lys Phe Pro Phe Val Ala
   85 90 95
   Leu Ser Lys Thr Tyr Asn Thr Asn Ala Gln Val Pro Asp Ser Ala Gly
   100 105 110
   Thr Ala Thr Ala Tyr Leu Cys Gly Val Lys Ala Asn Glu Gly Thr Val
   115 120 125
   Gly Val Ser Ala Ala Thr Glu Arg Thr Arg Cys Asn Thr Thr Gln Gly
   130 135 140
   Asn Glu Val Thr Ser Ile Leu Arg Trp Ala Lys Asp Ala Gly Lys Ser
   145 150 155 160
   Val Gly Ile Val Thr Thr Thr Arg Val Asn His Ala Thr Pro Ser Ala
   165 170 175
   Ala Tyr Ala His Ser Ala Asp Arg Asp Trp Tyr Ser Asp Asn Glu Met
   180 185 190
   Pro Pro Glu Ala Leu Ser Gln Gly Cys Lys Asp Ile Ala Tyr Gln Leu
   195 200 205
   Met His Asn Ile Lys Asp Ile Asp Val Ile Met Gly Gly Gly Arg Lys
   210 215 220
   Tyr Met Tyr Pro Lys Asn Arg Thr Asp Val Glu Tyr Glu Leu Asp Glu
   225 230 235 240
   Lys Ala Arg Gly Thr Arg Leu Asp Gly Leu Asp Leu Ile Ser Ile Trp
   245 250 255
   Lys Ser Phe Lys Pro Arg His Lys His Ser His Tyr Val Trp Asn Arg
   260 265 270
   Thr Glu Leu Leu Ala Leu Asp Pro Ser Arg Val Asp Tyr Leu Leu Gly
   275 280 285
   Leu Phe Glu Pro Gly Asp Met Gln Tyr Glu Leu Asn Arg Asn Asn Leu
   290 295 300
   Thr Asp Pro Ser Leu Ser Glu Met Val Glu Val Ala Leu Arg Ile Leu
   305 310 315 320
   Thr Lys Asn Leu Lys Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Gln Ala Ile Gly Lys Ala Gly Ala Met Thr Ser Gln
   355 360 365
   Lys Asp Thr Leu Thr Val Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Val Ser Asp Thr Asp Lys Lys Pro Phe Thr Ala Ile Leu Tyr Gly
   405 410 415
   Asn Gly Pro Gly Tyr Lys Val Val Asp Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Ala Lys
   450 455 460
   Gly Pro Met Ala His Leu Leu His Gly Val His Glu Gln Asn Tyr Ile
   465 470 475 480
   Pro His Val Met Ala Tyr Ala Ser Cys Ile Gly Ala Asn Leu Asp His
   485 490 495
   Cys Ala Trp Ala Gly Ser Gly Ser Ala Pro Ser Pro Gly Ala Leu Leu
   500 505 510
   Leu Pro Leu Ala Val Leu Ser Leu Pro Thr Leu Phe
   515 520
<210> 10
   <211> 524
   <212> PRT
   <213> rattus norvegicus
<400> 10
   Met Ile Leu Pro Phe Leu Val Leu Ala Ile Gly Thr Cys Leu Thr Asn
   1               5                   10                  15
   Ser Phe Val Pro Glu Lys Glu Lys Asp Pro Ser Tyr Trp Arg Gln Gln
               20                  25                  30
   Ala Gln Glu Thr Leu Lys Asn Ala Leu Lys Leu Gln Lys Leu Asn Thr
           35                  40                  45
   Asn Val Ala Lys Asn Ile Ile Met Phe Leu Gly Asp Gly Met Gly Val
       50                  55                  60
   Ser Thr Val Thr Ala Ala Arg Ile Leu Lys Gly Gln Leu His His Asn
   65                  70                  75                  80
   Thr Gly Glu Glu Thr Arg Leu Glu Met Asp Lys Phe Pro Phe Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Glu Arg Thr Arg Cys Asn Thr Thr Gln Gly
       130                 135                 140
   Asn Glu Val Thr Ser Ile Leu Arg Trp Ala Lys Asp Ala Gly Lys Ser
   145                 150                 155                 160
   Val Gly Ile Val Thr Thr Thr Arg Val Asn His Ala Thr Pro Ser Ala
                   165                 170                 175
   Ala Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
   195 200 205
   Met His Asn Ile Lys Asp Ile Asp Val Ile Met Gly Gly Gly Arg Lys
   210 215 220
   Tyr Met Tyr Pro Lys Asn Arg Thr Asp Val Glu Tyr Glu Leu Asp Glu
   225 230 235 240
   Lys Ala Arg Gly Thr Arg Leu Asp Gly Leu Asp Leu Ile Ser Ile Trp
   245 250 255
   Lys Ser Phe Lys Pro Arg His Lys His Ser His Tyr Val Trp Asn Arg
   260 265 270
   Thr Glu Leu Leu Ala Leu Asp Pro Ser Arg Val Asp Tyr Leu Leu Gly
   275 280 285
   Leu Phe Glu Pro Gly Asp Met Gln Tyr Glu Leu Asn Arg Asn Asn Leu
   290 295 300
   Thr Asp Pro Ser Leu Ser Glu Met Val Glu Val Ala Leu Arg Ile Leu
   305 310 315 320
   Thr Lys Asn Pro Lys Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Glu Ala Ile Gly Lys Ala Gly Thr Met Thr Ser Gln
   355 360 365
   Lys Asp Thr Leu Thr Val Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Val Ser Asp Thr Asp Lys Lys Pro Phe Thr Ala Ile Leu Tyr Gly
   405 410 415
   Asn Gly Pro Gly Tyr Lys Val Val Asp Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Ala Lys
   450 455 460
   Gly Pro Met Ala His Leu Leu His Gly Val His Glu Gln Asn Tyr Ile
   465 470 475 480
   Pro His Val Met Ala Tyr Ala Ser Cys Ile Gly Ala Asn Leu Asp His
   485 490 495
   Cys Ala Trp Ala Ser Ser Ala Ser Ser Pro Ser Pro Gly Ala Leu Leu
   500 505 510
   Leu Pro Leu Ala Leu Phe Pro Leu Arg Thr Leu Phe
   515 520
<210> 11
   <211> 502
   <212> PRT
   <213> Canis familiaris
<400> 11
   Glu Lys Asp Pro Lys Tyr Trp Arg Asp Gln Ala Gln Gln Thr Leu Lys
   1 5 10 15
   Tyr Ala Leu Arg Leu Gln Asn Leu Asn Thr Asn Val Ala Lys Asn Val
   20 25 30
   Ile Met Phe Leu Gly Asp Gly Met Gly Val Ser Thr Val Thr Ala Thr
   35 40 45
   Arg Ile Leu Lys Gly Gln Leu His His Asn Pro Gly Glu Glu Thr Arg
   50 55 60
   Leu Glu Met Asp Lys Phe Pro Tyr Val Ala Leu Ser Lys Thr Tyr Asn
   65 70 75 80
   Thr Asn Ala Gln Val Pro Asp Ser Ala Gly Thr Ala Thr Ala Tyr Leu
   85 90 95
   Cys Gly Val Lys Ala Asn Glu Gly Thr Val Gly Val Ser Ala Ala Thr
   100 105 110
   Gln Arg Thr His Cys Asn Thr Thr Gln Gly Asn Glu Val Thr Ser Ile
   115 120 125
   Leu Arg Trp Ala Lys Asp Ala Gly Lys Ser Val Gly Ile Val Thr Thr
   130 135 140
   Thr Arg Val Asn His Ala Thr Pro Ser Ala Ala Tyr Ala His Ser Ala
   145 150 155 160
   Asp Arg Asp Trp Tyr Ser Asp Asn Glu Met Pro Pro Glu Ala Leu Ser
   165 170 175
   Gln Gly Cys Lys Asp Ile Ala Tyr Gln Leu Met His Asn Val Lys Asp
   180 185 190
   Ile Glu Val Ile Met Gly Gly Gly Arg Lys Tyr Met Phe Pro Lys Asn
   195 200 205
   Arg Thr Asp Val Glu Tyr Glu Met Asp Glu Lys Ser Thr Gly Ala Arg
   210 215 220
   Leu Asp Gly Leu Asn Leu Ile Asp Ile Trp Lys Asn Phe Lys Pro Arg
   225 230 235 240
   His Lys His Ser His Tyr Val Trp Asn Arg Thr Glu Leu Leu Ala Leu
   245 250 255
   Asp Pro Tyr Thr Val Asp Tyr Leu Leu Gly Leu Phe Asp Pro Gly Asp
   260 265 270
   Met Gln Tyr Glu Leu Asn Arg Asn Asn Val Thr Asp Pro Ser Leu Ser
   275 280 285
   Glu Met Val Glu Ile Ala Ile Lys Ile Leu Ser Lys Lys Pro Arg Gly
   290 295 300
   Phe Phe Leu Leu Val Glu Gly Gly Arg Ile Asp His Gly His His Glu
   305 310 315 320
   Gly Lys Ala Lys Gln Ala Leu His Glu Ala Val Glu Met Asp Arg Ala
   325 330 335
   Ile Gly Lys Ala Gly Val Met Thr Ser Leu Glu Asp Thr Leu Thr Val
   340 345 350
   Val Thr Ala Asp His Ser His Val Phe Thr Phe Gly Gly Tyr Thr Pro
   355 360 365
   Arg Gly Asn Ser Ile Phe Gly Leu Ala Pro Met Val Ser Asp Thr Asp
   370 375 380
   Lys Lys Pro Phe Thr Ala Ile Leu Tyr Gly Asn Gly Pro Gly Tyr Lys
   385 390 395 400
   Val Val Gly Gly Glu Arg Glu Asn Val Ser Met Val Asp Tyr Ala His
   405 410 415
   Asn Asn Tyr Gln Ala Gln Ser Ala Val Pro Leu Arg His Glu Thr His
   420 425 430
   Gly Gly Glu Asp Val Ala Val Phe Ala Lys Gly Pro Met Ala His Leu
   435 440 445
   Leu His Gly Val His Glu Gln Asn Tyr Ile Pro His Val Met Ala Tyr
   450 455 460
   Ala Ala Cys Ile Gly Ala Asn Gln Asp His Cys Ala Ser Ala Ser Ser
   465 470 475 480
   Ala Gly Gly Pro Ser Pro Gly Pro Leu Leu Leu Leu Leu Ala Leu Leu
   485 490 495
   Pro Val Gly Ile Leu Phe
   500
<210> 12
   <211> 528
   <212> PRT
   <213> homo sapiens
<400> 12
   Met Gln Gly Pro Trp Val Leu Leu Leu Leu Gly Leu Arg Leu Gln Leu
   1 5 10 15
   Ser Leu Gly Val Ile Pro Ala Glu Glu Glu Asn Pro Ala Phe Trp Asn
               20                  25                  30
   Arg Gln Ala Ala Glu Ala Leu Asp Ala Ala Lys Lys Leu Gln Pro Ile
           35                  40                  45
   Gln Lys Val Ala Lys Asn Leu Ile Leu Phe Leu Gly Asp Gly Leu Gly
       50                  55                  60
   Val Pro Thr Val Thr Ala Thr Arg Ile Leu Lys Gly Gln Lys Asn Gly
   65                  70                  75                  80
   Lys Leu Gly Pro Glu Thr Pro Leu Ala Met Asp Arg Phe Pro Tyr Leu
                   85                  90                  95
   Ala Leu Ser Lys Thr Tyr Asn Val Asp Arg Gln Val Pro Asp Ser Ala
               100                 105                 110
   Ala Thr Ala Thr Ala Tyr Leu Cys Gly Val Lys Ala Asn Phe Gln Thr
           115                 120                 125
   Ile Gly Leu Ser Ala Ala Ala Arg Phe Asn Gln Cys Asn Thr Thr Arg
       130                 135                 140
   Gly Asn Glu Val Ile Ser Val Met Asn Arg Ala Lys Gln Ala Gly Lys
   145                 150                 155                 160
   Ser Val Gly Val Val Thr Thr Thr Arg Val Gln His Ala Ser Pro Ala
                   165                 170                 175
   Gly Thr Tyr Ala His Thr Val Asn Arg Asn Trp Tyr Ser Asp Ala Asp
               180                 185                 190
   Met Pro Ala Ser Ala Arg Gln Glu Gly Cys Gln Asp Ile Ala Thr Gln
           195                 200                 205
   Leu Ile Ser Asn Met Asp Ile Asp Val Ile Leu Gly Gly Gly Arg Lys
       210                 215                 220
   Tyr Met Phe Pro Met Gly Thr Pro Asp Pro Glu Tyr Pro Ala Asp Ala
   225                 230                 235                 240
   Ser Gln Asn Gly Ile Arg Leu Asp Gly Lys Asn Leu Val Gln Glu Trp
   245 250 255
   Leu Ala Lys His Gln Gly Ala Trp Tyr Val Trp Asn Arg Thr Glu Leu
   260 265 270
   Met Gln Ala Ser Leu Asp Gln Ser Val Thr His Leu Met Gly Leu Phe
   275 280 285
   Glu Pro Gly Asp Thr Lys Tyr Glu Ile His Arg Asp Pro Thr Leu Asp
   290 295 300
   Pro Ser Leu Met Glu Met Thr Glu Ala Ala Leu Arg Leu Leu Ser Arg
   305 310 315 320
   Asn Pro Arg Gly Phe Tyr Leu Phe Val Glu Gly Gly Arg Ile Asp His
   325 330 335
   Gly His His Glu Gly Val Ala Tyr Gln Ala Leu Thr Glu Ala Val Met
   340 345 350
   Phe Asp Asp Ala Ile Glu Arg Ala Gly Gln Leu Thr Ser Glu Glu Asp
   355 360 365
   Thr Leu Thr Leu Val Thr Ala Asp His Ser His Val Phe Ser Phe Gly
   370 375 380
   Gly Tyr Thr Leu Arg Gly Ser Ser Ile Phe Gly Leu Ala Pro Ser Lys
   385 390 395 400
   Ala Gln Asp Ser Lys Ala Tyr Thr Ser Ile Leu Tyr Gly Asn Gly Pro
   405 410 415
   Gly Tyr Val Phe Asn Ser Gly Val Arg Pro Asp Val Asn Glu Ser Glu
   420 425 430
   Ser Gly Ser Pro Asp Tyr Gln Gln Gln Ala Ala Val Pro Leu Ser Ser
   435 440 445
   Glu Thr His Gly Gly Glu Asp Val Ala Val Phe Ala Arg Gly Pro Gln
   450 455 460
   Ala His Leu Val His Gly Val Gln Glu Gln Ser Phe Val Ala His Val
   465 470 475 480
   Met Ala Phe Ala Ala Cys Leu Glu Pro Tyr Thr Ala Cys Asp Leu Ala
   485 490 495
   Pro Pro Ala Cys Thr Thr Asp Ala Ala His Pro Val Ala Ala Ser Leu
   500 505 510
   Pro Leu Leu Ala Gly Thr Leu Leu Leu Leu Gly Ala Ser Ala Ala Pro
   515 520 525
<210> 13
   <211> 532
   <212> PRT
   <213> homo sapiens
<400> 13
   Met Gln Gly Pro Trp Val Leu Leu Leu Leu Gly Leu Arg Leu Gln Leu
   1 5 10 15
   Ser Leu Gly Ile Ile Pro Val Glu Glu Glu Asn Pro Asp Phe Trp Asn
   20 25 30
   Arg Gln Ala Ala Glu Ala Leu Gly Ala Ala Lys Lys Leu Gln Pro Ala
   35 40 45
   Gln Thr Ala Ala Lys Asn Leu Ile Ile Phe Leu Gly Asp Gly Met Gly
   50 55 60
   Val Ser Thr Val Thr Ala Ala Arg Ile Leu Lys Gly Gln Lys Lys Asp
   65 70 75 80
   Lys Leu Gly Pro Glu Thr Phe Leu Ala Met Asp Arg Phe Pro Tyr Val
   85 90 95
   Ala Leu Ser Lys Thr Tyr Ser Val Asp Lys His Val Pro Asp Ser Gly
   100 105 110
   Ala Thr Ala Thr Ala Tyr Leu Cys Gly Val Lys Gly Asn Phe Gln Thr
   115 120 125
   Ile Gly Leu Ser Ala Ala Ala Arg Phe Asn Gln Cys Asn Thr Thr Arg
   130 135 140
   Gly Asn Glu Val Ile Ser Val Met Asn Arg Ala Lys Lys Ala Gly Lys
   145 150 155 160
   Ser Val Gly Val Val Thr Thr Thr Arg Val Gln His Ala Ser Pro Ala
   165 170 175
   Gly Ala Tyr Ala His Thr Val Asn Arg Asn Trp Tyr Ser Asp Ala Asp
   180 185 190
   Val Pro Ala Ser Ala Arg Gln Glu Gly Cys Gln Asp Ile Ala Thr Gln
   195 200 205
   Leu Ile Ser Asn Met Asp Ile Asp Val Ile Leu Gly Gly Gly Arg Lys
   210 215 220
   Tyr Met Phe Pro Met Gly Thr Pro Asp Pro Glu Tyr Pro Asp Asp Tyr
   225 230 235 240
   Ser Gln Gly Gly Thr Arg Leu Asp Gly Lys Asn Leu Val Gln Glu Trp
   245 250 255
   Leu Ala Lys His Gln Gly Ala Arg Tyr Val Trp Asn Arg Thr Glu Leu
   260 265 270
   Leu Gln Ala Ser Leu Asp Pro Ser Val Thr His Leu Met Gly Leu Phe
   275 280 285
   Glu Pro Gly Asp Met Lys Tyr Glu Ile His Arg Asp Ser Thr Leu Asp
   290 295 300
   Pro Ser Leu Met Glu Met Thr Glu Ala Ala Leu Leu Leu Leu Ser Arg
   305 310 315 320
   Asn Pro Arg Gly Phe Phe Leu Phe Val Glu Gly Gly Arg Ile Asp His
   325 330 335
   Gly His His Glu Ser Arg Ala Tyr Arg Ala Leu Thr Glu Thr Ile Met
   340 345 350
   Phe Asp Asp Ala Ile Glu Arg Ala Gly Gln Leu Thr Ser Glu Glu Asp
   355 360 365
   Thr Leu Ser Leu Val Thr Ala Asp His Ser His Val Phe Ser Phe Gly
   370 375 380
   Gly Tyr Pro Leu Arg Gly Ser Ser Ile Phe Gly Leu Ala Pro Gly Lys
   385 390 395 400
   Ala Arg Asp Arg Lys Ala Tyr Thr Val Leu Leu Tyr Gly Asn Gly Pro
   405 410 415
   Gly Tyr Val Leu Lys Asp Gly Ala Arg Pro Asp Val Thr Glu Ser Glu
   420 425 430
   Ser Gly Ser Pro Glu Tyr Arg Gln Gln Ser Ala Val Pro Leu Asp Gly
   435 440 445
   Glu Thr His Ala Gly Glu Asp Val Ala Val Phe Ala Arg Gly Pro Gln
   450 455 460
   Ala His Leu Val His Gly Val Gln Glu Gln Thr Phe Ile Ala His Val
   465 470 475 480
   Met Ala Phe Ala Ala Cys Leu Glu Pro Tyr Thr Ala Cys Asp Leu Ala
   485 490 495
   Pro Arg Ala Gly Thr Thr Asp Ala Ala His Pro Gly Pro Ser Val Val
   500 505 510
   Pro Ala Leu Leu Pro Leu Leu Ala Gly Thr Leu Leu Leu Leu Gly Thr
   515 520 525
   Ala Thr Ala Pro
   530
<210> 14
   <211> 535
   <212> PRT
   <213> homo sapiens
<400> 14
   Met Leu Gly Pro Cys Met Leu Leu Leu Leu Leu Leu Leu Gly Leu Arg
   1 5 10 15
   Leu Gln Leu Ser Leu Gly Ile Ile Pro Val Glu Glu Glu Asn Pro Asp
   20 25 30
   Phe Trp Asn Arg Glu Ala Ala Glu Ala Leu Gly Ala Ala Lys Lys Leu
   35 40 45
   Gln Pro Ala Gln Thr Ala Ala Lys Asn Leu Ile Ile Phe Leu Gly Asp
   50 55 60
   Gly Met Gly Val Ser Thr Val Thr Ala Ala Arg Ile Leu Lys Gly Gln
   65 70 75 80
   Lys Lys Asp Lys Leu Gly Pro Glu Ile Pro Leu Ala Met Asp Arg Phe
   85 90 95
   Pro Tyr Val Ala Leu Ser Lys Thr Tyr Asn Val Asp Lys His Val Pro
   100 105 110
   Asp Ser Gly Ala Thr Ala Thr Ala Tyr Leu Cys Gly Val Lys Gly Asn
   115 120 125
   Phe Gln Thr Ile Gly Leu Ser Ala Ala Ala Arg Phe Asn Gln Cys Asn
   130 135 140
   Thr Thr Arg Gly Asn Glu Val Ile Ser Val Met Asn Arg Ala Lys Lys
   145 150 155 160
   Ala Gly Lys Ser Val Gly Val Val Thr Thr Thr Arg Val Gln His Ala
   165 170 175
   Ser Pro Ala Gly Thr Tyr Ala His Thr Val Asn Arg Asn Trp Tyr Ser
   180 185 190
   Asp Ala Asp Val Pro Ala Ser Ala Arg Gln Glu Gly Cys Gln Asp Ile
   195 200 205
   Ala Thr Gln Leu Ile Ser Asn Met Asp Ile Asp Val Ile Leu Gly Gly
   210 215 220
   Gly Arg Lys Tyr Met Phe Arg Met Gly Thr Pro Asp Pro Glu Tyr Pro
   225 230 235 240
   Asp Asp Tyr Ser Gln Gly Gly Thr Arg Leu Asp Gly Lys Asn Leu Val
   245 250 255
   Gln Glu Trp Leu Ala Lys Arg Gln Gly Ala Arg Tyr Val Trp Asn Arg
               260                 265                 270
   Thr Glu Leu Met Gln Ala Ser Leu Asp Pro Ser Val Thr His Leu Met
           275                 280                 285
   Gly Leu Phe Glu Pro Gly Asp Met Lys Tyr Glu Ile His Arg Asp Ser
       290                 295                 300
   Thr Leu Asp Pro Ser Leu Met Glu Met Thr Glu Ala Ala Leu Arg Leu
   305                 310                 315                 320
   Leu Ser Arg Asn Pro Arg Gly Phe Phe Leu Phe Val Glu Gly Gly Arg
                   325                 330                 335
   Ile Asp His Gly His His Glu Ser Arg Ala Tyr Arg Ala Leu Thr Glu
               340                 345                 350
   Thr Ile Met Phe Asp Asp Ala Ile Glu Arg Ala Gly Gln Leu Thr Ser
           355                 360                 365
   Glu Glu Asp Thr Leu Ser Leu Val Thr Ala Asp His Ser His Val Phe
       370                 375                 380
   Ser Phe Gly Gly Tyr Pro Leu Arg Gly Ser Ser Ile Phe Gly Leu Ala
   385                 390                 395                 400
   Pro Gly Lys Ala Arg Asp Arg Lys Ala Tyr Thr Val Leu Leu Tyr Gly
                   405                 410                 415
   Asn Gly Pro Gly Tyr Val Leu Lys Asp Gly Ala Arg Pro Asp Val Thr
               420                 425                 430
   Glu Ser Glu Ser Gly Ser Pro Glu Tyr Arg Gln Gln Ser Ala Val Pro
           435                 440                 445
   Leu Asp Glu Glu Thr His Ala Gly Glu Asp Val Ala Val Phe Ala Arg
       450                 455                 460
   Gly Pro Gln Ala His Leu Val His Gly Val Gln Glu Gln Thr Phe Ile
   465                 470                 475                 480
   Ala His Val Met Ala Phe Ala Ala Cys Leu Glu Pro Tyr Thr Ala Cys
   485 490 495
   Asp Leu Ala Pro Pro Ala Gly Thr Thr Asp Ala Ala His Pro Gly Arg
   500 505 510
   Ser Val Val Pro Ala Leu Leu Pro Leu Leu Ala Gly Thr Leu Leu Leu
   515 520 525
   Leu Glu Thr Ala Thr Ala Pro
   530 535
<210> 15
   <211> 541
   <212> PRT
   <213> Artificial
<220> <223> Consensus ALP: TNALP from various mammalian species and human ALP isozymes PLAP, GCALP, IALP (with signal peptide and GPI anchor domain)
<220>
   <221> misc_feature
   <222> (1)..(4)
   <223> Xaa can be any naturally occurring amino acid
<220>
   <221> VARIANT
   <222> (5)..(9)
   <223> Xaa can be any naturally occurring amino acid or absent
<220>
   <221> misc_feature
   <222> (10)..(21)
   <223> Xaa can be any naturally occurring amino acid
<220>
   <221> VARIANT
   <222> (22)..(22)
   <223> Xaa can be any naturally occurring amino acid except phenylalanine
<220>
   <221> misc_feature
   <222> (23)..(27)
   <223> Xaa can be any naturally occurring amino acid
<220>
   <221> misc_feature
   <222> (29)..(30) <223> Xaa can be any naturally occurring amino acid
<220>
   <221> misc_feature
   <222> (32)..(32)
   <223> Xaa can be any naturally occurring amino acid
<220>
   <221> VARIANT
   <222> (33)..(33)
   <223> Xaa can be any naturally occurring amino acid except cysteine
<220>
   <221> misc_feature
   <222> (35)..(37)
   <223> Xaa can be any naturally occurring amino acid
<220>
   <221> misc_feature
   <222> (39)..(41)
   <223> Xaa can be any naturally occurring amino acid
<220>
   <221> misc_feature
   <222> (43)..(44)
   <223> Xaa can be any naturally occurring amino acid
<220>
   <221> misc_feature
   <222> (46)..(47)
   <223> Xaa can be any naturally occurring amino acid
<220>
   <221> misc_feature
   <222> (50)..(53)
   <223> Xaa can be any naturally occurring amino acid
<220>
   <221> VARIANT
   <222> (54)..(54)
   <223> Xaa can be any naturally occurring amino acid or absent
<220>
   <221> misc_feature
   <222> (55)..(55)
   <223> Xaa can be any naturally occurring amino acid
<220>
   <221> VARIANT
   <222> (56)..(56)
   <223> Xaa can be any naturally occurring amino acid except serine or
   valine
<220>
   <221> misc_feature
   <222> (59)..(59)
   <223> Xaa can be any naturally occurring amino acid
<220>
   <221> misc_feature
   <222> (61)..(61)
   <223> Xaa can be any naturally occurring amino acid
<220>
   <221> VARIANT
   <222> (67)..(67)
   <223> Xaa can be any naturally occurring amino acid except leucine, isoleucine or valine
<220>
   <221> misc feature
   <222> (70)..(70)
   <223> Xaa can be any naturally occurring amino acid
<220>
   <221> misc feature
   <222> (75)..(75)
   <223> Xaa can be any naturally occurring amino acid
<220>
   <221> misc feature
   <222> (B2)..(86)
   <223> Xaa can be any naturally occurring amino acid
<220>
   <221> misc feature
   <222> (88)..(88)
   <223> Xaa can be any naturally occurring amino acid
<220>
   <221> misc feature
   <222> (90)..(91)
   <223> Xaa can be any naturally occurring amino acid
<220>
   <221> misc feature
   <222> (93)..(93)
   <223> Xaa can be any naturally occurring amino acid
<220>
   <221> misc feature
   <222> (96) ..(96)
   <223> Xaa can be any naturally occurring amino acid
<220>
   <221> misc feature
   <222> (99)..(100)
   <223> Xaa can be any naturally occurring amino acid
<220>
   <221> misc_feature
   <222> (107)..(111)
   <223> Xaa can be any naturally occurring amino acid

<220>
   <221> VARIANT
   <222> (116)..(116)
   <223> Xaa can be any naturally occurring amino acid except threonine
<220>
   <221> VARIANT
   <222> (117)..(117)
   <223> Xaa can be any naturally occurring amino acid except serine
<220>
   <221> VARIANT
   <222> (128)..(128)
   <223> Xaa can be any naturally occurring amino acid except aspartate
<220>
   <221> misc_feature
   <222> (130)..(131)
   <223> Xaa can be any naturally occurring amino acid
<220>
   <221> VARIANT
   <222> (133)..(133)
   <223> Xaa can be any naturally occurring amino acid except methionine
<220>
   <221> misc_feature
   <222> (135)..(135)
   <223> Xaa can be any naturally occurring amino acid
<220>
   <221> VARIANT
   <222> (139)..(139)
   <223> Xaa can be any naturally occurring amino acid except histidine or asparagine
<220>
   <221> misc_feature
   <222> (140)..(140)
   <223> Xaa can be any naturally occurring amino acid
<220>
   <221> VARIANT
   <222> (141)..(141)
   <223> Xaa can be any naturally occurring amino acid except histidine
<220>
   <221> misc_feature
   <222> (142)..(143)
   <223> Xaa can be any naturally occurring amino acid
<220>
   <221> misc_feature
   <222> (148)..(148)
   <223> Xaa can be any naturally occurring amino acid
<220> <221> VARIANT
   <222> (153)..(153)
   <223> Xaa can be any naturally occurring amino acid except alanine or isoleucine
<220>
   <221> misc_feature
   <222> (155)..(158)
   <223> Xaa can be any naturally occurring amino acid
<220>
   <221> misc_feature
   <222> (161)..(162)
   <223> Xaa can be any naturally occurring amino acid
<220>
   <221> misc_feature
   <222> (168)..(168)
   <223> Xaa can be any naturally occurring amino acid
<220>
   <221> VARIANT
   <222> (175)..(175)
   <223> Xaa can be any naturally occurring amino acid except aspartate
<220>
   <221> misc_feature
   <222> (178)..(178)
   <223> Xaa can be any naturally occurring amino acid
<220>
   <221> misc feature
   <222> (180)..(180)
   <223> Xaa can be any naturally occurring amino acid
<220>
   <221> VARIANT
   <222> (181)..(181)
   <223> Xaa can be any naturally occurring amino acid except threonine
<220>
   <221> VARIANT
   <222> (182)..(182)
   <223> Xaa can be any naturally occurring amino acid except threonine
<220> <221> VARIANT
   <222> (186)..(186)
   <223> Xaa can be any naturally occurring amino acid except leucine
<220>
   <221> misc_feature
   <222> (187)..(188)
   <223> Xaa can be any naturally occurring amino acid
<220>
   <221> misc_feature <222> (190)..(190)
   <223> Xaa can be any naturally occurring amino acid
<220>
   <221> misc_feature
   <222> (195)..(195)
   <223> Xaa can be any naturally occurring amino acid
<220>
   <221> VARIANT
   <222> (196)..(196)
   <223> Xaa can be any naturally occurring amino acid except lysine or glycine
<220>
   <221> VARIANT
   <222> (197)..(197)
   <223> Xaa can be any naturally occurring amino acid except threonine
<220>
   <221> misc_feature
   <222> (199)..(200)
   <223> Xaa can be any naturally occurring amino acid
<220>
   <221> misc_feature
   <222> (202)..(204)
   <223> Xaa can be any naturally occurring amino acid
<220>
   <221> misc_feature
   <222> (207)..(207)
   <223> Xaa can be any naturally occurring amino acid
<220>
   <221> misc_feature
   <222> (211)..(211)
   <223> Xaa can be any naturally occurring amino acid
<220>
   <221> misc_feature
   <222> (214)..(215)
   <223> Xaa can be any naturally occurring amino acid
<220>
   <221> VARIANT
   <222> (217)..(217)
   <223> Xaa can be any naturally occurring amino acid except phenylalanine
<220>
   <221> VARIANT
   <222> (218)..(218)
   <223> Xaa can be any naturally occurring amino acid or absent
<220>
   <221> misc_feature
   <222> (221)..(221)
   <223> Xaa can be any naturally occurring amino acid
<220>
   <221> misc_feature
   <222> (224)..(224)
   <223> Xaa can be any naturally occurring amino acid
<220>
   <221> misc_feature
   <222> (232)..(237)
   <223> Xaa can be any naturally occurring amino acid
<220>
   <221> misc_feature
   <222> (239)..(239)
   <223> Xaa can be any naturally occurring amino acid
<220>
   <221> VARIANT
   <222> (240)..(240)
   <223> Xaa can be any naturally occurring amino acid except glycine
<220>
   <221> misc_feature
   <222> (242)..(243)
   <223> Xaa can be any naturally occurring amino acid
<220>
   <221> misc_feature
   <222> (245)..(248)
   <223> Xaa can be any naturally occurring amino acid
<220>
   <221> misc_feature
   <222> (250)..(250)
   <223> Xaa can be any naturally occurring amino acid
<220>
   <221> misc_feature
   <222> (255)..(256)
   <223> Xaa can be any naturally occurring amino acid
<220>
   <221> misc_feature
   <222> (258)..(260)
   <223> Xaa can be any naturally occurring amino acid
<220>
   <221> misc_feature
   <222> (262)..(262)
   <223> Xaa can be any naturally occurring amino acid
<220>
   <221> VARIANT
   <222> (263)..(265)
   <223> Xaa can be any naturally occurring amino acid or absent
<220>
   <221> misc_feature
   <222> (266)..(268)
   <223> Xaa can be any naturally occurring amino acid
<220>
   <221> VARIANT
   <222> (269)..(269)
   <223> Xaa can be any naturally occurring amino acid except arginine
<220>
   <221> misc_feature
   <222> (270)..(274)
   <223> Xaa can be any naturally occurring amino acid
<220>
   <221> misc_feature
   <222> (279)..(279)
   <223> Xaa can be any naturally occurring amino acid
<220>
   <221> misc_feature
   <222> (281)..(286)
   <223> Xaa can be any naturally occurring amino acid
<220>
   <221> VARIANT
   <222> (287)..(287)
   <223> Xaa can be any naturally occurring amino acid or absent
<220>
   <221> misc_feature
   <222> (288)..(288)
   <223> Xaa can be any naturally occurring amino acid
<220>
   <221> misc_feature
   <222> (290)..(291)
   <223> Xaa can be any naturally occurring amino acid
<220>
   <221> misc_feature
   <222> (293)..(293)
   <223> Xaa can be any naturally occurring amino acid
<220>
   <221> VARIANT
   <222> (297)..(297)
   <223> Xaa can be any naturally occurring amino acid except lysine
<220>
   <221> VARIANT
   <222> (301)..(301)
   <223> Xaa can be any naturally occurring amino acid except valine, threonine or isoleucine
<220>
   <221> misc_feature
   <222> (302)..(302)
   <223> Xaa can be any naturally occurring amino acid
<220>
   <221> VARIANT
   <222> (305)..(305)
   <223> Xaa can be any naturally occurring amino acid except proline
<220>
   <221> misc_feature
   <222> (306)..(306)
   <223> Xaa can be any naturally occurring amino acid
<220>
   <221> misc_feature
   <222> (308)..(311)
   <223> Xaa can be any naturally occurring amino acid
<220>
   <221> misc_feature
   <222> (316)..(316)
   <223> Xaa can be any naturally occurring amino acid
<220>
   <221> misc_feature
   <222> (319)..(321)
   <223> Xaa can be any naturally occurring amino acid
<220>
   <221> misc_feature
   <222> (323)..(325)
   <223> Xaa can be any naturally occurring amino acid
<220>
   <221> misc_feature
   <222> (327)..(331)
   <223> Xaa can be any naturally occurring amino acid
<220>
   <221> VARIANT
   <222> (334)..(334)
   <223> Xaa can be any naturally occurring amino acid except leucine
<220>
   <221> misc_feature
   <222> (336)..(336)
   <223> Xaa can be any naturally occurring amino acid
<220>
   <221> misc_feature
   <222> (349)..(350)
   <223> Xaa can be any naturally occurring amino acid
<220>
   <221> misc_feature
   <222> (352)..(353)
   <223> Xaa can be any naturally occurring amino acid
<220>
   <221> misc_feature
   <222> (356)..(356)
   <223> Xaa can be any naturally occurring amino acid
<220>
   <221> misc_feature
   <222> (358)..(359)
   <223> Xaa can be any naturally occurring amino acid
<220>
   <221> VARIANT
   <222> (360)..(360)
   <223> Xaa can be any naturally occurring amino acid except aspartate
<220>
   <221> VARIANT
   <222> (361)..(361)
   <223> Xaa can be any naturally occurring amino acid except threonine or isoleucine
<220>
   <221> misc_feature
   <222> (363)..(363)
   <223> Xaa can be any naturally occurring amino acid
<220>
   <221> misc_feature
   <222> (366)..(367)
   <223> Xaa can be any naturally occurring amino acid
<220>
   <221> misc_feature
   <222> (370)..(371)
   <223> Xaa can be any naturally occurring amino acid
<220>
   <221> misc_feature
   <222> (374)..(375)
   <223> Xaa can be any naturally occurring amino acid
<220>
   <221> misc_feature
   <222> (379)..(379)
   <223> Xaa can be any naturally occurring amino acid
<220>
   <221> VARIANT
   <222> (380)..(380)
   <223> Xaa can be any naturally occurring amino acid except methionine
<220>
   <221> misc_feature
   <222> (390)..(390) <223> Xaa can be any naturally occurring amino acid
<220>
   <221> VARIANT
   <222> (395)..(395)
   <223> Xaa can be any naturally occurring amino acid except leucine
<220>
   <221> misc_feature
   <222> (396)..(396)
   <223> Xaa can be any naturally occurring amino acid
<220>
   <221> misc_feature
   <222> (399)..(399)
   <223> Xaa can be any naturally occurring amino acid
<220>
   <221> misc_feature
   <222> (407)..(410)
   <223> Xaa can be any naturally occurring amino acid
<220>
   <221> VARIANT
   <222> (411)..(411)
   <223> Xaa can be any naturally occurring amino acid or absent
<220>
   <221> misc_feature
   <222> (413)..(413)
   <223> Xaa can be any naturally occurring amino acid
<220>
   <221> misc_feature
   <222> (415)..(415)
   <223> Xaa can be any naturally occurring amino acid
<220>
   <221> VARIANT
   <222> (416)..(416)
   <223> Xaa can be any naturally occurring amino acid except leucine
<220>
   <221> misc_feature
   <222> (418)..(419)
   <223> Xaa can be any naturally occurring amino acid
<220>
   <221> misc_feature
   <222> (428)..(428)
   <223> Xaa can be any naturally occurring amino acid
<220>
   <221> VARIANT
   <222> (429)..(429)
   <223> Xaa can be any naturally occurring amino acid except alanine
<220>
   <221> VARIANT
   <222> (430)..(430)
   <223> Xaa can be any naturally occurring amino acid except methionine
<220>
   <221> misc_feature
   <222> (431)..(431)
   <223> Xaa can be any naturally occurring amino acid
<220>
   <221> misc_feature
   <222> (433)..(433)
   <223> Xaa can be any naturally occurring amino acid
<220>
   <221> VARIANT
   <222> (435)..(435)
   <223> Xaa can be any naturally occurring amino acid except lysine
<220>
   <221> misc_feature
   <222> (436)..(436)
   <223> Xaa can be any naturally occurring amino acid
<220>
   <221> misc_feature
   <222> (438)..(441)
   <223> Xaa can be any naturally occurring amino acid
<220>
   <221> VARIANT
   <222> (442)..(442)
   <223> Xaa can be any naturally occurring amino acid except histidine
<220>
   <221> misc feature
   <222> (443)..(446)
   <223> Xaa can be any naturally occurring amino acid
<220>
   <221> misc_feature
   <222> (448)..(449)
   <223> Xaa can be any naturally occurring amino acid
<220>
   <221> VARIANT
   <222> (451)..(451)
   <223> Xaa can be any naturally occurring amino acid except proline
<220>
   <221> VARIANT
   <222> (456).. (456)
   <223> Xaa can be any naturally occurring amino acid except histidine or cysteine
<220> <221> misc_feature
   <222> (457)..(457)
   <223> Xaa can be any naturally occurring amino acid
<220>
   <221> VARIANT
   <222> (461)..(461)
   <223> Xaa can be any naturally occurring amino acid except serine or aspartate
<220>
   <221> misc_feature
   <222> (469)..(470)
   <223> Xaa can be any naturally occurring amino acid
<220>
   <221> VARIANT
   <222> (473)..(473)
   <223> Xaa can be any naturally occurring amino acid except threonine
<220>
   <221> misc_feature
   <222> (477)..(477)
   <223> Xaa can be any naturally occurring amino acid
<220>
   <221> misc_feature
   <222> (481)..(481)
   <223> Xaa can be any naturally occurring amino acid
<220>
   <221> VARIANT
   <222> (484)..(484)
   <223> Xaa can be any naturally occurring amino acid except leucine
<220>
   <221> misc_feature
   <222> (485)..(487)
   <223> Xaa can be any naturally occurring amino acid
<220>
   <221> misc_feature
   <222> (492)..(492)
   <223> Xaa can be any naturally occurring amino acid
<220>
   <221> misc_feature
   <222> (494)..(494)
   <223> Xaa can be any naturally occurring amino acid
<220>
   <221> VARIANT
   <222> (496)..(496)
   <223> Xaa can be any naturally occurring amino acid except phenylalanine
<220>
   <221> VARIANT
   <222> (497)..(497)
   <223> Xaa can be any naturally occurring amino acid except arginine
<220>
   <221> VARIANT
   <222> (498)..(501)
   <223> Xaa can be any naturally occurring amino acid or absent
<220>
   <221> misc_feature
   <222> (502)..(507)
   <223> Xaa can be any naturally occurring amino acid
<220>
   <221> misc_feature
   <222> (509)..(516)
   <223> Xaa can be any naturally occurring amino acid
<220>
   <221> VARIANT
   <222> (517)..(520)
   <223> Xaa can be any naturally occurring amino acid or absent
<220>
   <221> misc_feature
   <222> (521)..(524)
   <223> Xaa can be any naturally occurring amino acid
<220>
   <221> misc_feature
   <222> (526)..(532)
   <223> Xaa can be any naturally occurring amino acid
<220>
   <221> misc_feature
   <222> (534)..(534)
   <223> Xaa can be any naturally occurring amino acid
<220>
   <221> VARIANT
   <222> (535)..(541)
   <223> Xaa can be any naturally occurring amino acid or absent
<400> 15
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Glu Xaa Xaa Pro Xaa
               20                  25                  30
   Xaa Trp Xaa Xaa Xaa Ala Xaa Xaa Xaa Leu Xaa Xaa Ala Xaa Xaa Leu
           35                  40                  45
   Gln Xaa Xaa Xaa Xaa Xaa Xaa Xaa Lys Asn Xaa Ile Xaa Phe Leu Gly
       50                  55                  60
   Asp Gly Xaa Gly Val Xaa Thr Val Thr Ala Xaa Arg Ile Leu Lys Gly
   65                  70                  75                  80
   Gln Xaa Xaa Xaa Xaa Xaa Gly Xaa Glu Xaa Xaa Leu Xaa Met Asp Xaa
                   85                  90                  95
   Phe Pro Xaa Xaa Ala Leu Ser Lys Thr Tyr Xaa Xaa Xaa Xaa Xaa Val
               100                 105                 110
   Pro Asp Ser Xaa Xaa Thr Ala Thr Ala Tyr Leu Cys Gly Val Lys Xaa
           115                 120                 125
   Asn Xaa Xaa Thr Xaa Gly Xaa Ser Ala Ala Xaa Xaa Xaa Xaa Xaa Cys
       130                 135                 140
   Asn Thr Thr Xaa Gly Asn Glu Val Xaa Ser Xaa Xaa Xaa Xaa Ala Lys
   145                 150                 155                 160
   Xaa Xaa Gly Lys Ser Val Gly Xaa Val Thr Thr Thr Arg Val Xaa His
                   165                 170                 175
   Ala Xaa Pro Xaa Xaa Xaa Tyr Ala His Xaa Xaa Xaa Arg Xaa Trp Tyr
               180                 185                 190
   Ser Asp Xaa Xaa Xaa Pro Xaa Xaa Ala Xaa Xaa Xaa Gly Cys Xaa Asp
           195                 200                 205
   Ile Ala Xaa Gln Leu Xaa Xaa Asn Xaa Xaa Asp Ile Xaa Val Ile Xaa
       210                 215                 220
   Gly Gly Gly Arg Lys Tyr Met Xaa Xaa Xaa Xaa Xaa Xaa Asp Xaa Glu
   225                 230                 235                 240
   Tyr Xaa Xaa Asp Xaa Xaa Xaa Xaa Gly Xaa Arg Leu Asp Gly Xaa Xaa
                   245                 250                 255
   Leu Xaa Xaa Xaa Trp Xaa Xaa Xaa Xaa Xaa Xaa Xaa Xaa Xaa Xaa Xaa
               260                 265                 270
   Xaa Xaa Trp Asn Arg Thr Xaa Leu Xaa Xaa Xaa Xaa Xaa Xaa Xaa Xaa
   275 280 285
   Val Xaa Xaa Leu Xaa Gly Leu Phe Xaa Pro Gly Asp Xaa Xaa Tyr Glu
   290 295 300
   Xaa Xaa Arg Xaa Xaa Xaa Xaa Asp Pro Ser Leu Xaa Glu Met Xaa Xaa
   305 310 315 320
   Xaa Ala Xaa Xaa Xaa Leu Xaa Xaa Xaa Xaa Xaa Gly Phe Xaa Leu Xaa
   325 330 335
   Val Glu Gly Gly Arg Ile Asp His Gly His His Glu Xaa Xaa Ala Xaa
   340 345 350
   Xaa Ala Leu Xaa Glu Xaa Xaa Xaa Xaa Asp Xaa Ala Ile Xaa Xaa Ala
   355 360 365
   Gly Xaa Xaa Thr Ser Xaa Xaa Asp Thr Leu Xaa Xaa Val Thr Ala Asp
   370 375 380
   His Ser His Val Phe Xaa Phe Gly Gly Tyr Xaa Xaa Arg Gly Xaa Ser
   385 390 395 400
   Ile Phe Gly Leu Ala Pro Xaa Xaa Xaa Xaa Xaa Asp Xaa Lys Xaa Xaa
   405 410 415
   Thr Xaa Xaa Leu Tyr Gly Asn Gly Pro Gly Tyr Xaa Xaa Xaa Xaa Gly
   420 425 430
   Xaa Arg Xaa Xaa Val Xaa Xaa Xaa Xaa Xaa Xaa Xaa Xaa Xaa Tyr Xaa
   435 440 445
   Xaa Gln Xaa Ala Val Pro Leu Xaa Xaa Glu Thr His Xaa Gly Glu Asp
   450 455 460
   Val Ala Val Phe Xaa Xaa Gly Pro Xaa Ala His Leu Xaa His Gly Val
   465 470 475 480
   Xaa Glu Gln Xaa Xaa Xaa Xaa His Val Met Ala Xaa Ala Xaa Cys Xaa
   485 490 495
   Xaa Xaa Xaa Xaa Xaa Xaa Xaa Xaa Xaa Xaa Xaa Ala Xaa Xaa Xaa Xaa
   500 505 510
   Xaa Xaa Xaa Xaa Xaa Xaa Xaa Xaa Xaa Xaa Xaa Xaa Leu Xaa Xaa Xaa
   515 520 525
   Xaa Xaa Xaa Xaa Leu Xaa Xaa Xaa Xaa Xaa Xaa Xaa Xaa
   530 535 540
<210> 16
   <211> 524
   <212> PRT
   <213> Artificial
<220>
   <223> Consensus TNALP from various mammalian species (with signal peptide and GPI anchor domain)
<220>
   <221> misc_feature
   <222> (3)..(3)
   <223> Xaa can be any naturally occurring amino acid
<220>
   <221> misc_feature
   <222> (7)..(7)
   <223> Xaa can be any naturally occurring amino acid
<220>
   <221> misc_feature
   <222> (14) ..(16)
   <223> Xaa can be any naturally occurring amino acid
<220>
   <221> misc_feature
   <222> (18)..(18)
   <223> Xaa can be any naturally occurring amino acid
<220>
   <221> misc_feature
   <222> (24)..(24)
   <223> Xaa can be any naturally occurring amino acid
<220>
   <221> misc_feature
   <222> (27)..(27)
   <223> Xaa can be any naturally occurring amino acid
<220>
   <221> misc_feature
   <222> (31)..(31)
   <223> Xaa can be any naturally occurring amino acid
<220> <221> misc_feature
   <222> (35)..(35)
   <223> Xaa can be any naturally occurring amino acid
<220>
   <221> misc_feature
   <222> (39)..(39)
   <223> Xaa can be any naturally occurring amino acid
<220>
   <221> misc feature
   <222> (42)..(42)
   <223> Xaa can be any naturally occurring amino acid
<220>
   <221> misc_feature
   <222> (45)..(45)
   <223> Xaa can be any naturally occurring amino acid
<220>
   <221> VARIANT
   <222> (51)..(51)
   <223> Xaa can be any naturally occurring amino acid except serine or valine
<220>
   <221> misc feature
   <222> (54)..(54)
   <223> Xaa can be any naturally occurring amino acid
<220>
   <221> misc_feature
   <222> (70)..(70)
   <223> Xaa can be any naturally occurring amino acid
<220>
   <221> misc_feature
   <222> (80)..(81)
   <223> Xaa can be any naturally occurring amino acid
<220>
   <221> misc feature
   <222> (86)..(86)
   <223> Xaa can be any naturally occurring amino acid
<220>
   <221> misc feature
   <222> (94)..(94)
   <223> Xaa can be any naturally occurring amino acid
<220>
   <221> misc_feature
   <222> (135)..(135)
   <223> Xaa can be any naturally occurring amino acid
<220>
   <221> misc_feature <222> (137)..(138)
   <223> Xaa can be any naturally occurring amino acid
<220>
   <221> misc_feature
   <222> (157)..(157)
   <223> Xaa can be any naturally occurring amino acid
<220>
   <221> VARIANT
   <222> (177)..(177)
   <223> Xaa can be any naturally occurring amino acid except threonine
<220>
   <221> misc_feature
   <222> (210)..(210)
   <223> Xaa can be any naturally occurring amino acid
<220>
   <221> VARIANT
   <222> (212)..(212)
   <223> Xaa can be any naturally occurring amino acid except phenylalanine
<220>
   <221> misc_feature
   <222> (213)..(213)
   <223> Xaa can be any naturally occurring amino acid
<220>
   <221> misc_feature
   <222> (216)..(216)
   <223> Xaa can be any naturally occurring amino acid
<220>
   <221> misc_feature
   <222> (227)..(227)
   <223> Xaa can be any naturally occurring amino acid
<220>
   <221> misc feature
   <222> (231)..(231)
   <223> Xaa can be any naturally occurring amino acid
<220>
   <221> misc_feature
   <222> (238)..(238)
   <223> Xaa can be any naturally occurring amino acid
<220>
   <221> misc feature
   <222> (242)..(243)
   <223> Xaa can be any naturally occurring amino acid
<220>
   <221> misc_feature
   <222> (245)..(245)
   <223> Xaa can be any naturally occurring amino acid
<220>
   <221> misc_feature
   <222> (251)..(251)
   <223> Xaa can be any naturally occurring amino acid
<220>
   <221> misc_feature
   <222> (253)..(255)
   <223> Xaa can be any naturally occurring amino acid
<220>
   <221> misc_feature
   <222> (258)..(258)
   <223> Xaa can be any naturally occurring amino acid
<220>
   <221> misc_feature
   <222> (262)..(263)
   <223> Xaa can be any naturally occurring amino acid
<220>
   <221> misc_feature
   <222> (268)..(269)
   <223> Xaa can be any naturally occurring amino acid
<220>
   <221> misc_feature
   <222> (274)..(274)
   <223> Xaa can be any naturally occurring amino acid
<220>
   <221> misc_feature
   <222> (277)..(277)
   <223> Xaa can be any naturally occurring amino acid
<220>
   <221> misc_feature
   <222> (281)..(282)
   <223> Xaa can be any naturally occurring amino acid
<220>
   <221> VARIANT
   <222> (291)..(291)
   <223> Xaa can be any naturally occurring amino acid except lysine
<220>
   <221> misc_feature
   <222> (303)..(304)
   <223> Xaa can be any naturally occurring amino acid
<220>
   <221> misc_feature
   <222> (314)..(315)
   <223> Xaa can be any naturally occurring amino acid
<220>
   <221> misc_feature
   <222> (317)..(318)
   <223> Xaa can be any naturally occurring amino acid
<220>
   <221> misc_feature
   <222> (321)..(321)
   <223> Xaa can be any naturally occurring amino acid
<220>
   <221> misc_feature
   <222> (323)..(325)
   <223> Xaa can be any naturally occurring amino acid
<220>
   <221> misc_feature
   <222> (357)..(357)
   <223> Xaa can be any naturally occurring amino acid
<220>
   <221> misc_feature
   <222> (361)..(361)
   <223> Xaa can be any naturally occurring amino acid
<220>
   <221> misc_feature
   <222> (364)..(365)
   <223> Xaa can be any naturally occurring amino acid
<220>
   <221> misc_feature
   <222> (368)..(369)
   <223> Xaa can be any naturally occurring amino acid
<220>
   <221> VARIANT
   <222> (374)..(374)
   <223> Xaa can be any naturally occurring amino acid except methionine
<220>
   <221> misc_feature
   <222> (402)..(402)
   <223> Xaa can be any naturally occurring amino acid
<220>
   <221> misc_feature
   <222> (412)..(412)
   <223> Xaa can be any naturally occurring amino acid
<220>
   <221> misc_feature
   <222> (425)..(425)
   <223> Xaa can be any naturally occurring amino acid
<220>
   <221> misc_feature
   <222> (463)..(463)
   <223> Xaa can be any naturally occurring amino acid
<220>
   <221> misc_feature
   <222> (475)..(475)
   <223> Xaa can be any naturally occurring amino acid
<220>
   <221> misc_feature
   <222> (480)..(480)
   <223> Xaa can be any naturally occurring amino acid
<220>
   <221> misc_feature
   <222> (488)..(488)
   <223> Xaa can be any naturally occurring amino acid
<220>
   <221> misc_feature
   <222> (494)..(495)
   <223> Xaa can be any naturally occurring amino acid
<220>
   <221> misc_feature
   <222> (499)..(499)
   <223> Xaa can be any naturally occurring amino acid
<220>
   <221> misc feature
   <222> (501)..(501)
   <223> Xaa can be any naturally occurring amino acid <220>
   <221> misc_feature
   <222> (503)..(508)
   <223> Xaa can be any naturally occurring amino acid
<220>
   <221> misc_feature
   <222> (510)..(510)
   <223> Xaa can be any naturally occurring amino acid
<220>
   <221> misc feature
   <222> (512)..(512)
   <223> Xaa can be any naturally occurring amino acid
<220>
   <221> misc_feature
   <222> (514)..(514)
   <223> Xaa can be any naturally occurring amino acid
<220>
   <221> misc_feature
   <222> (517)..(522)
   <223> Xaa can be any naturally occurring amino acid
<400> 16
   Met Ile Xaa Pro Phe Leu Xaa Leu Ala Ile Gly Thr Cys Xaa Xaa Xaa
   1               5                   10                  15
   Ser Xaa Val Pro Glu Lys Glu Xaa Asp Pro Xaa Tyr Trp Arg Xaa Gln
               20                  25                  30
   Ala Gln Xaa Thr Leu Lys Xaa Ala Leu Xaa Leu Gln Xaa Leu Asn Thr
           35                  40                  45
   Asn Val Xaa Lys Asn Xaa Ile Met Phe Leu Gly Asp Gly Met Gly Val
       50                  55                  60
   Ser Thr Val Thr Ala Xaa Arg Ile Leu Lys Gly Gln Leu His His Xaa
   65                  70                  75                  80
   Xaa Gly Glu Glu Thr Xaa Leu Glu Met Asp Lys Phe Pro Xaa Val Ala
                   85                  90                  95
   Leu Ser Lys Thr Tyr Asn Thr Asn Ala Gln Val Pro Asp Ser Ala Gly
               100                 105                 110
   Thr Ala Thr Ala Tyr Leu Cys Gly Val Lys Ala Asn Glu Gly Thr Val
           115                 120                 125
   Gly Val Ser Ala Ala Thr Xaa Arg Xaa Xaa Cys Asn Thr Thr Gln Gly
       130                 135                 140
   Asn Glu Val Thr Ser Ile Leu Arg Trp Ala Lys Asp Xaa Gly Lys Ser
   145                 150                 155                 160
   Val Gly Ile Val Thr Thr Thr Arg Val Asn His Ala Thr Pro Ser Ala
                   165                 170                 175
   Xaa Tyr Ala His Ser Ala Asp Arg Asp Trp Tyr Ser Asp Asn Glu Met
               180                 185                 190
   Pro Pro Glu Ala Leu Ser Gln Gly Cys Lys Asp Ile Ala Tyr Gln Leu
           195                 200                 205
   Met Xaa Asn Xaa Xaa Asp Ile Xaa Val Ile Met Gly Gly Gly Arg Lys
       210                 215                 220
   Tyr Met Xaa Pro Lys Asn Xaa Thr Asp Val Glu Tyr Glu Xaa Asp Glu
   225 230 235 240
   Lys Xaa Xaa Gly Xaa Arg Leu Asp Gly Leu Xaa Leu Xaa Xaa Xaa Trp
   245 250 255
   Lys Xaa Phe Lys Pro Xaa Xaa Lys His Ser His Xaa Xaa Trp Asn Arg
   260 265 270
   Thr Xaa Leu Leu Xaa Leu Asp Pro Xaa Xaa Val Asp Tyr Leu Leu Gly
   275 280 285
   Leu Phe Xaa Pro Gly Asp Met Gln Tyr Glu Leu Asn Arg Asn Xaa Xaa
   290 295 300
   Thr Asp Pro Ser Leu Ser Glu Met Val Xaa Xaa Ala Xaa Xaa Ile Leu
   305 310 315 320
   Xaa Lys Xaa Xaa Xaa Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Xaa Ala Ile Gly Xaa Ala Gly Xaa Xaa Thr Ser Xaa
   355 360 365
   Xaa Asp Thr Leu Thr Xaa Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Xaa Ser Asp Thr Asp Lys Lys Pro Phe Thr Xaa Ile Leu Tyr Gly
   405 410 415
   Asn Gly Pro Gly Tyr Lys Val Val Xaa Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Xaa Lys
   450 455 460
   Gly Pro Met Ala His Leu Leu His Gly Val Xaa Glu Gln Asn Tyr Xaa
   465 470 475 480
   Pro His Val Met Ala Tyr Ala Xaa Cys Ile Gly Ala Asn Xaa Xaa His
   485 490 495
   Cys Ala Xaa Ala Xaa Ser Xaa Xaa Xaa Xaa Xaa Xaa Gly Xaa Leu Xaa
   500 505 510
   Leu Xaa Leu Ala Xaa Xaa Xaa Xaa Xaa Xaa Leu Phe
   515 520
<210> 17
   <211> 2232
   <212> DNA
   <213> Artificial
<220>
   <223> hsTNALP-FcD10
<400> 17
   atggtttcac cattcttagt actggccatt ggcacctgcc ttactaactc cttagtgcca 60
   gagaaagaga aagaccccaa gtactggcga gaccaagcgc aagagacact gaaatatgcc 120
   ctggagcttc agaagctcaa caccaacgtg gctaagaatg tcatcatgtt cctgggagat 180
   gggatgggtg tctccacagt gacggctgcc cgcatcctca agggtcagct ccaccacaac 240
   cctggggagg agaccaggct ggagatggac aagttcccct tcgtggccct ctccaagacg 300
   tacaacacca atgcccaggt ccctgacagc gccggcaccg ccaccgccta cctgtgtggg 360
   gtgaaggcca atgagggcac cgtgggggta agcgcagcca ctgagcgttc ccggtgcaac 420
   accacccagg ggaacgaggt cacctccatc ctgcgctggg ccaaggacgc tgggaaatct 480
   gtgggcattg tgaccaccac gagagtgaac catgccaccc ccagcgccgc ctacgcccac 540
   tcggctgacc gggactggta ctcagacaac gagatgcccc ctgaggcctt gagccagggc 600
   tgtaaggaca tcgcctacca gctcatgcat aacatcaggg acattgacgt gatcatgggg 660
   ggtggccgga aatacatgta ccccaagaat aaaactgatg tggagtatga gagtgacgag 720
   aaagccaggg gcacgaggct ggacggcctg gacctcgttg acacctggaa gagcttcaaa 780
   ccgagataca agcactccca cttcatctgg aaccgcacgg aactcctgac ccttgacccc 840
   cacaatgtgg actacctatt gggtctcttc gagccagggg acatgcagta cgagctgaac     900
   aggaacaacg tgacggaccc gtcactctcc gagatggtgg tggtggccat ccagatcctg     960
   cggaagaacc ccaaaggctt cttcttgctg gtggaaggag gcagaattga ccacgggcac    1020
   catgaaggaa aagccaagca ggccctgcat gaggcggtgg agatggaccg ggccatcggg    1080
   caggcaggca gcttgacctc ctcggaagac actctgaccg tggtcactgc ggaccattcc    1140
   cacgtcttca catttggtgg atacaccccc cgtggcaact ctatctttgg tctggccccc    1200
   atgctgagtg acacagacaa gaagcccttc actgccatcc tgtatggcaa tgggcctggc    1260
   tacaaggtgg tgggcggtga acgagagaat gtctccatgg tggactatgc tcacaacaac    1320
   taccaggcgc agtctgctgt gcccctgcgc cacgagaccc acggcgggga ggacgtggcc    1380
   gtcttctcca agggccccat ggcgcacctg ctgcacggcg tccacgagca gaactacgtc    1440
   ccccacgtga tggcgtatgc agcctgcatc ggggccaacc tcggccactg tgctcctgcc    1500
   agctcgctta aggacaaaac tcacacatgc ccaccgtgcc cagcacctga actcctgggg    1560
   ggaccgtcag tcttcctctt ccccccaaaa cccaaggaca ccctcatgat ctcccggacc    1620
   cctgaggtca catgcgtggt ggtggacgtg agccacgaag accctgaggt caagttcaac    1680
   tggtacgtgg acggcgtgga ggtgcataat gccaagacaa agccgcggga ggagcagtac    1740
   aacagcacgt accgtgtggt cagcgtcctc accgtcctgc accaggactg gctgaatggc    1800
   aaggagtaca agtgcaaggt ctccaacaaa gccctcccag cccccatcga gaaaaccatc    1860
   tccaaagcca aagggcagcc ccgagaacca caggtgtaca ccctgccccc atcccgggag    1920
   gagatgacca agaaccaggt cagcctgacc tgcctggtca aaggcttcta tcccagcgac    1980
   atcgccgtgg agtgggagag caatgggcag ccggagaaca actacaagac cacgcctccc    2040
   gtgctggact ccgacggctc cttcttcctc tacagcaagc tcaccgtgga caagagcagg    2100
   tggcagcagg ggaacgtctt ctcatgctcc gtgatgcatg aggctctgca caaccactac    2160
   acgcagaaga gcctctccct gtctccgggt aaagatatcg atgacgatga cgatgacgat    2220
   gacgatgact ag                                                        2232
<210> 18
   <211> 541
   <212> PRT
   <213> Artificial
<220>
   <223> Consensus ALP: TNALP from various mammalian species and human ALP isozymes PLAP, GCALP, IALP (with signal peptide and GPI anchor domain)
<220>
   <221> misc_feature
   <222> (1)..(4)
   <223> Xaa can be any naturally occurring amino acid
<220>
   <221> VARIANT
   <222> (5)..(9)
   <223> Xaa can be any naturally occurring amino acid or absent
<220>
   <221> misc feature
   <222> (10)..(21)
   <223> Xaa can be any naturally occurring amino acid
<220>
   <221> VARIANT
   <222> (22).. (22)
   <223> Xaa is a serine or a glycine
<220>
   <221> misc_feature
   <222> (23)..(27)
   <223> Xaa can be any naturally occurring amino acid
<220>
   <221> misc_feature
   <222> (29)..(30)
   <223> Xaa can be any naturally occurring amino acid
<220>
   <221> misc_feature
   <222> (32)..(32)
   <223> Xaa can be any naturally occurring amino acid
<220>
   <221> VARIANT
   <222> (33)..(33)
   <223> Xaa is a tyrosine or a phenylalanine
<220>
   <221> misc_feature
   <222> (35)..(37)
   <223> Xaa can be any naturally occurring amino acid
<220>
   <221> misc_feature
   <222> (39).. (41)
   <223> Xaa can be any naturally occurring amino acid
<220>
   <221> misc_feature
   <222> (43)..(44)
<223> Xaa can be any naturally occurring amino acid
<220>
   <221> misc_feature
   <222> (46).. (47)
   <223> Xaa can be any naturally occurring amino acid
<220>
   <221> misc_feature
   <222> (50).. (53)
   <223> Xaa can be any naturally occurring amino acid
<220>
   <221> VARIANT
   <222> (54)..(54)
   <223> Xaa can be any naturally occurring amino acid or absent
<220>
   <221> misc_feature
   <222> (55)..(55)
   <223> Xaa can be any naturally occurring amino acid
<220>
   <221> misc feature
   <222> (59)..(59)
   <223> Xaa can be any naturally occurring amino acid
<220>
   <221> misc_feature
   <222> (61).. (61)
   <223> Xaa can be any naturally occurring amino acid
<220>
   <221> misc feature
   <222> (70) ..(70)
   <223> Xaa can be any naturally occurring amino acid
<220>
   <221> misc feature
   <222> (75)..(75)
   <223> Xaa can be any naturally occurring amino acid
<220>
   <221> misc_feature
   <222> (82)..(86)
   <223> Xaa can be any naturally occurring amino acid
<220>
   <221> misc_feature
   <222> (88)..(88)
   <223> Xaa can be any naturally occurring amino acid
<220>
   <221> misc_feature
   <222> (90)..(91)
   <223> Xaa can be any naturally occurring amino acid
<220>
   <221> misc_feature
   <222> (93).. (93)
   <223> Xaa can be any naturally occurring amino acid
<220>
   <221> misc_feature
   <222> (96).. (96)
   <223> Xaa can be any naturally occurring amino acid
<220>
   <221> misc feature
   <222> (99)..(100)
   <223> Xaa can be any naturally occurring amino acid
<220>
   <221> misc_feature
   <222> (107)..(111)
   <223> Xaa can be any naturally occurring amino acid
<220>
   <221> VARIANT
   <222> (116)..(117)
   <223> Xaa is an alanine or a glycine
<220>
   <221> VARIANT
   <222> (128)..(128)
   <223> Xaa is an alanine or a glycine
<220>
   <221> misc_feature
   <222> (130)..(131)
   <223> Xaa can be any naturally occurring amino acid
<220>
   <221> VARIANT
   <222> (133)..(133)
   <223> Xaa is a valine or an isoleucine
<220>
   <221> misc_feature
   <222> (135)..(135)
   <223> Xaa can be any naturally occurring amino acid
<220>
   <221> VARIANT
   <222> (139)..(139)
   <223> Xaa is a threonine or an alanine
<220>
   <221> misc_feature
   <222> (140)..(140)
   <223> Xaa can be any naturally occurring amino acid
<220>
   <221> VARIANT
   <222> (141)..(141)
   <223> Xaa is an arginine or a phenylalanine
<220>
   <221> misc_feature
   <222> (142)..(143)
   <223> Xaa can be any naturally occurring amino acid
<220>
   <221> misc_feature
   <222> (148)..(148)
   <223> Xaa can be any naturally occurring amino acid
<220>
   <221> misc feature
   <222> (155)..(158)
   <223> Xaa can be any naturally occurring amino acid
<220>
   <221> misc_feature
   <222> (161)..(162)
   <223> Xaa can be any naturally occurring amino acid
<220>
   <221> misc_feature
   <222> (168)..(168)
   <223> Xaa can be any naturally occurring amino acid
<220>
   <221> VARIANT
   <222> (175)..(175)
   <223> Xaa is an asparagine or a glutamine
<220>
   <221> misc_feature
   <222> (178)..(178)
   <223> Xaa can be any naturally occurring amino acid
<220>
   <221> misc_feature
   <222> (180)..(180)
   <223> Xaa can be any naturally occurring amino acid
<220>
   <221> VARIANT
   <222> (181)..(181)
   <223> Xaa is a alanine or a glycine
<220>
   <221> VARIANT
   <222> (182)..(182)
   <223> Xaa is an alanine, a serine or a threonine
<220>
   <221> VARIANT
   <222> (186)..(186)
   <223> Xaa is a serine or a threonine
<220>
   <221> misc_feature

   <222> (187)..(188)
   <223> Xaa can be any naturally occurring amino acid
<220>
   <221> misc_feature
   <222> (190)..(190)
   <223> Xaa can be any naturally occurring amino acid
<220>
   <221> misc_feature
   <222> (195)..(195)
   <223> Xaa can be any naturally occurring amino acid
<220>
   <221> VARIANT
   <222> (196)..(196)
   <223> Xaa is a glutamate or an aspartate
<220>
   <221> VARIANT
   <222> (197)..(197)
   <223> Xaa is a methionine or a valine
<220>
   <221> misc_feature
   <222> (199)..(200)
   <223> Xaa can be any naturally occurring amino acid
<220>
   <221> misc_feature
   <222> (202)..(204)
   <223> Xaa can be any naturally occurring amino acid
<220>
   <221> misc_feature
   <222> (207)..(207)
   <223> Xaa can be any naturally occurring amino acid
<220>
   <221> misc_feature
   <222> (211)..(211)
   <223> Xaa can be any naturally occurring amino acid
<220>
   <221> misc_feature
   <222> (214)..(215)
   <223> Xaa can be any naturally occurring amino acid
<220>
   <221> VARIANT
   <222> (217)..(217)
   <223> Xaa is an isoleucine, a valine or a methionine
<220> <221> VARIANT
   <222> (218)..(218)
   <223> Xaa can be any naturally occurring amino acid or absent
<220>
   <221> misc_feature
   <222> (221)..(221)
   <223> Xaa can be any naturally occurring amino acid
<220>
   <221> misc_feature
   <222> (224)..(224)
   <223> Xaa can be any naturally occurring amino acid
<220>
   <221> misc_feature
   <222> (232)..(237)
   <223> Xaa can be any naturally occurring amino acid
<220>
   <221> misc_feature
   <222> (239)..(239)
   <223> Xaa can be any naturally occurring amino acid
<220>
   <221> VARIANT
   <222> (240)..(240)
   <223> Xaa is a valine or a proline
<220>
   <221> misc_feature
   <222> (242)..(243)
   <223> Xaa can be any naturally occurring amino acid
<220>
   <221> misc_feature
   <222> (245)..(248)
   <223> Xaa can be any naturally occurring amino acid
<220>
   <221> misc_feature
   <222> (250) .. (250)
   <223> Xaa can be any naturally occurring amino acid <220>
   <221> misc_feature
   <222> (255)..(256)
   <223> Xaa can be any naturally occurring amino acid
<220>
   <221> misc_feature
   <222> (258)..(260)
   <223> Xaa can be any naturally occurring amino acid
<220>
   <221> misc_feature
   <222> (262)..(262) <223> Xaa can be any naturally occurring amino acid
<220>
   <221> VARIANT
   <222> (263)..(265)
   <223> Xaa can be any naturally occurring amino acid or absent
<220>
   <221> misc_feature
   <222> (266)..(268)
   <223> Xaa can be any naturally occurring amino acid
<220>
   <221> VARIANT
   <222> (269)..(269)
   <223> Xaa is a lysine or a glutamine
<220>
   <221> misc_feature
   <222> (270)..(274)
   <223> Xaa can be any naturally occurring amino acid
<220>
   <221> misc_feature
   <222> (279)..(279)
   <223> Xaa can be any naturally occurring amino acid
<220>
   <221> misc_feature
   <222> (281)..(286)
   <223> Xaa can be any naturally occurring amino acid
<220>
   <221> VARIANT
   <222> (287)..(287)
   <223> Xaa can be any naturally occurring amino acid or absent
<220>
   <221> misc_feature
   <222> (288)..(288)
   <223> Xaa can be any naturally occurring amino acid
<220>
   <221> misc_feature
   <222> (290)..(291)
   <223> Xaa can be any naturally occurring amino acid
<220>
   <221> misc_feature
   <222> (293)..(293)
   <223> Xaa can be any naturally occurring amino acid
<220>
   <221> VARIANT
   <222> (297)..(297)
   <223> Xaa is a glutamate or an aspartate
<220>
   <221> misc_feature
   <222> (302)..(302)
   <223> Xaa can be any naturally occurring amino acid
<220>
   <221> VARIANT
   <222> (305)..(305)
   <223> Xaa is a leucine or an isoleucine
<220>
   <221> misc_feature
   <222> (306)..(306)
   <223> Xaa can be any naturally occurring amino acid
<220>
   <221> misc_feature
   <222> (308)..(311)
   <223> Xaa can be any naturally occurring amino acid
<220>
   <221> misc_feature
   <222> (316)..(316)
   <223> Xaa can be any naturally occurring amino acid
<220>
   <221> misc_feature
   <222> (319)..(321)
   <223> Xaa can be any naturally occurring amino acid
<220>
   <221> misc_feature
   <222> (323)..(325)
   <223> Xaa can be any naturally occurring amino acid
<220>
   <221> misc_feature
   <222> (327)..(331)
   <223> Xaa can be any naturally occurring amino acid
<220>
   <221> VARIANT
   <222> (334)..(334)
   <223> Xaa is a phenylalanine or a tyrosine
<220>
   <221> misc_feature
   <222> (336)..(336)
   <223> Xaa can be any naturally occurring amino acid
<220>
   <221> misc_feature
   <222> (349)..(350)
   <223> Xaa can be any naturally occurring amino acid
<220>
   <221> misc_feature <222> (352)..(353)
   <223> Xaa can be any naturally occurring amino acid
<220>
   <221> misc_feature
   <222> (356)..(356)
   <223> Xaa can be any naturally occurring amino acid
<220>
   <221> misc_feature
   <222> (358)..(359)
   <223> Xaa can be any naturally occurring amino acid
<220>
   <221> VARIANT
   <222> (360)..(360)
   <223> Xaa is a glutamate or a methionine
<220>
   <221> VARIANT
   <222> (361)..(361)
   <223> Xaa is a methionine or a phenylalanine
<220>
   <221> misc_feature
   <222> (363)..(363)
   <223> Xaa can be any naturally occurring amino acid
<220>
   <221> misc_feature
   <222> (366)..(367)
   <223> Xaa can be any naturally occurring amino acid
<220>
   <221> misc_feature
   <222> (370)..(371)
   <223> Xaa can be any naturally occurring amino acid
<220>
   <221> misc_feature
   <222> (374)..(375)
   <223> Xaa can be any naturally occurring amino acid
<220>
   <221> misc_feature
   <222> (379)..(379)
   <223> Xaa can be any naturally occurring amino acid
<220>
   <221> VARIANT
   <222> (380)..(380)
   <223> Xaa is a valine, an isoleucine or a leucine
<220>
   <221> misc_feature
   <222> (390)..(390)
   <223> Xaa can be any naturally occurring amino acid
<220>
   <221> VARIANT
   <222> (395)..(395)
   <223> Xaa is a threonine or a proline
<220>
   <221> misc_feature
   <222> (396)..(396)
   <223> Xaa can be any naturally occurring amino acid
<220>
   <221> misc_feature
   <222> (399)..(399)
   <223> Xaa can be any naturally occurring amino acid <220>
   <221> misc_feature
   <222> (407)..(410)
   <223> Xaa can be any naturally occurring amino acid
<220>
   <221> VARIANT
   <222> (411)..(411)
   <223> Xaa can be any naturally occurring amino acid or absent
<220>
   <221> misc_feature
   <222> (413)..(413)
   <223> Xaa can be any naturally occurring amino acid
<220>
   <221> misc_feature
   <222> (415)..(415)
   <223> Xaa can be any naturally occurring amino acid
<220>
   <221> VARIANT
   <222> (416)..(416)
   <223> Xaa is a phenylalanine or a tyrosine
<220>
   <221> misc_feature
   <222> (418)..(419)
   <223> Xaa can be any naturally occurring amino acid
<220>
   <221> misc_feature
   <222> (428)..(428)
   <223> Xaa can be any naturally occurring amino acid
<220>
   <221> VARIANT
   <222> (429)..(429)
   <223> Xaa is a valine, a leucine or a phenylalanine
<220>
   <221> VARIANT
   <222> (430)..(430)
   <223> Xaa is a valine, a lysine or an asparagine
<220>
   <221> misc_feature
   <222> (431)..(431)
   <223> Xaa can be any naturally occurring amino acid
<220>
   <221> misc_feature
   <222> (433)..(433)
   <223> Xaa can be any naturally occurring amino acid
<220>
   <221> VARIANT
   <222> (435)..(435)
   <223> Xaa is a glutamate or a proline
<220>
   <221> misc_feature
   <222> (436)..(436)
   <223> Xaa can be any naturally occurring amino acid
<220>
   <221> misc_feature
   <222> (438)..(441)
   <223> Xaa can be any naturally occurring amino acid
<220>
   <221> VARIANT
   <222> (442)..(442)
   <223> Xaa is a tyrosine or a serine
<220>
   <221> misc_feature
   <222> (443)..(446)
   <223> Xaa can be any naturally occurring amino acid
<220>
   <221> misc_feature
   <222> (448)..(449)
   <223> Xaa can be any naturally occurring amino acid
<220>
   <221> VARIANT
   <222> (451)..(451)
   <223> Xaa is a serine or an alanine
<220>
   <221> VARIANT
   <222> (456)..(456)
   <223> Xaa is a arginine, an aspartate or a serine
<220>
   <221> VARIANT
   <222> (456)..(456) <223> Xaa is an arginine, an aspartate or a serine
<220>
   <221> misc_feature
   <222> (457)..(457)
   <223> Xaa can be any naturally occurring amino acid
<220>
   <221> VARIANT
   <222> (461)..(461)
   <223> Xaa is a glycine or an alanine
<220>
   <221> misc_feature
   <222> (469)..(470)
   <223> Xaa can be any naturally occurring amino acid
<220>
   <221> VARIANT
   <222> (473)..(473)
   <223> Xaa is a methionine or a glutamine
<220>
   <221> misc_feature
   <222> (477)..(477)
   <223> Xaa can be any naturally occurring amino acid
<220>
   <221> misc_feature
   <222> (481)..(481)
   <223> Xaa can be any naturally occurring amino acid
<220>
   <221> VARIANT
   <222> (484)..(484)
   <223> Xaa is a asparagine, a threonine or a serine
<220>
   <221> misc_feature
   <222> (485)..(487)
   <223> Xaa can be any naturally occurring amino acid
<220>
   <221> misc_feature
   <222> (492)..(492)
   <223> Xaa can be any naturally occurring amino acid
<220>
   <221> misc_feature
   <222> (494)..(494)
   <223> Xaa can be any naturally occurring amino acid
<220>
   <221> VARIANT
   <222> (496)..(496)
   <223> Xaa is an isoleucine or a leucine
<220>
   <221> VARIANT
   <222> (497)..(497)
   <223> Xaa is a glycine or a glutamate
<220>
   <221> VARIANT
   <222> (498)..(501)
   <223> Xaa can be any naturally occurring amino acid or absent
<220>
   <221> misc_feature
   <222> (502)..(507)
   <223> Xaa can be any naturally occurring amino acid
<220>
   <221> misc_feature
   <222> (509)..(516)
   <223> Xaa can be any naturally occurring amino acid
<220>
   <221> VARIANT
   <222> (517)..(520)
   <223> Xaa can be any naturally occurring amino acid or absent
<220>
   <221> misc_feature
   <222> (521)..(524)
   <223> Xaa can be any naturally occurring amino acid
<220>
   <221> misc_feature
   <222> (526)..(532)
   <223> Xaa can be any naturally occurring amino acid
<220>
   <221> misc_feature
   <222> (534)..(534)
   <223> Xaa can be any naturally occurring amino acid
<220>
   <221> VARIANT
   <222> (535)..(540)
   <223> Xaa can be any naturally occurring amino acid or absent
<220>
   <221> misc_feature
   <222> (541)..(541)
   <223> Xaa can be any naturally occurring amino acid
<400> 18
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Glu Xaa Xaa Pro Xaa
   20 25 30
   Xaa Trp Xaa Xaa Xaa Ala Xaa Xaa Xaa Leu Xaa Xaa Ala Xaa Xaa Leu
   35 40 45
   Gln Xaa Xaa Xaa Xaa Xaa Xaa Ala Lys Asn Xaa Ile Xaa Phe Leu Gly
   50 55 60
   Asp Gly Met Gly Val Xaa Thr Val Thr Ala Xaa Arg Ile Leu Lys Gly
   65 70 75 80
   Gln Xaa Xaa Xaa Xaa Xaa Gly Xaa Glu Xaa Xaa Leu Xaa Met Asp Xaa
   85 90 95
   Phe Pro Xaa Xaa Ala Leu Ser Lys Thr Tyr Xaa Xaa Xaa Xaa Xaa Val
   100 105 110
   Pro Asp Ser Xaa Xaa Thr Ala Thr Ala Tyr Leu Cys Gly Val Lys Xaa
   115 120 125
   Asn Xaa Xaa Thr Xaa Gly Xaa Ser Ala Ala Xaa Xaa Xaa Xaa Xaa Cys
   130 135 140
   Asn Thr Thr Xaa Gly Asn Glu Val Thr Ser Xaa Xaa Xaa Xaa Ala Lys
   145 150 155 160
   Xaa Xaa Gly Lys Ser Val Gly Xaa Val Thr Thr Thr Arg Val Xaa His
   165 170 175
   Ala Xaa Pro Xaa Xaa Xaa Tyr Ala His Xaa Xaa Xaa Arg Xaa Trp Tyr
   180 185 190
   Ser Asp Xaa Xaa Xaa Pro Xaa Xaa Ala Xaa Xaa Xaa Gly Cys Xaa Asp
   195 200 205
   Ile Ala Xaa Gln Leu Xaa Xaa Asn Xaa Xaa Asp Ile Xaa Val Ile Xaa
   210 215 220
   Gly Gly Gly Arg Lys Tyr Met Xaa Xaa Xaa Xaa Xaa Xaa Asp Xaa Glu
   225 230 235 240
   Tyr Xaa Xaa Asp Xaa Xaa Xaa Xaa Gly Xaa Arg Leu Asp Gly Xaa Xaa
   245 250 255
   Leu Xaa Xaa Xaa Trp Xaa Xaa Xaa Xaa Xaa Xaa Xaa Xaa Xaa Xaa Xaa
   260 265 270
   Xaa Xaa Trp Asn Arg Thr Xaa Leu Xaa Xaa Xaa Xaa Xaa Xaa Xaa Xaa
   275 280 285
   Val Xaa Xaa Leu Xaa Gly Leu Phe Xaa Pro Gly Asp Met Xaa Tyr Glu
   290 295 300
   Xaa Xaa Arg Xaa Xaa Xaa Xaa Asp Pro Ser Leu Xaa Glu Met Xaa Xaa
   305 310 315 320
   Xaa Ala Xaa Xaa Xaa Leu Xaa Xaa Xaa Xaa Xaa Gly Phe Xaa Leu Xaa
   325 330 335
   Val Glu Gly Gly Arg Ile Asp His Gly His His Glu Xaa Xaa Ala Xaa
   340 345 350
   Xaa Ala Leu Xaa Glu Xaa Xaa Xaa Xaa Asp Xaa Ala Ile Xaa Xaa Ala
   355 360 365
   Gly Xaa Xaa Thr Ser Xaa Xaa Asp Thr Leu Xaa Xaa Val Thr Ala Asp
   370 375 380
   His Ser His Val Phe Xaa Phe Gly Gly Tyr Xaa Xaa Arg Gly Xaa Ser
   385 390 395 400
   Ile Phe Gly Leu Ala Pro Xaa Xaa Xaa Xaa Xaa Asp Xaa Lys Xaa Xaa
   405 410 415
   Thr Xaa Xaa Leu Tyr Gly Asn Gly Pro Gly Tyr Xaa Xaa Xaa Xaa Gly
   420 425 430
   Xaa Arg Xaa Xaa Val Xaa Xaa Xaa Xaa Xaa Xaa Xaa Xaa Xaa Tyr Xaa
   435 440 445
   Xaa Gln Xaa Ala Val Pro Leu Xaa Xaa Glu Thr His Xaa Gly Glu Asp
   450 455 460
   Val Ala Val Phe Xaa Xaa Gly Pro Xaa Ala His Leu Xaa His Gly Val
   465 470 475 480
   Xaa Glu Gln Xaa Xaa Xaa Xaa His Val Met Ala Xaa Ala Xaa Cys Xaa
   485 490 495
   Xaa Xaa Xaa Xaa Xaa Xaa Xaa Xaa Xaa Xaa Xaa Ala Xaa Xaa Xaa Xaa
   500 505 510
   Xaa Xaa Xaa Xaa Xaa Xaa Xaa Xaa Xaa Xaa Xaa Xaa Leu Xaa Xaa Xaa
   515 520 525
   Xaa Xaa Xaa Xaa Leu Xaa Xaa Xaa Xaa Xaa Xaa Xaa Xaa
   530 535 540
<210> 19
   <211> 524
   <212> PRT
   <213> Artificial
<220>
   <223> Consensus TNALP from various mammalian species (with signal peptide and GPI anchor domain)
<220>
   <221> misc feature
   <222> (3)..(3)
   <223> Xaa can be any naturally occurring amino acid
<220>
   <221> misc feature
   <222> (7)..(7)
   <223> Xaa can be any naturally occurring amino acid
<220>
   <221> misc_feature
   <222> (14) ..(16)
   <223> Xaa can be any naturally occurring amino acid
<220>
   <221> misc_feature
   <222> (18)..(18)
   <223> Xaa can be any naturally occurring amino acid
<220>
   <221> misc_feature
   <222> (24)..(24)
   <223> Xaa can be any naturally occurring amino acid
<220>
   <221> misc feature
   <222> (27)..(27)
   <223> Xaa can be any naturally occurring amino acid
<220>
   <221> misc_feature
   <222> (31)..(31)
   <223> Xaa can be any naturally occurring amino acid
<220>
   <221> misc_feature
   <222> (35)..(35)
   <223> Xaa can be any naturally occurring amino acid
<220>
   <221> misc_feature
   <222> (39)..(39)
   <223> Xaa can be any naturally occurring amino acid
<220>
   <221> misc_feature
   <222> (42) ..(42)
   <223> Xaa can be any naturally occurring amino acid
<220>
   <221> misc_feature
   <222> (45)..(45)
   <223> Xaa can be any naturally occurring amino acid
<220>
   <221> misc_feature
   <222> (54)..(54)
   <223> Xaa can be any naturally occurring amino acid
<220>
   <221> misc_feature
   <222> (70)..(70)
   <223> Xaa can be any naturally occurring amino acid
<220>
   <221> misc_feature
   <222> (80)..(81)
   <223> Xaa can be any naturally occurring amino acid
<220>
   <221> misc_feature
   <222> (86)..(86)
   <223> Xaa can be any naturally occurring amino acid
<220>
   <221> misc_feature
   <222> (94)..(94)
   <223> Xaa can be any naturally occurring amino acid
<220>
   <221> misc_feature
   <222> (135)..(135)
   <223> Xaa can be any naturally occurring amino acid
<220>
   <221> misc_feature
   <222> (137)..(138)
   <223> Xaa can be any naturally occurring amino acid
<220>
   <221> misc_feature
   <222> (157)..(157)
   <223> Xaa can be any naturally occurring amino acid
<220>
   <221> VARIANT
   <222> (177)..(177)
   <223> Xaa can be a serine or an alanine
<220>
   <221> misc_feature
   <222> (210)..(210)
   <223> Xaa can be any naturally occurring amino acid
<220>
   <221> VARIANT
   <222> (212)..(212)
   <223> Xaa can be isoleucine or valine
<220>
   <221> misc_feature
   <222> (213)..(213)
   <223> Xaa can be any naturally occurring amino acid
<220>
   <221> misc_feature
   <222> (216)..(216)
   <223> Xaa can be any naturally occurring amino acid
<220>
   <221> misc_feature
   <222> (227)..(227)
   <223> Xaa can be any naturally occurring amino acid
<220>
   <221> misc_feature
   <222> (231)..(231)
   <223> Xaa can be any naturally occurring amino acid
<220>
   <221> misc_feature
   <222> (238)..(238)
   <223> Xaa can be any naturally occurring amino acid
<220>
   <221> misc_feature
   <222> (242)..(243)
   <223> Xaa can be any naturally occurring amino acid
<220>
   <221> misc_feature
   <222> (245)..(245)
   <223> Xaa can be any naturally occurring amino acid
<220>
   <221> misc_feature
   <222> (251)..(251)
   <223> Xaa can be any naturally occurring amino acid
<220>
   <221> misc_feature
   <222> (253)..(255)
   <223> Xaa can be any naturally occurring amino acid
<220>
   <221> misc_feature
   <222> (258)..(258)
   <223> Xaa can be any naturally occurring amino acid
<220>
   <221> misc_feature
   <222> (262)..(263)
   <223> Xaa can be any naturally occurring amino acid
<220>
   <221> misc_feature
   <222> (268)..(269)
   <223> Xaa can be any naturally occurring amino acid
<220>
   <221> misc_feature
   <222> (274)..(274)
   <223> Xaa can be any naturally occurring amino acid
<220>
   <221> misc_feature
   <222> (277)..(277)
   <223> Xaa can be any naturally occurring amino acid
<220>
   <221> misc_feature
   <222> (281)..(282)
   <223> Xaa can be any naturally occurring amino acid
<220>
   <221> VARIANT
   <222> (291)..(291)
   <223> Xaa can be a glutamate or an aspartate
<220>
   <221> misc_feature
   <222> (303)..(304)
   <223> Xaa can be any naturally occurring amino acid <220>
   <221> misc_feature
   <222> (314)..(315)
   <223> Xaa can be any naturally occurring amino acid
<220> <221> misc_feature
   <222> (317)..(318)
   <223> Xaa can be any naturally occurring amino acid
<220>
   <221> misc_feature
   <222> (321)..(321)
   <223> Xaa can be any naturally occurring amino acid
<220>
   <221> misc_feature
   <222> (323)..(325)
   <223> Xaa can be any naturally occurring amino acid
<220>
   <221> misc_feature
   <222> (357)..(357)
   <223> Xaa can be any naturally occurring amino acid
<220>
   <221> misc_feature
   <222> (361)..(361)
   <223> Xaa can be any naturally occurring amino acid
<220>
   <221> misc_feature
   <222> (364)..(365)
   <223> Xaa can be any naturally occurring amino acid
<220>
   <221> misc_feature
   <222> (368)..(369)
   <223> Xaa can be any naturally occurring amino acid
<220>
   <221> VARIANT
   <222> (374)..(374)
   <223> Xaa can be a valine or an isoleucine
<220>
   <221> misc_feature
   <222> (402)..(402)
   <223> Xaa can be any naturally occurring amino acid
<220>
   <221> misc_feature -
   <222> (412)..(412)
   <223> Xaa can be any naturally occurring amino acid
<220>
   <221> misc_feature
   <222> (425)..(425)
   <223> Xaa can be any naturally occurring amino acid
<220>
   <221> misc_feature
   <222> (463)..(463)
   <223> Xaa can be any naturally occurring amino acid
<220>
   <221> misc_feature
   <222> (475)..(475)
   <223> Xaa can be any naturally occurring amino acid
<220>
   <221> misc_feature
   <222> (480)..(480)
   <223> Xaa can be any naturally occurring amino acid
<220>
   <221> misc_feature
   <222> (488)..(488)

   <223> Xaa can be any naturally occurring amino acid
<220>
   <221> misc_feature
   <222> (494)..(495)
   <223> Xaa can be any naturally occurring amino acid
<220>
   <221> misc_feature
   <222> (499)..(499)
   <223> Xaa can be any naturally occurring amino acid
<220>
   <221> misc_feature
   <222> (501)..(501)
   <223> Xaa can be any naturally occurring amino acid
<220>
   <221> misc_feature
   <222> (503)..(508)
   <223> Xaa can be any naturally occurring amino acid
<220>
   <221> misc_feature
   <222> (510)..(510)
   <223> Xaa can be any naturally occurring amino acid
<220>
   <221> misc_feature
   <222> (512)..(512)
   <223> Xaa can be any naturally occurring amino acid
<220>
   <221> misc_feature
   <222> (514)..(514)
   <223> Xaa can be any naturally occurring amino acid
<220>
   <221> misc_feature
   <222> (517)..(522)
   <223> Xaa can be any naturally occurring amino acid
<400> 19
   Met Ile Xaa Pro Phe Leu Xaa Leu Ala Ile Gly Thr Cys Xaa Xaa Xaa
   1 5 10 15
   Ser Xaa Val Pro Glu Lys Glu Xaa Asp Pro Xaa Tyr Trp Arg Xaa Gln
   20 25 30
   Ala Gln Xaa Thr Leu Lys Xaa Ala Leu Xaa Leu Gln Xaa Leu Asn Thr
   35 40 45
   Asn Val Ala Lys Asn Xaa Ile Met Phe Leu Gly Asp Gly Met Gly Val
   50 55 60
   Ser Thr Val Thr Ala Xaa Arg Ile Leu Lys Gly Gln Leu His His Xaa
   65 70 75 80
   Xaa Gly Glu Glu Thr Xaa Leu Glu Met Asp Lys Phe Pro Xaa Val Ala
   85 90 95
   Leu Ser Lys Thr Tyr Asn Thr Asn Ala Gln Val Pro Asp Ser Ala Gly
   100 105 110
   Thr Ala Thr Ala Tyr Leu Cys Gly Val Lys Ala Asn Glu Gly Thr Val
   115 120 125
   Gly Val Ser Ala Ala Thr Xaa Arg Xaa Xaa Cys Asn Thr Thr Gln Gly
   130 135 140
   Asn Glu Val Thr Ser Ile Leu Arg Trp Ala Lys Asp Xaa Gly Lys Ser
   145 150 155 160
   Val Gly Ile Val Thr Thr Thr Arg Val Asn His Ala Thr Pro Ser Ala
   165 170 175
   Xaa Tyr Ala His Ser Ala Asp Arg Asp Trp Tyr Ser Asp Asn Glu Met
   180 185 190
   Pro Pro Glu Ala Leu Ser Gln Gly Cys Lys Asp Ile Ala Tyr Gln Leu
   195 200 205
   Met Xaa Asn Xaa Xaa Asp Ile Xaa Val Ile Met Gly Gly Gly Arg Lys
   210 215 220
   Tyr Met Xaa Pro Lys Asn Xaa Thr Asp Val Glu Tyr Glu Xaa Asp Glu
   225 230 235 240
   Lys Xaa Xaa Gly Xaa Arg Leu Asp Gly Leu Xaa Leu Xaa Xaa Xaa Trp
   245 250 255
   Lys Xaa Phe Lys Pro Xaa Xaa Lys His Ser His Xaa Xaa Trp Asn Arg
   260 265 270
   Thr Xaa Leu Leu Xaa Leu Asp Pro Xaa Xaa Val Asp Tyr Leu Leu Gly
   275 280 285
   Leu Phe Xaa Pro Gly Asp Met Gln Tyr Glu Leu Asn Arg Asn Xaa Xaa
   290 295 300
   Thr Asp Pro Ser Leu Ser Glu Met Val Xaa Xaa Ala Xaa Xaa Ile Leu
   305 310 315 320
   Xaa Lys Xaa Xaa Xaa Gly Phe Phe Leu Leu Val Glu Gly Gly Arg Ile
   325 330 335
   Asp His Gly His His Glu Gly Lys Ala Lys Gln Ala Leu His Glu Ala
   340 345 350
   Val Glu Met Asp Xaa Ala Ile Gly Xaa Ala Gly Xaa Xaa Thr Ser Xaa
   355 360 365
   Xaa Asp Thr Leu Thr Xaa Val Thr Ala Asp His Ser His Val Phe Thr
   370 375 380
   Phe Gly Gly Tyr Thr Pro Arg Gly Asn Ser Ile Phe Gly Leu Ala Pro
   385 390 395 400
   Met Xaa Ser Asp Thr Asp Lys Lys Pro Phe Thr Xaa Ile Leu Tyr Gly
   405 410 415
   Asn Gly Pro Gly Tyr Lys Val Val Xaa Gly Glu Arg Glu Asn Val Ser
   420 425 430
   Met Val Asp Tyr Ala His Asn Asn Tyr Gln Ala Gln Ser Ala Val Pro
   435 440 445
   Leu Arg His Glu Thr His Gly Gly Glu Asp Val Ala Val Phe Xaa Lys
       450                 455                 460
   Gly Pro Met Ala His Leu Leu His Gly Val Xaa Glu Gln Asn Tyr Xaa
   465                 470                 475                 480
   Pro His Val Met Ala Tyr Ala Xaa Cys Ile Gly Ala Asn Xaa Xaa His
                   485                 490                 495
   Cys Ala Xaa Ala Xaa Ser Xaa Xaa Xaa Xaa Xaa Xaa Gly Xaa Leu Xaa
               500                 505                 510
   Leu Xaa Leu Ala Xaa Xaa Xaa Xaa Xaa Xaa Leu Phe
           515                 520